(19) Europäisches Patentamt
European Patent Office
Office européen des brevets

(11) EP 2 365 076 A1

(12) **EUROPEAN PATENT APPLICATION**

(43) Date of publication:
**14.09.2011 Bulletin 2011/37**

(21) Application number: **10184573.3**

(22) Date of filing: **20.09.2001**

(51) Int Cl.:
*C12N 15/11* (2006.01) *C12N 15/85* (2006.01)
*C12N 5/10* (2006.01) *C12Q 1/68* (2006.01)
*A61K 48/00* (2006.01) *A01K 67/027* (2006.01)
*C12N 15/00* (2006.01)

(84) Designated Contracting States:
**AT BE CH CY DE DK ES FI FR GB GR IE IT LI LU MC NL PT SE TR**
Designated Extension States:
**AL LT LV MK RO SI**

(30) Priority: **20.09.2000 GB 0022995**
**20.11.2000 US 252048 P**

(62) Document number(s) of the earlier application(s) in accordance with Art. 76 EPC:
**01969944.6 / 1 430 124**

(71) Applicant: **Millipore Corporation**
**Billerica, MA 01821 (US)**

(72) Inventors:
- **Antoniou, Michael**
**Keele, Staffordshire ST5 5SP (GB)**
- **Crombie, Robert**
**Keele, Staffordshire ST5 5SP (GB)**
- **Williams , Steve Geraint**
**Crewe, Cheshire CW2 5RE (GB)**

(74) Representative: **Williams, Richard Andrew Norman**
**Harrison Goddard Foote**
**40- 43 Chancery Lane**
**London WC2A 1JA (GB)**

Remarks:
•Claims filed after the date of filing of the application/ after the date of receipt of the divisional application (Rule 68(4) EPC).
•This application was filed on 30-09-2010 as a divisional application to the application mentioned under INID code 62.

(54) **Artificial ubiquitous chromatin opening elements (ucoe)**

(57) The present invention relates to a polynucleotide comprising a ubiquitous chromatin opening element (UCOE) that does not occur in nature. The present invention also relates to a vector comprising the polynucleotide sequence, a host cell comprising the vector and use of the polynucleotide, vector or host cell in therapy, or for in vitro protein expression applications.

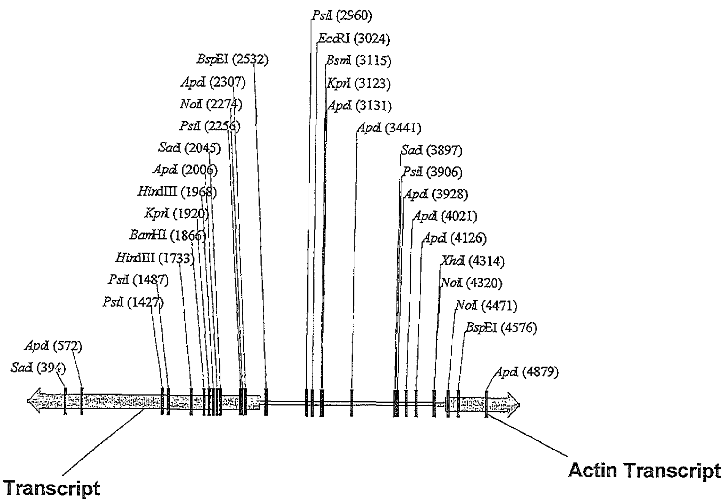


Fig 1: ARTIFICIAL UCOE Restriction map (Nco I fragment)
5232 bp



Printed by Jouve, 75001 PARIS (FR)

## Description

### FIELD OF THE INVENTION

The present invention relates to a polynucleotide comprising a ubiquitous chromatin opening element (UCOE) that does not occur in nature. The present invention also relates to a vector comprising the polynucleotide sequence, a host cell comprising the vector and use of the polynucleotide, vector or host cell in therapy, or for in vitro protein expression applications.

### BACKGROUND OF THE INVENTION

The current model of chromatin structure in higher eukaryotes postulates that genes are organised in "domains"[1,2]. Chromatin domains are envisaged to exist in either a condensed, "closed", transcriptionally silent state, or in a de-condensed, "open" and transcriptionally competent configuration. The establishment of an open chromatin structure characterised by increased DNaseI sensitivity, DNA hypomethylation and histone hyperacetylation, is considered a pre-requisite to the commencement of gene expression.

The open and closed nature of chromatin regions is reflected in the behaviour of transgenes that are randomly integrated into the host cell genome. Identical constructs give different patterns of tissue-specific and development stage-specific expression when integrated at different locations in the mouse genome[3-5]. A variegated expression pattern within a given transgenic mouse tissue, known as position effect variegation (PEV), is also frequently observed[6]. When exogenous genes are integrated into the chromosome of mammalian cells cultures *in vitro,* many of the integration events result in rapid silencing of the transgene and the remainder give large variability in expression levels[7,8]. These position effects render transgene expression inefficient, with implication for both basic research and' biotechnology applications.

The chromatin domain model of gene organisation suggests that genetic control elements that are able to establish and maintain a transcriptionally competent open chromatin structure should be associated with active regions of the genome.

Locus Control Regions (LCRs) are a class of transcriptional regulatory elements with long-range chromatin remodelling capability. LCRs are functionally defined in transgenic mice by their ability to confer site-of-integration independent, transgene copy number-dependent, physiological levels of expression on a gene linked in *cis,,* especially single copy transgenes[9,10]. Crucially, such expression is tissue-specific. LCRs are able to obstruct the spread of heterochromatin, prevent PEV[6] and consist of a series of Dnase I hypersensitive (HS) sites which can be located either 5' or 3' of the genes that they regulate[10].

LCRs appear to be comprised of two separate, although not necessary independent components. First, the establishment of an open chromatin domain, and second a dominant transcriptional activation capacity to confer transgene copy number dependent expression[9]. The molecular mechanisms by which LCRs exert their function remain a point of contention[2,11-13].

The generation of cultured mammalian cell lines producing high levels of a therapeutic protein product is a major developing industry. Chromatin position effects make it a difficult, time consuming and expensive process. The most commonly used approach to the production of such mammalian "cell factories" relies on gene amplification induced by a combination of a drug resistance gene (e.g., DHFR, glutamine synthetase (Kaufman, 1990, Methods Enzymol., 185:537-566, which is incorporated herein by reference)) and the mainatenance of stringent selective pressure. The use of vectors containing LCRs from highlyexpressed gene domains, using cells derived from the appropriate tissue, greatly simplifies the procedure (Needham et al., 1992, Nucleic Acids Res., 20:997-1003; Needham et al., 1995, Protein Expr. Purif., 6:124-131, each of which is incorporated herein by reference).

However, the tissue-specificity of LCRs, although useful in some circumstances, is also a major limitation for many applications, for instance where no LCR is known for the tissue in which expression is required, or where expression in many, or all, tissues is required.

Our co-pending patent application PCT/GB99/02357 (WO 00/05393), incorporated by reference herein, describes elements that are responsible for establishing an open chromatin structure across a locus that consists exclusively of ubiquitously expressed, housekeeping genes. These elements are not derived from an LCR. The invention provides a polynucleotide comprising a ubiquitous chromatin opening element (UCOE) which opens chromatin or maintains chromatin in an open state and facilitates reproducible expression of an operably-linked gene in cells of at least two different tissue types, wherein the polynucleotide is not derived from a locus control region.

Methylation-free CpG islands are well-known in the art (Bird et al (1985) Cell 40: 91-99, Tazi and Bird (1990) Cell 60: 909-920) and may be defined as CpG-rich regions of DNA with above average (>60%) content of CpG dinucleotides where the cytosine residues are not methylated and which extend over the 5' ends of two closely spaced (0.1-3 kb) divergently transcribed genes. These regions of DNA remain unmethylated in all tissues throughout development (Wise and Pravtcheva (1999) Genomics 60: 258-271). They are associated with the 5' ends of all ubiquitously expressed genes, as well as an estimated 40% of genes showing a tissue restricted expression profile[16,17] and are known to be localised regions of active chromatin[18].

An 'extended' methylation-free CpG island is a methylation-free CpG island that extends across a region encompassing more than one transcriptional start site and/or extends for more than 300bp and preferably more than 500bp. The borders of the extended methylation-free CpG island are functionally defined through the use of PCR over the region in combination with restriction endonuclease enzymes whose ability to digest (cut) DNA at their recognition sequence is sensitive to the methylation status of any CpG residues that are present. One such enzyme is HpaII, which recognises and digests at the site CCGG, which is commonly found within CpG islands, but only if the central CG residues are *not* methylated. Therefore, PCR conducted with HpaII-digested DNA and over a region harbouring HpaII sites, does *not* give an amplification product due to HpaII digestion if the DNA is *unmethylated.* The PCR will *only* give an amplified product if the DNA is *methylated.* Therefore, beyond the methylation-free region HpaII will not digest the DNA a PCR amplified product will be observed thereby defining the boundaries of the "extended methylation-free CpG island".

We have demonstrated that regions spanning methylation-free CpG islands encompassing dual, divergently transcribed promoters from the human TATA binding protein (*TBP*)/proteosome component-B1 (*PSMBI*) and heterogenous nuclear ribonucleoprotein A2/B1 (*hnRNPA2*)/heterochromatin. protein 1Hs$\gamma$ (*HP1* $^{Hs\gamma}$) gene loci give reproducible, physiological levels of gene expression and that they are able to prevent a variegated expression pattern and silencing that normally occurs with transgene integration within centromeric heterochomatin.

We have shown that methylation-free CpG islands associated with actively transcribing promoters possess the ability to remodel chromatin and are thus thought to be a prime determinant in establishing and maintaining an open domain at housekeeping gene loci.

UCOE's confer an increased proportion of productive gene delivery events with improvements in the level and stability of transgene expression. This has important research and biotechnological applications including the generation of transgenic animals and recombinant protein products in cultured cells. We have shown beneficial effects of UCOEs on expression of the CMV-EGFP reporter construct and with the secreted, pharmaceutically valuable protein erythropoietin, The properties of UCOEs also suggest utility in gene therapy, the effectiveness of which is often limited by a low frequency of productive gene delivery events and an inadequate level and duration of expression[45].

Given these significant implications and wide ranging applications, there is a desire to further optimise transgene expression levels and achieve improved stability of gene expression over a prolonged period of culture.

One particular need is to overcome the directional bias observed in some naturally-occurring UCOEs, Although UCOEs confer position-independent transcriptional enhancement on operably-linked promoters, this is, to some extent, orientation-dependent (i.e. the UCOE is significantly more effective in one orientation than the other). In some circumstances, such as an expression vector comprising two expression units transcribed divergently with a UCOE situated between them, there is an advantage in being able to obtain balanced, high-level expression from both promoters, which may not be possible with a natural UCOE. There is therefore a need for artificially-constructed UCOEs that are effective in both orientations.

## STATEMENTS OF THE INVENTION

According to the present invention there is provided a polynucleotide comprising a UCOE, which opens chromatin or maintains chromatin in an open state and facilitates reproducible expression of an operably-linked gene in cells of at least two different tissue types, wherein the nucleotide sequence of the UCOE does not occur in nature.

Genomic regions comprising regulatory sequences from at least two genes were combined to form a chimeric UCOE which significantly enhanced gene expression over a prolonged period of culture. Such a chimeric UCOE constitutes a nucleotide sequence that does not occur in nature. Accordingly the phrase "does not occur in nature" refers to a situation wherein the nucleotide sequence of the element constituting the UCOE does not naturally exist as such and is man-made or artificially constructed, being a combination of naturally-occurring and/or artificially-generated sequences.

As used herein, the terms "artificial", "artificially-constructed", "chimeric", and the like, in reference to a UCOE, are used interchangeably throughout to mean that the UCOE or the element constituting the UCOE does not naturally exist; i.e., "does not occur in nature." Where the UCOE is a combination of naturally-occurring sequences, it is their arrangement or organization into the UCOE that is non-natural. By way of non-limiting example, an artificial UCOE could be comprised of two naturally-occurring sequences that are normally disparate (from different regions of a chromosome, from different chromosomes, from different organisms, etc.) and that have been brought together in a non-natural organization, to create a chimeric or artificial UCOE.

As used herein, the terms "artificially synthesized" and "artificially-generated" in reference to sequences in a UCOE, refer to sequences that are non-natural; i.e., sequences that are not naturally-occurring and are wholly synthetic. Such "artificially synthesized" and "artificially-generated" sequences can also be combined with naturally-occurring sequences to make up or create an artificial UCOE.

According to an alternative aspect of the invention there is provided a nucleic acid molecule comprising a DNA sequence selected from:

i) the DNA sequence as represented in Figure 2 or Figure 7;
ii) DNA sequences which hybridise to the sequence presented in Figure 2 or Figure 7 which encode a polypeptide according to the invention.

In a preferred embodiment of the invention there is provided an isolated nucleic acid molecule which anneals under stringent hybridisation conditions to the sequence presented in Figure 2 or Figure 7.

Stringent hybridisation/washing conditions are well known in the art. For example, nucleic acid hybrids that are stable after washing in 0.1 x SSC ,0.1% SDS at 60°C. It is well known in the art that optimal hybridisation conditions can be calculated if the sequence of the nucleic acid is known. For example, hybridisation conditions can be determined by the GC content of the nucleic acid subject to hybridisation. Please see Sambrook et al (1989), Molecular Cloning; A Laboratory Approach. A common formula for calculating the stringency conditions required to achieve hybridisation between nucleic acid molecules of a specified homology is:

$$T_m = 81.5^0\,C + 16.6\,\mathrm{Log}\,[Na^+] + 0.41[\,\%\,G + C] - 0.63\ (\%\mathrm{formamide})$$

Preferably the polynucleotide of the present invention facilitates reproducible expression of an operably-linked gene non-tissue specifically.

Preferably the polynucleotide of the present invention facilitates reproducible expression of an operably-linked gene in all tissue types where active gene expression occurs.

Preferably the polynucleotide of the present invention facilitates expression of an operably-linked gene at a physiological level.

Preferably the polynucleotide of the present invention comprises an extended methylation-free, CpG-island.

Preferably the polynucleotide of the present invention comprises one or more naturally-occurring sequences associated with the control of gene expression.

Preferably the polynucleotide of the present invention comprises one or more naturally-occurring promoters.

Preferably the polynucleotide of the present invention comprises dual or bi-directional promoters that transcribe divergently.

Preferably the polynucleotide of the present invention comprises the human β-actin CpG island/ promoter region, or fragment thereof.

Preferably the polynucleotide of the present invention comprises a DNA fragment within the range of 100bp to 3 kb spanning the human β-actin CpG island/ promoter region or a fragment thereof.

Preferably the polynucleotide of the present invention comprises the human PDCD2 CpG island/ promoter region or a fragment thereof.

Preferably the polynucleotide of the present invention comprises a DNA fragment within the range from 100bp to 3.0 kb spanning the human PDCD2 CpG island/ promoter region, or a fragment thereof.

Preferably the polynucleotide of the present invention comprises a DNA fragment within the range from 100bp to 3.0kb spanning the human β-actin CpG island/ promoter region and a DNA fragment within the range from 100bp to 3.0 kb spanning the human PDCD2 CpG island/ promoter region. Preferably said fragments are directly adjacent with their promoters oriented divergently.

Preferably the polynucleotide of the present invention comprises a 2kb DNA fragment spanning the human β-actin CpG island/ promoter region and a 1.8kb DNA fragment spanning the human PDCD2 CpG island/ promoter region. Preferably said fragments are directly adjacent with their promoters oriented divergently.

In further preferred embodiment the polynucleotide comprises the sequence of Figure 2 or a fragment thereof, in either orientation.

Preferably the polynucleotide comprises the human RNP CpG island / promoter region or a fragment thereof.

Preferably the polynucleotide comprises a 4kb DNA fragment spanning the human RNP CpG island / promoter region.

Preferably the polynucleotide comprises an extended methylation-free CpG island containing bidivergent promoters, adjacent to at least one further sequence comprising a methylation-free CpG island.

Preferably the polynucleotide comprises the human RNP CpG island /promoter region and a DNA fragment in the range 100bp to 3.0kb spanning the human β-actin CpG island / promoter region.

In a further preferred embodiment the polynucleotide comprises the sequence of Figure 7 or a fragment thereof

in either orientation. Preferably the polynucleotide comprises one or more promoter sequences.

It is known in the art that initiation of transcription may, under some circumstances, be inhibited by read-through transcripts from upstream promoters (Youssoufian and Lodish (1993) Transcriptional inhibition of the murine erythropoietin receptor gene by an upstream repetitive element, Mol Cell Biol 13 (1) 98-104). Therefore, one embodiment of the invention comprises the polynucleotide of the present invention wherein one or more of the promoter sequences are mutated in such a way that they are incapable of initiating transcription.

Preferably the promoter is selected from CMV, EF-1$\alpha$, RSV LTR, or HIV2 LTR or combinations of sequences derived therefrom. More preferably the promoter is a CMV promoter. Most preferably it is the mouse CMV promoter.

Preferably the polynucleotide of the present invention comprises at least one sequence which is artificially synthesised.

The present invention also provides a vector comprising the polynucleotide of the present invention.

Preferably said vector is an expression vector adapted for eukaryotic gene expression.

Typically said adaptation includes, by example and not by way of limitation, the provision of transcription control sequences (promoter sequences) which mediate cell/tissue specific expression.

Promoter and enhancer are terms well-known in the art and include the following features which are provided by example only, and not by way of limitation. Promoters are 5', *cis*-acting regulatory sequences directly linked to the initiation of transcription. Promoter elements include so-called TATA box and RNA polymerase initiation selection (RIS) sequences which function to select a site of transcription initiation. These sequences also bind polypeptides which function, *Inter alia,* to facilitate transcription initiation selection by RNA polymerase.

Enhancer elements are *cis* acting nucleic acid sequences often found 5' to the transcription initiation site of a gene (enhancers can also be found 3' to a gene sequence or even located in intronic sequences and is therefore position independent). Enhancers function to increase the rate of transcription of the gene to which the enhancer is linked. Enhancer activity is responsive to *trans* acting transcription factors (polypeptides) which have been shown to bind specifically to enhancer elements. The binding/activity of transcription factors is responsive to a number of environmental cues which include, by way of example and not by way of limitation, intermediary metabolites (eg glucose), environmental effectors ( eg heat,). (See Eukaryotic Transcription Factors, by David S. Latchman, Academic Press Ltd, San Diego)

Adaptations also include the provision of selectable markers and autonomous replication sequences which both facilitate the maintenance of said vector in either the eukaryotic cell or prokaryotic host. Vectors which are maintained autonomously are referred to as episomal vectors. Episomal vectors are desirable since they are self-replicating and so persist without the need for integration. Episomal vectors of this type are described in WO98/07876.

Adaptations which facilitate the expression of vector encoded genes include the provision of transcription termination/polyadenylation sequences. This also includes the provision of internal ribosome entry sites (IRES) which function to maximise expression of vector encoded genes arranged in bicistronic or multi-cistronic expression cassettes.

These adaptations are well-known in the art. There is a significant amount of published literature with respect to expression vector construction and recombinant DNA techniques in general. Please see, Sambrook et al (1989) Molecular Cloning: A Laboratory Manual, Cold Spring Harbour Laboratory, Cold Spring Harbour, NY and references therein; Marston, F (1987) DNA Cloning Techniques: A Practical Approach Vol III IRL Press, Oxford UK; DNA Cloning: F M Ausubel et al, Current Protocols in Molecular Biology, John Wiley & Sons, Inc.(1994).

Preferably the vector comprises an expressible gene operably-linked to a promoter and the polynucleotide.

Preferably the vector is an episomal or integrating vector.

In a preferred embodiment, the vector of the present invention is a plasmid,

Alternatively, the vector may be a virus, such as an adenovirus, adeno-associated virus, a herpesvirus, vaccinia virus, lentivirus or other retrovirus.

Preferably the operably-linked gene is a therapeutic nucleic acid sequence.

Preferably the vector comprises two sites for insertion of open reading frames to be expressed, each transcribed from a distinct promoter, said promoters being arranged so as to transcribe divergently and both promoters being operably-linked to an artifically-constructed UCOE situated between them, and wherein said UCOE has been so constructed as to be effective in both orientations. This is particularly useful for the production of proteins that comprise two or more polypeptide chains, including, but not limited to, immunoglobulins. The insertion sites in the vector may be inserted with nucleic acid encoding different polypeptides of interest, including, but not limited to an open reading frame encoding an immunoglobulin heavy and an immunoglobulin light chain.

Preferably the vector comprises the sequence of Figure 2, the CMV promoter, a multiple cloning site, a polyadenylation sequence and genes encoding selectable markers under suitable control elements. It will be apparent to one of skill in the art that the UCOE can be inserted in both orientations.

Preferably the vector comprising the artificial UCOE is CET 500 as shown schematically in Figure 3a.

In an alternative embodiment, the vector is CET 501 as shown in Figure 3b.

Alternatively, the vector of any of claims comprises the sequence of Figure 7, the CMV promoter, a multiple cloning site, a polyadenylation sequence and genes encoding selectable markers under suitable control elements.

Similarly, it will be apparent to one of skill in the art that the artificial UCOE can be inserted in both orientations.

In an preferred embodiment of this alternative, the vector comprising the artificial UCOE is known as CET 600 as shown schematically in Figure 8a.

In a further alternative embodiment, the vector is CET 601 as shown schematically in Figure 8b.

The present invention also provides a host cell that is transfected with the vector of the present invention.

The present invention also provides the polynucleotide, vector or the host cell for use in therapy.

The present invention also provides use of the polynucleotide, vector or host cell in the manufacture of a composition for use in gene therapy.

The present invention also provides a method of treatment, comprising administering to a patient in need of such treatment a pharmaceutically effective amount of the polynucleotide, vector or host cell of the present invention. Preferably the patient is suffering from a disease treatable by gene therapy.

The present invention also provides a pharmaceutical composition comprising the polynucleotide and/or the vector and/or host cell, optionally in admixture with a pharmaceutically acceptable carrier or diluent, for therapy to treat a disease or provide the cells of a particular tissue with an advantageous protein or function.

The polynucleotide, vector or host cell of the invention or the pharmaceutical composition may be administered via a route which includes systemic intramuscular, intravenous, aerosol, oral (solid or liquid form), topical, ocular, rectal, intraperitoneal and/or intrathecal and local direct injection.

The exact dosage regime will, of course, need to be determined by individual clinicians for individual patients and this, in turn, will be controlled by the exact nature of the protein expressed by the gene of interest and the type of tissue that is being targeted for treatment.

The dosage also will depend upon the disease indication and the route of administration. The number of doses will depend upon the disease, and the efficacy data from clinical trials.

The amount of polynucleotide or vector DNA delivered for effective gene therapy according to the invention will preferably be in the range of between 50 ng -1000 μg of vector DNA/kg body weight; and more preferably in the range of between about 1-100 μg vector DNA/kg.

Although it is preferred according to the invention to administer the polynucleotide, vector or host cell to a mammal for *in vivo* cell uptake, an *ex vivo* approach may be utilised whereby cells are removed from an animal, transduced with the polynucleotide or vector, and then re-implanted into the animal. The liver, for example, can be accessed by an *ex vivo* approach by removing hepatocytes from an animal, transducing the hepatocytes *in vitro* and re-implanting the transduced hepatocytes into the animal (e.g., as described for rabbits by Chowdhury et al., Science 254:1802-1805, 1991, or in humans by Wilson, Hum. Gene Ther. 3:179-222, 1992). Such methods also may be effective for delivery to various populations of cells in the circulatory or lymphatic systems, such as erythrocytes, T cells, B cells and haematopoietic stem cells.

In another embodiment of the invention, there is provided a mammalian model for determining the efficacy of gene therapy using the polynucleotide, vector or host cell of the invention. The mammalian model comprises a transgenic animal whose cells contain the vector of the present invention. Methods of making transgenic mice (Gordon et al., Proc. Natl. Acad. Sci. USA 77:7380 (1980); Harbers et al., Nature293:540 (1981); Wagner et al., Proc. Natl. Acad. Sci. USA 78:5016 (1981); and Wagner et al., Proc. Natl. Acad. Sci. USA 78:6376 (1981), sheep pigs, chickens (see Hammer et al., Nature 315:680 (1985)), etc., are well-known in the art and are contemplated for use according to the invention. Such animals permit testing prior to clinical trials in humans.

Transgenic animals containing the polynucleotide of the invention also may be used for long-term production of a protein of interest.

The present invention also provides for use of the polynucleotide and/or vector and/or host cell in a cell culture system in order to obtain a desired gene product. Suitable cell culture systems are well known in the art and are fully described in the body of literature known to those skilled in the art.

The present invention also provides for use of the polynucleotide to increase the expression of an endogenous gene comprising inserting the polynucleotide into the genome of a cell in a position operably associated with the endogenous gene thereby increasing the level of expression of the gene.

The present invention also provides the use of the polynucleotide of the present invention in producing transgenic plants.

The generation of transgenic plants which have increased yield, resistance, etc. are well known to those skilled in the art. The present invention also provides for transgenic plant containing cells which contain the polynucleotide of the present invention. Some or all of the cells comprising the artificial UCOE may originate from plants.

The present invention also provides for a transgenic non-human animal containing cells which contain the polynucleotide.

The present invention also relates to the use of polynucleotide of the present invention in functional genomics applications. Functional genomics relates principally to the sequencing of genes specifically expressed in particular cell types or disease states and now provides thousands of novel gene sequences of potential interest for drug discovery or gene therapy purposes. The major problem in using this information for the development of novel therapies lies in how to determine the functions of these genes. UCOEs can be used in a number of functional genomic applications in order to determine the function of gene sequences. The functional genomic applications of the present invention include, but are not limited to:

(1) Using the polynucleotide of the present invention to achieve sustained expression of anti-sense versions of the gene sequences or ribozyme knockdown libraries, thereby determining the effects of inactivating the gene on cell phenotype.

(2) Using the polynucleotide of the present invention to prepare expression libraries for the gene sequences, such that delivery into cells will result in reliable, reproducible, sustained expression of the gene sequences. The resulting cells, expressing the gene sequences can be used in a variety of approaches to function determination and drug discovery. For example, raising neutralising antibodies to the gene product; rapid purification of the protein product of the gene itself for use in structural, functional or drug screening studies; or in cell-based drug screening.

(3) Using the polynucleotide of the present, invention in approaches involving mouse embryonic stem (ES) cells and transgenic mice. One of the most powerful functional genomics approaches involves random insertion into genes in mouse ES cells of constructs which only allow drug selection following insertion into expressed genes, and which can readily be rescued for sequencing (G.Hicks et al., Nature Genetics, 16, 338-334). Transgenic mice with knockout mutations in genes with novel sequences can then readily be made to probe their function. At present this technology works well for the 10% of mouse genes which are well expressed in mouse ES cells. Incorporation of UCOEs into the integrating constructs will enable this technique to be extended to identify all genes expressed in mice.

In an alternative embodiment of the invention, there is provided a method for the production of the polypeptide according to the invention comprising:

i) providing a cell transformed/transfected with a nucleic acid molecule according to the invention;

ii) growing said cell in conditions conducive to the manufacture of said polypeptide; and

iii) purifying said polypeptide from said cell, or its growth environment.

In a preferred embodiment of the invention said nucleic acid molecule is the vector according to the invention.
In a preferred method of the invention said vector encodes, and thus said polypeptide is provided with, a secretion signal to facilitate purification of said polypeptide.
Alternatively, other preferred embodiments may include further refinements to facilitate purification of expressed recombinant protein, such as affinity tags or epitopes, or enzymatic cleavage sites.

## DETAILED DESCRIPTION OF THE INVENTION

The invention will now be described by way of example only and with reference to the accompanying figures wherein;

**Figure 1** is a linear restriction map of the artificial UCOE *Nco* I fragment. The PDCD2 and actin transcripts are shown as shaded arrows, with the direction of the arrow depicting the direction of transcription from their respective promoters.

**Figure 2** shows the nucleotide sequence of the PDCD2/Actin artificial UCOE *Nco* I fragment according to the invention.

**Figures 3a** and **3b** depict plasmid maps of the PDCD2/Actin artificial UCOE-containing expression vectors.

**Figure 3a** shows CET 500, comprising the PDCD2/Actin artificial UCOE upstream of the CMV promoter and a multiple cloning site suitable for insertion of the open reading frame to be expressed. In this particular embodiment the plasmid backbone is from pEGFPN-1 and carries a kanamycin/ neomycin resistance gene.

**Figure 3b** shows CET 501, comprising the PDCD2/actin artificial UCOE in the reverse orientation.

**Figure 4** shows levels of expression of a reporter transgene (EGFP) in pools of stably-transfected CHO cells maintained under G418 selection over a period of 85 days. Expression was driven by the CMV promoter alone (CMV), or in combination with the 8kb RNP/HP-1 UCOE (CET 220) or artificial PDCD2/actin UCOE CET 510) and was measured as median fluorescence by FACS.

**Figure 5** shows the proportion of cells expressing the transgene in the above experiment expressed as % positive cells on FACS analysis over a period of 85 days.

**Figure 6** is a linear restriction map of the RNP/HP-1/actin artificial UCOE fragment. The methylation-free islands are shown as shaded arrows, with the direction of the arrow depicting the direction of transcription from their respective promoters.

**Figure 7** shows the nucleotide sequence of the RNP/HP-1/actin artificial UCOE fragment according to the invention.

**Figure 8** depicts plasmid maps of RNP/HP-1/actin artificial UCOE-containing expression vectors.

**Figure 8a** shows CET 600, comprising the RNP/HP-1/actin artificial UCOE upstream of the CMV promoter and a multiple cloning site suitable for insertion of the open reading frame to be expressed. In this particular embodiment the plasmid backbone is from pEGFPN-1 and carries a kanamycin/ neomycin resistance gene.

**Figure 8b** shows CET 601, comprising the RNP/HP-1/actin artificial UCOE in the reverse orientation.

**Figure 9** shows levels of expression of a reporter transgene (EGFP) in pools of stably-transfected CHO cells maintained under G418 selection over a period of 127 days. Expression was driven by the CMV promoter alone (CMV), or in combination with the 8kb RNP UCOE in either forward (CET 220) or reverse (CET 221) or by RNP/HP-1/actin artificial UCOE in either forward (CET 610) or reverse (CET 611) orientation and was measured as median fluorescence by FACS. It will be understood that CET 610 is therefore the equivalent of CET 600, but containing EGFP as the inserted gene, and that CET 611 is the corresponding EGFP-containing equivalent of CET 601.

**Figure 10** shows the proportion of cells expressing the transgene in the above experiment expressed as % positive cells on FACS analysis over a period of 127 days.

**Figures 11a- 11d** depict plasmid maps of the bi-directional UCOE vectors for the expression of immunoglobulins.

## Example 1

### Heterologous combinations of UCOE fragments

We have found that the substitution of the *A2*/*HP1* fragments in the 4.0CMV and 8.0CMV constructs with the comparable region from the *TBP*/*B1* locus gave substantial increases in the level and stability of EGFP expression from the hCMV promoter. These findings in tissue culture cells provide evidence that regions of DNA encompassing an extended CpG island and divergently transcribed promoters are responsible for conferring an increase in the proportion of viable integration events, significantly improved transgene expression and a resistance to transcriptional silencing even from within centromeric heterochromatin.

Genomic regions encompassing CpG islands from housekeeping genes associated with a single promoter were combined. The construct comprised the 2kb CpG island from the 5' end of the human β-*actin* gene[37] joined to a 3.2kb fragment containing the CpG island from the 5' end of the human *PDCD2* gene. The promoters were divergently transcribed and their transcriptional start sites separated by 1.9kb.

The entire 5.2kb combination was then linked to an EGFP reporter gene driven by the CMV promoter (PDCD2/ACTIN). As a comparison, the β-*actin* CpG island/promoter region *alone was also* inserted *upstream* of *the* CMV-EGFP expression vector (ACTIN).

### Materials and Methods

With particular reference to Figure 2, the CMV promoter was removed from pEGFP-N1 by digestion with *Ase*I and *Nhe*I followed by blunting with T4 DNA polymerase and religation to produce pΔ-EGFP-N1. A β-Actin promoter EGFP fusion was constructed by digesting MA39 (HSACCYBB) with *Bsm*I and *Nco*I, isolating the appropriate fragment and blunting it with T4 DNA polymerase. This fragment was ligated into pΔ-EGFP-N1 that had been digested with *Age*I, blunted with T4 DNA polymerase and then digested with SmaI. This produced an in frame fusion of the first codon of the β-Actin gene with EGFP, expression of which was driven off the β-Actin promoter/MFI (methylation free island). The construct, pActin-EGFP, was shown to express EGFP in stably transfected CHOK1 cells.

To construct a vector with bi-directional promoters and an extended methylation free island, the PDCD2 gene and some of its promoter was initially removed from pCP2-TNN (approximately 160kb of the TBP locus in pCYPAC-2) by digestion with *Swa*I and *Bsp*EI and sub cloned into pBluescriptKS+ that had been digested with *Eco*RV and *Xma*I (pPDCD2-KS). As this vector did not contain the whole of the PDCD2 methylation free island, the remaining 5' region of the PDCD2 methylation free island, which also contained the promoter, was obtained by PCR from pCP2-TNN using the following priers 5'GCGGTACCAAGGGCATTCTGAAGTTAACC-3', 5-AGCTCCACAGGCCTGG-3'. The PCR product was then digested with *Kpn*I (site generated with PCR primers) and *Stu*I (internal site), pActin-EGFP was digested with *Sal*I and *Kpn*I, and the PDCD2 gene was removed from pPDCD2-KS by digestion with *Sal*I and *Stu*I. All three fragments were ligated together to create pPDCD2-Actin-EGFP.

The region containing the methylation free island of pF-PDCD2-Actin-EGFP was removed as an *Nco* I fragment (approximately 5.2kb), this was blunted with T4 DNA polymerase and then ligated, in both orientations, into pEGFPN-1 that had been digested with *Ase* I and then blunted. These vectors were called CET 510 (UCOE in forward orientation) and CET511 (UCOE in reverse orientation). The corresponding empty expression vectors with no transgene inserted into the multiple cloning sites were termed CET 500 and CET 501, respectively (see Figure 3).

Constructs were linearized with *Pvu* I, transfected into CHO-K1 cells and selected under G418 selection (0.6mg/ml) for both experiments.

It will be understood that one of skill in the art may adapt these procedures for preparation and testing of other polynucleotides of the invention.

**Results**

As shown in Figures 4 and 5, the artificial UCOE construct gave levels of reporter gene expression that were comparable with those achieved with the 8kb RNP/HP-1 UCOE in terms of median fluorescence, and slightly better in terms of the proportion of cells expressing over the time course of the experiment.

__**Example 2**__

**Heterologous combinations of UCOE and methylation-free CpG island fragments**

**Materials and Methods**

The actin methylation-free island was removed from pActin-EGFP by digestion with *Nco* I, blunted followed by digestion with *Kpn* I. The RNP 4kb fragment was removed from CET 20 by digestion with *Kpn* I and *Hind* III. These two fragments were then ligated into pBKS which had been digested with *Cla* I, blunted and then cut with *Hind* III. This gave the artificial UCOE in pBKS.

The artificial UCOE was then removed from pBKS by digestion with *Sal* I and *Hind* III, blunted and ligated into pEGFP-N1 that had been digested with *Ase* I and blunted. The UCOE was inserted in both orientations to create CET 610 and CET 611 respectively. The corresponding expression vectors with no transgene inserted into multiple cloning sites were termed CET 600 and CET 601, respectively (see Figure 8).

Constructs were linearised and transfected into CHO-K1 cells and selected under G418 selection (0.6mg/ml) for the duration of the experiments.

**Results**

The effects of the artificial UCOE (in both orientations) on levels of transgene expression were assessed by comparison with results obtained with CMV promoter alone, and the 8kb RNP/HP-1 UCOE (in both orientations). Figure 9 shows that, in terms of median fluorescence on FACS analysis, the CMV promoter alone gave very low levels of expression. Both the RNP UCOE (CET 220) and artificial UCOE (CET 600) gave greatly increased (30-fold) expression in the forward orientation and were comparable. However, a directional bias seen with the RNP UCOE was less marked with the artificial UCOE, i.e. in the reverse orientation the artificial UCOE was superior, although still slightly less good than either UCOE in the forward orientation. This was true for both overall level of expression and ability to maintain expression over a prolonged period (more than 120 days).

When the same experiment was analysed interms of proportion of cells maintaining expression over time, both UCOEs, in both orientations, gave consistently better results than CMV alone (approximately twice as many cells expressing in the early part of the experiment). This difference became more marked over longer periods, with the CMV only population progressively losing expressing cells, until by 120 days only approximately 10% cells were still expressing. This contrasts with maintenance at approximately 90% for both UCOEs in the forward orientation, and only slightly lower levels (75-85%) for the reverse orientations by the end of the experiment.

## Example 3

### Co-expression of two genes on the same vector

Efficient functional antibody production requires appropriately balanced expression of the heavy and light chains. Transfection of the two chains on separate plasmids makes the maintenance of an equal copy number difficult and provides the potential for transcriptional interference between the genes if the vectors integrate close to one another in the genome.

A series of new vectors for the co-expression of two genes on the same vector have been constructed to compare neo versus puro as resistance markers and hCMV, beta actin or mCMV promoters to drive light or heavy chain expression (figure 11).

### Material and Methods

The two *Sfi* I sites of pORT1 (Cobra) were changed to *Mfe* I sites by introduction of adapter molecules comprised of annealed oligos Mfe.F, 5'-AACAATTGGCGGC and Mfe.R, 5'-GCCAATTGTTGCC.

The HSV TK polyA site was amplified from pVgRXR (Invitrogen) with primers TK.F, 5'ACGCGTCGACGGAAGGAGACAATACCGGAAG and TK.R, 5'-CCGCTCGAGTTGGGGTGGGGAAAAGGAA. The *Sal* I to *Xho* I fragment was inserted into the *Sal* I site. The murine PGK polyA site was amplified from male BALB/c genomic DNA (Clontech) using primers mPGK.F, 5'-CGGGATCCGCCTGAGAAAGGAAGTGAGCTG and mPGK.R, 5'-GAAGATCTGGAGGAATGAGCTGGCCCTTA, and the *Bam*H I to *Bgl* II fragment was cloned into the *Bam*H I site.

The *Ase* I to *Sal* I fragment of pcDNA3.1 containing the neo expression cassette was treated with T4 DNA polymerase, ligated to *Spe* I linkers (5'-GACTAGTC). The *Spe* I fragment was then cloned into the *Spe* I site to give pORTneoF or the *Eco*R I to *Not* I fragment of CET700 (Cobra) carrying the puromycin resistance cassette was treated with T4 DNA polymerase, ligated to *Xba* I linkers, and the *Xba* I fragment was cloned into the *Xba* I site to give pORTpuroF.

The *Hind* III to *Bam*H I murine CMV promoter fragment from pCMVEGFPN-1 (Cobra) was subcloned into the *Hind* III to *Bam*H I sites of the Hybrid UCOE in BKS+ (Cobra). The human CMV promoter was then amplified from plasmid pIRESneo (Clontech) using primers hCMVF, 5'-CTCGAGTTATTAATAGTAATCAATTACGGGGTCAT and hCMVR, 5'-GTCGACGATCTGACGGTTCACTAAACCAGCTCT and the *Xho* I to *Sal* I fragment was cloned into the *Sal* I site. The *Bam*H I to *Sal* I fragment was then cloned into the *Bam*H I to *Sal* I sites of pORTneoF to give pBDUneo100, or into pORTpuroF to give pBDUpuro300.

The two ATG codons upstream of the *Sal* I cloning site in the Hybrid UCOE in BKS+ were altered by site-directed mutagenesis and the *Bam*H I to *Sal* I fragment was cloned into the *Bam*H I to *Sal* I sites of pORTneoF to give pBDUneo200, or into pORTpuroF to give pBDUpuro400.

Human antibody light chain coding sequences were cloned into either the *Bam*H I or *Sal* I sites of all four bi-directional UCOE vectors (pBDUneo100, pBDUneo200, pBDUpuro300 and pBDUpuro400), followed by immunoglobulin heavy chain coding sequence at the remaining *Bam*H I or *Sal* I cloning site to give pBDUneo112, pBDUneo121, pBDUneo212, pBDUneo221, pBDUpuro112, pBDUpuro121, pBDUpuro212 and pBDUpuro221. All eight antibody expression constructs were transfected into CHO-K1 cells using Lipofectamine (Invitrogen) following the manufacturer's instructions, and selected with 500 μg/ml G418 (*neo* vectors) or 12.5 μg/ml puromycin (*puro* vectors).

Pools were selected and antibody production rates compared between the different constructs to determine the optimal promoter and selectable marker combination for antibody expression in CHO cells.

### Results

Table 1 demonstrates that vectors containing the light chain expressed from the murine CMV promoter gave the best expression of the antibody. No significant difference was observed between production rates obtained with vectors containing the G418 or puromycin-resistance cassettes. The production rate from the pool of a co-transfection experiment performed separately is compared. Clones from this pool were isolated with production rates of 3-18pg/cell/day. However, clones above 5pg/cell/day were unstable and rapidly decreased in expression or stopped producing. Clones expressing approx. 5pg/cell/day were used for initial fermentation experiments. These preliminary indications are very encouraging that higher production rates will be observed in clones isolated from the bi-directional UCOE vector transfectants.

Table 1: Expression of hAb1 (IgG4) from Bi-directional UCOE vectors (CHO-K1 pools)

**CHO-K1 POOLS**

| *Vector* (pg/cell/day) | *H3 Promoter* | *K1 Promoter* | Production Rate |
|---|---|---|---|
| pBDUneo112 | murine CMV | human CMV | 0.3 |
| pBDUneo121 | human CMV | murine CMV | 1.5 |
| pBDUneo212 | murine CMV | human beta-actin | 0.06 |
| pBDUneo221 | human beta-actin | murine CMV | 1.3 |
| pBDUpuro312 | murine CMV | human CMV | 0.5 |
| pBDUpuro321 | human CMV | murine CMV | 1.4 |
| pBDUpuro412 | murine CMV | human beta-actin | 0.05 |
| pBDUpuro421 | human beta-actin | murine CMV | 2.3 |
| Cotransfection** | human CMV | human CMV | 0.7 |

**Contransfection was carried out previously using the same antibody genes each driven from 4kb UCOE CMV vectors (hydro and neo selection).

## REFERENCES

1. Dillon, N. & Grosveld, F. Chromatin domains as potential units of eukaryotic gene function. Curr. Opin, Genet. Dev. 4, 260-264 (1994).

2. Higgs, D.R. Do LCRs open chromatin domains? Cell 95,299-302 (1998).

3. Palmiter, R.D. & Brinster, R.L. Germline transformation of mice. Ann. Ref. Genet. 20, 465-499 (1986).

4. Allen, N.D. et al. Transgenes as probes for active chromosomal domains in mouse development. Nature 333, 852-855 (1988).

5. Bonnerot, C., Grimber, G., Briand, P. & Nicolas, J.F. Patterns of expression of position-dependent integrated transgenes in mouse embryo. Proc. Natl. Acad. Sci. USA 87:6331-6335 (1990).

6. Kioussis, D. & Festenstein, R. Locus control regions: overcoming heterochromatin-induced gene inactivation in mammals. Curr. Opin. Genet. Dev. 7,614-619 (1997).

7. Pikaart, M.J., Recillas-Targa, F. & Felsenfield, G. Loss of transcriptional activity of a transgene is accompanied by DNA methylation and histone deacetylation and is prevented by insulators. Genes Dev. 12, 2852-2862 (1998).

8. Fussenegger, M., Bailey, J.E., Hauser, H. & Mueller, P.P Genetic optimization of recombinant glycoprotein production by mammalian cells. Trends Biotech. 17,35-42 (1999).

9. Fraser, P. & Grosveld, F. Locus control regions; chromatin activation and transcription. Curr. Opin. Cell Biol. 10, 361-365 (1998).

10. Li, Q., Harju, S. & Peterson, K.R. Locus Control Regions: coming of age at a decade plus. Trends Genet. 15: 403-408 (1999).

11. Bulger, M. & Groudine, M. Looping versus linking: toward a model for long-distance gene activation. Genes Dev. 13, 2465-2477 (1999).

12. Grosveld, F. Activation by locus control regions? Curr. Opin. Genet. Dev. 9 152-157(1999).

13. Bender, M.A., Bulger, M., Close, J. & Groudine, M. β-globin Gene Switching and Dnase I Sensitivity of the Endogenous β-globin Locus in Mice Do Not Require the Locus Control Region. Mol. Cell 5, 387-393 (2000).

14. Ortiz, B.D., Cado, D., Chen, V., Diaz, P.W. & Winoto, A. Adjacent DNA elements dominantly restrict the ubiquitous activity of a novel chromatin-opening region to specific tissues. EMBO J. 16, 5037-5045 (1997).

15. Ortiz, B.D., Cado, D. & Winoto, A. A new element within the T-cell receptor alpha locus required for tissue-specific locus control region activity. Mol. Cell. Biol. 19, 1901-1909 (1999).

16. Antequera, F. & Bird, A. Number of CpG islands and genes in human and mouse. Proc. Natl. Acad Sci. USA 90, 1195-11999 (1993).

17. Cross, S.H. & Bird, A.P. CpG islands and genes. Curr. Opin, Genet. Dev. 5, 309-314 (1995).

18. Tazi, J. & Bird, A. Alternative chromatin structure at CpG islands. Cell 60, 909-920 (1990).

19. Imbert, G., Trottier, Y., Bechmann, J., & Mandel, J.L. The gene for the TATA binding protein (TBP) that contains a highly polymorphic protein coding CAG repeat maps to 6q27. Genomics 21: 667-668 (1994).

20. Purrello, M. et al. Physical mapping at 6q27 of the locus for the TATA-box binding protein, the DNA bining subunit of TFIID and a component of SL1 and TFIIIB, strongly suggests that it is single copy in the human genome. Genomics 22, 94-100 (1994).

21. Trachtulec, Z et al. Linkage of TATA-binding protein and proteasome subunit C5 genes in mice and humans reveals synteny conserved between mammals and invertebrates. Genomics 44:1-7 (1997).

22. Owens, G.P., Hahan, W.E., & Cohen, J.J. Identification of mRNAs associated with programmed cell death in immature thymocytes. Mol. Cell. Biol. 11, 4177-4188 (1991).

23. Chalut, C., Gallois, Y., Poterszman, A., Moncollin, V. & Egly, J.-M. Genomic structure of the human TATA-box-binding protein (TBP). Gene 161, 277-282 (1995).

24. Schmidt, E.E. & Schibler, U. High accumulation of components of the RNA polymerase II transcription machinery in rodent spermatids. Development 121,2373-2383 (1995).

25. Biamonti, G., Ruggiu, M., Saccone, S., Della Valle, G. & Riva, S. Two homologous genes, originated by duplication, encode the human hnRNP proteins A2 and A1. Nucl. Acids Res. 22, 1996-2002 (1994).

26. Kozu, T., Henrich, B. & Schafer, K.P. Structure and expression of the gene (HNRPA2B1) encoding the human hnRNP protein A2/B1. Genomics 25, 365-371 (1995).

27. Ye, Q. & Woman, H.J. Interaction between an integral protein of the nuclear envelope inner membrane and human chromodomain proteins homologous to Drosophila HP1. J. Biol. Chem. 271, 14653-14656 (1996).

28. James, T.C. & Elgin, S.C.R: Identification of a nonhistone chromosomal protein associated with heterochromatin in Drosophila melanogaster and its gene. Mol. Cell. Biol. 6,3861-3872 (1986).

29. Singh, P.B., et al. A sequence motif found in a drosophila heterochromatin protein is conserved in animals and plants. Nucl. Acids Res. 19, 789-794 (1991).

30. Gilliand, G., Perrin,m S., Blanchard, K. & Bunn, H.F, Analysis of cytokine Mrna and DNA: detection and quantitation by competitive polymerase chain reaction. Proc. Natl. Acad. Sci. USA 87, 2725-2729 (1990).

31. Furth, P.A. Hennighausen, L., Baker, C., Beatty, B. & Woychick, R. The viarability in activity of the universally expressed human cytomegalovirus immediate early gene 1 promoter/enhancer in transgenic mice. Nucl. Acids Res. 19 6205-6208 (1991).

32. Ray, P. et al. Ectopic expression of a c-kit W42 minigene in transgenic mice: recapitulation of W phenotypes and evidence for c-kit function in melanoblast progenitors. Genes Dev. 5, 2265-2273 (1991).

33. Yamashita, T. et al High level expression of human $\alpha$-fetoprotoin in transgenic mice. Biochem, Biophys, Res, Comm. 191, 715-720 (1993).

34. Milot, E. et al. Heterochromatin effects on the frequency and duration of LCR-mediated gene transcription. Cell 87, 104-114 (1996).

35. Festenstein, R. et al. Locus control region function and heterochromatin-induced position effect variegation. Science 271, 1123-1125 (1996).

36. Sabbattini, P., Georgiou, A., Sinclaire, C. & Dillon, N. Analysis of mice with single and multiple copies of transgenes reveals a novel arrangement for the λ5-V-preBI locus control region. Mol. Cell. Biol. 19, 671-679 (1999).

37. Ng, S.Y. et al. Evolution of the functional human ß-actin gene and its multi-pseudogene family: conservation of noncoding regions and chromosomal dispersion of pseudogenes. Mol. Cell. Biol. 5, 2720-2732 (1985).

38. Pravtcheva, D.D., Wise, T.L., Ensor, N.J. & Ruddle, F.H. Mosaic expression of an HPRT transgene integrated in a region of Y heterochromatin. J. Exp. Zool, 268,452-468 (1994).

39. Bell, A.C. & Felsenfield, G. Stopped at the border: boundaries and insulators. Curr. Opin. Genet. Dev. 9, 191-198 (1999).

40. Winston, J.H., Hong, L., Datta, S.K. & Kellems, R.E. An intron 1 regulatory region from the murine adenosine deaminase gene can activate Heterologous promoters for ubiquitous expression in transgenic mice. Somat, Cell Mol. Genet. 22, 261-278 (1996).

41. Hart, C.M. & Laemmli, U.K. Facilitation of chromatin dynamics by SARs. Curr. Opin. Genet. Develop. 8,519-525 (1998).

42. Klehr, D., Maass, K. & Bode, J. Scaffold-attached regions from the human interferon ß domain can be used to enhance the stable expression of genes under the control of various promoters. Biochemistry 30, 1264-1270 (1991).

43. McKnight, R.A., Shamay, L., Sankaran, L., Wall, R.J. & Henninghausen, L. Matrix-attachment regions can impart position-independent regulation of a tissue-specific gene in transgenic mice. Proc. Natl. Acad. Sci. USA 89, 6943-6947 (1992).

44. Van Drunen, C.M., et al. A bipartite sequence element associated with matrix/scaffold attachment regions. Nucl. Acids Res. 27, 2924-2930 (1999).

45. Verma, I.M. & Somia, N. Gene Therapy - promises, problems and prospects. Nature 389: 239-242 (1997).

46. Brown, S.A, & Kingston, R.E. Disruption of downstream chromatin directed by a transcriptional activator. Genes Dev. 11.3 116-3121 (1997).

47. Orphanides, G., LeRoy, G., Chang, C.H., Luse, D.S. & Reinberg, D. FACT, a factor that facilitates transcript elongation through nucleosomes. Cell92, 105-116 (1998).

48. Schnitzler, G., Sif, S. & Kingston, R.E. Human SWI/SNP interconverts a nucleosome between its base state and a stable remodelled state. Cell 94, 17-. 27(1998).

49. Travers, A. Chromatin modification by DNA tracking. Proc. Natl. Acad. Sci. USA 96,13634-13637 (1999).

50. Ashe, H.L., Monks, J., Wijgerde, M., Fraser, P. & Proudfoot, N.J. Intergenic transcription and transinduction of the human ß-globin locus. Genes Dev 11, 2494-2509 (1997).

51. Rogan, D.F., Cousins, D.J. & Staynov, D.Z. Intergenic transcription occurs throughout the human IL-4/IL-13 gene cluster. Biochem, Biophys, Res. Commun. 255,556-561 (1999).

52. Gribnau, J., Diderich, K., Pruzina, S., Calzorlari, R. & Fraser P. Intergenic Transcription and Developmental Remodeling of Chromatin Subdomains in the Human ß-globin Locus. Mol. Cell 5, 377-386 (2000).

53. Vyas, P. et al. Cis-acting sequences regulating expression of the human α-globin cluster lie within constitutively open Chromatin. Cell 69,781-793 (1992).

54. Ioannou, P.A. et al. A new bacteriophage' P1-derrived vector for the propagation of large human DNA fragments. Nature Gene. 6, 84-89 (1994).

55. Raguz, S. et al. Muscle-specific locus control region activity associated with the human desmin gene. Dev. Biol, 201,26-42 (1998).

56. Dillon, N. & Grosveld, F. Transcriptional analysis using transgenic animals, in Gene Transcription: A practical approach (eds Hames, B. D. & Higgins, S.J.) 153-188 (IRL Press, Oxford, 1993).

57. Morgenstern, J.P. & Land, H. Advanced mammalian gene transfer: high titre retroviral vectors with multiple drug selection markers and a complementary helper-free packaging cell line. Nucl. Acids Res 18, 3587-3595 (1990).

58. Horz, W. & Altenburger, W. Nucleotide sequence of mouse satellite DNA. Nucl. Acids Res. 9, 683-696 (1981).

59. Monfouilloux, S. et al. Recent human-specific spreading of a subtelomeric domain. Genomics 51, 165-176 (1998).

The following section of the description consists of numbered paragraphs simply providing statements of the Invention already described herein. The numbered paragraphs in this section are not claims. The claims are set forth below in the later section headed "claims".

1. A polynucleotide comprising a UCOE, which opens chromatin or maintains chromatin in an open state and facilitates reproducible expression of an operably-linked gene in cells of at least two different tissue types, wherein the nucleotide sequence of the UCOE does not occur in nature.

2. The polynucleotide of 1, which facilitates reproducible expression of an operably-linked gene non-tissue specifically.

3. The polynucleotide of 1, which facilitates reproducible expression of an operably-linked gene in all tissue types where active gene expression occurs.

4. The polynucleotide of any one of the preceding claims which facilitates expression of an operably-linked gene at a physiological level.

5. The polynucleotide of any one of the preceding claims wherein the' UCOE comprises an extended methylation-free, CpG-island.

6. The polynucleotide of any one of the preceding claims wherein the UCOE comprises one or more naturally-occurring sequences associated with the control of gene expression.

7. The polynucleotide of any one of the preceding claims wherein the UCOE comprises one or more naturally-occurring promoters.

8. The polynucleotide of any one of the preceding claims wherein the UCOE comprises dual or bi-directional promoters that transcribe divergently.

9. The polynucleotide of any one of the preceding claims wherein the UCOE comprises the human ß-actin CpG island/ promoter region or a fragment thereof.

10. The polynucleotide of . 9 wherein the UCOE comprises a DNA fragment within the range of 100bp to 3.0 kb spanning the human ß-actin CpG island/ promoter region or a fragment thereof,

11. The polynucleotide of any one of the preceding claims wherein the UCOE comprises the human PDCD2 CpG island/promoter region or a fragment thereof.

12. The polynucleotide of 11 wherein the UCOE comprises a DNA fragment within the range of 100bp to 3.0 kb spanning the human PDCD2 CpG island/ promoter region or a fragment thereof.

13. The polynucleotide of any one of the preceding ,wherein the UCOE comprises a DNA fragment within the range of 100bp to 3.0 kb spanning the human ß-actin CpG island/ promoter region and a DNA fragment within the range of 100bp to 3.0 kb spanning the human PDCD2 CpG island/ promoter region.

14. The polynucleotide of any one of the preceding , wherein the UCOE , comprises a 2.0 kb DNA fragment spanning the human ß-actin CpG island/ promoter region and a 1.8 kb DNA fragment spanning the human PDCD2 CpG island/ promoter region.

15. The polynucleotide of 13 or 14 wherein the promoters are orientated divergently,

16. The polynucleotide of any of the preceeding claims wherein the UCOE comprises the sequence of Figure 2 or a fragment thereof in either orientation.

17. The polynucleotide of any of the preceeding claims wherein the UCOE comprises the human RNP CpG island / promoter region or a fragment thereof.

18. The polynucleotide of 17 wherein the UCOE, comprises a 4kb DNA fragment spanning the human RNP CpG island / promoter region.

19. A polynucleotide of any of the preceding comprising an extended methylation-free CpG island containing bidivergent promoters adjacent to at least one further sequence comprising a methylation-free CpG island.

20. The polynucleotide of any of the preceding , wherein the UCOE comprises the human RNP CpG island /promoter region and a DNA fragment in the range 100bp to 3.0kb spanning the human ß-actin CpG island / promoter region.

21. The polynucleotide of 20, wherein the UCOE comprises the sequence of Figure 7 or fragment thereof in either orientation.

22. The polynucleotide of any one of the previous ., further comprising a promoter.

23. The polynucleotide of any one of the previous wherein the UCOE comprises one or more promoter sequences that are mutated in such a way that they are incapable of initiating transcription.

24. The polynucleotide of 22 or 23 wherein the promoter is the CMV promoter.

25. The polynucleotide of 24 wherein the promoter is the mouse CMV promoter.

26. The polynucleotide of any one of the previous wherein the UCOE comprises at least one sequence which is artificially synthesised.

27. A vector comprising the polynucleotide of any one of the preceding

28. The vector of 27, which additionally comprises an expressible gene operably-linked to a promoter and the polynucleotide.

29. The vector of 27 or 28 wherein the vector is an episomal or integrating vector.

30. The vector according to any of 27 to 29 wherein the vector is a plasmid.

31. The vector according to any of 27 to 29 wherein the vector is a virus.

32. The vector according to any 27 to 31 wherein the operably-linked gene is a therapeutic nucleic acid sequence.

33. The vector according to any of 27 to 32 wherein the vector comprises two sites for insertion of open reading frames to be expressed, each transcribed from a distinct promoter, said promoters being arranged so as to transcribe divergently and both being operably-linked to an artifically-constructed UCOE situated between them, and wherein said UCOE has been so constructed as to be effective in both orientations.

34. The vector of 33, wherein one of the two sites for insertion of open reading frames to be expressed comprises a nucleic acid encoding an immunoglobulin heavy chain and the other site comprises an immunoglobulin light chain.

35. The vector of any of 27 to 34 comprising the sequence of Figure 2, the CMV promoter, a multiple cloning site, a polyadenylation sequence and genes encoding selectable markers under suitable control elements.

36. The vector of any of 27 to 34 comprising the sequence of Figure 7, the CMV promoter, a multiple cloning site, a polyadenylation sequence and genes encoding selectable markers under suitable control elements.

37. The vector of 35 or 36, wherein the orientation of the UCOE is reversed.

38. Vector CET 500 as shown schematically in Figure 3a.

39. Vector CET 501 as shown schematically in Figure 3b

40. Vector CET 600 as shown schematically in Figure 8a

41. Vector CET 601 as shown schematically in Figure 8b

42. A host cell transfected with the vector of any one of 27 to 41.

43. The polynucleotide of any one of 1 to 26, the vector of any one of 27 to 41 or the host cell of 42 for use in therapy.

44. Use of the polynucleotide of any one of 1 to 26, the vector of any one of 27 to 41 or the host cell of . 42 for the manufacture of a composition for use in gene therapy.

45. A method of treatment, comprising administering to a patient in need of such treatment *a* pharmaceutically effective amount of the polynucleotide of any one of 1 to 26, the vector of any one of 27 to 41 or the host cell of 42.

46. A pharmaceutical composition comprising the polynucleotide of any one of 1 to 26, and/or the vector of any one of 27 to 41 and/or the host cell of 42 in combination with a pharmaceutically acceptable excipient.

47. Use of the polynucleotide of any one of 1 to 26, and/or the vector of any one of 27 to 41 and/or the host cell of 42 in a cell culture system in order to obtain a desired gene product.

48. Use of the polynucleotide of any one of 1 to 26 to increase the expression of an endogenous gene comprising inserting the polynucleotide into the genome of a cell in a position operably associated with the endogenous gene thereby increasing the level of expression of the gene.

49. Use of the polynucleotide of any of 1 to 26 in producing transgenic plants.

50. A transgenic plant containing cells which contain the polynucleotide of any one of 1 to 26,

51. A transgenic non-human animal containing cells which contain the polynucleotide of any one of 1 to 26.

52. Use of the polynucleotide of any one of 1 to 26 to obtain expression of an antisense gene sequence to inactivate expression of the corresponding endogenous gene sequence.

53. Use of the polynucleotide of any one of 1 to 26 in the preparation of an expression library.

54. Use of the polynucleotide of any one of 1 to 26 in a method for identifying expressible genes in a non-human animal comprising inserting a construct comprising the polynucleotide into embryonic stem cells of the non-human animal wherein the construct only allows drug selection following insertion into expressed genes.

55. A nucleic acid molecule comprising a DNA sequence selected from:

(i)the DNA sequence as represented in Figure 2 or Figure 7;

(ii) DNA sequences which hybridise to the sequence presented in Figure 2 or Figure 7 which encode a polypeptide according to the invention.

56. An isolated nucleic acid molecule which anneals under stringent hybridisation conditions to the sequence presented in Figure 2 or Figure 7.

57. A method for the production of the polypeptide according to the invention comprising:

(1) providing a cell transformed/transieoted with a nucleic acid molecule according to claim 55 or claim 56;

(ii) growing said cell in conditions conducive to the production of said polypeptide; and

(iii) purifying said polypeptide from said cell, or its growth environment.

## Claims

1. A chimeric polynucleotide comprising two linked extended methylation-free CpG islands from different housekeeping genes, wherein each extended methylation-free CpG island comprises 300bp and a promoter, wherein the promoters of the linked extended methylation-free CpG islands are orientated to drive divergent transcription.

2. The polynucleotide of claim 1, wherein the nucleotide sequence comprises a DNA fragment within the range of 300bp to 3.0kb of the human programmed cell death-2 (PDCD2) gene CpG island/promoter region.

3. The polynucleotide of claim 1, wherein the nucleotide sequence comprises a DNA fragment within the range of 300bp to 3.0kb of the human β-actin gene CpG island/promoter region.

**4.** The polynucleotide of claim 1, wherein the nucleotide sequence comprises a DNA fragment within the range of 300bp to 3.0kb of the human β-actin gene CpG island/promoter region and a DNA fragment within the range of 300bp to 3.0kb of the human programmed cell death-2 (PDCD2) gene CpG island/promoter region.

**5.** The polynucleotide of claim 1, wherein the nucleotide sequence comprises a 4kb DNA fragment of the human ribonucleoprotein (RNP) gene CpG island/promoter region.

**6.** The polynucleotide of claim 1, wherein the nucleotide comprises a human ribonucleoprotein (RNP) gene CpG island/promoter region and a DNA fragment in the range of 300bp to 3.0kb of the human β-actin CpG island/promoter region.

**7.** The polynucleotide of any of claims 1 to 6, wherein the nucleotide sequence further comprises a promoter selected from: cytomegalovirus (CMV), elongation factor-1 alpha (EF-1α), respiratory syncytial virus (RSV) LTR and human immunodeficiency 2 (HIV2) LTR or combinations thereof.

**8.** The polynucleotide of claim 7, wherein the CMV promoter is a mouse CMV promoter.

**9.** The polynucleotide of claim 1, wherein the nucleotide sequence comprises a 2kb fragment comprising a CpG island from the 5' end of the human β-actin gene and a 3.2kb fragment comprising a CpG island from the 5' end of the human programmed cell death-2 gene.

**10.** The polynucleotide of claim 9, wherein the nucleotide sequence further comprises a CMV promoter.

**11.** The polynucleotide of claim 1, wherein the nucleotide sequence comprises the sequence set forth in SEQ ID NO: 1 or a fragment thereof, or in SEQ ID NO:2 or a fragment thereof, capable of opening chromatin or maintaining chromatin in an open state.

**12.** A vector comprising the polynucleotide of any of claims 1 to 11.

**13.** A host cell comprising the vector of claim 12.

**14.** A polynucleotide according to any of claims 1 to 11 for use as a medicament.

**15.** A method for removing a CMV promoter from pEGFP-N2 comprising digesting the pEGFP-N2 with *AseI* and *NheI* and blunting the digested pEGFP-N2 with T4 DNA polymerase to produce pA-EGFP-N1.

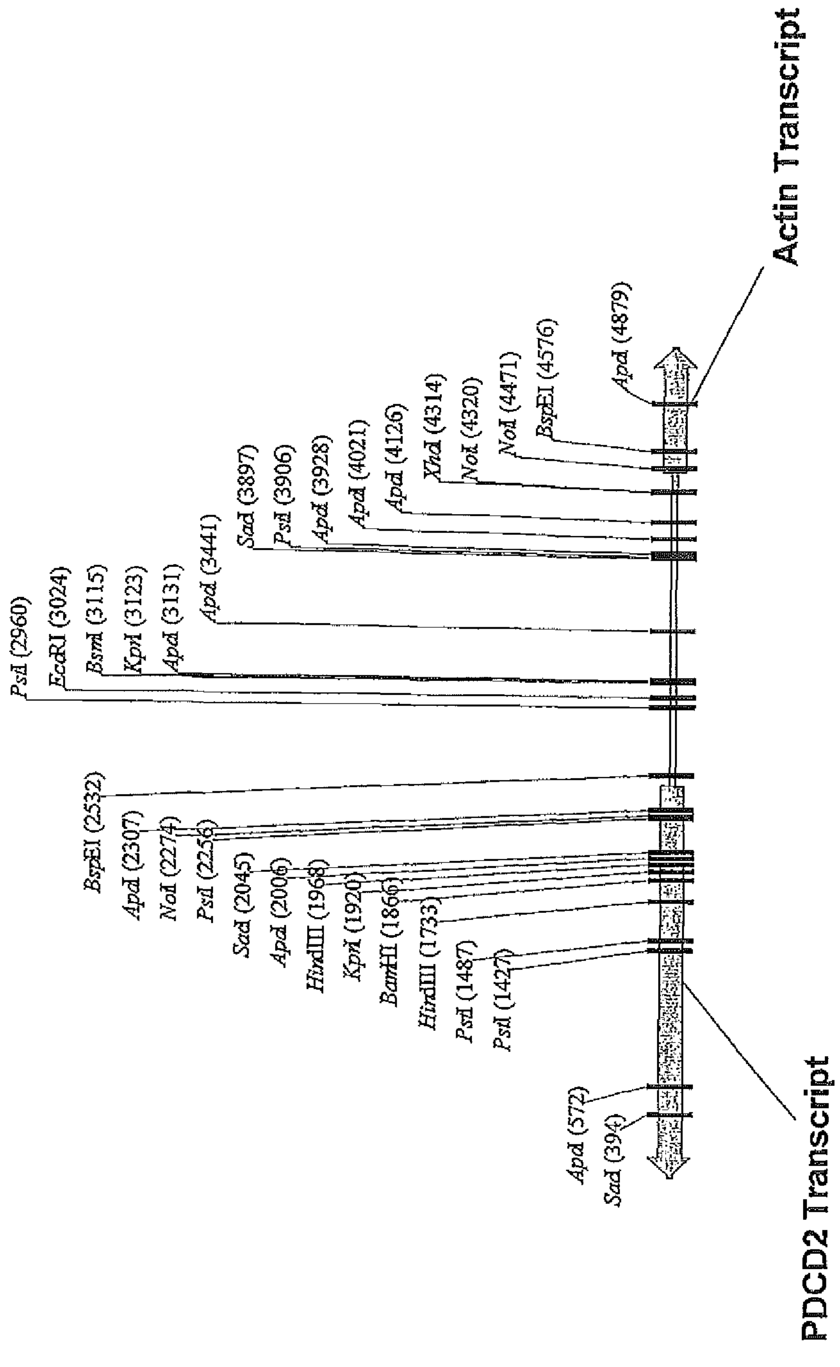


Fig 1: ARTIFICIAL UCOE Restriction map (Nco I fragment)

5232 bp

```
   1 CATGGCACCT GTATTGTACT CTTATCAGTC ATTATATGGA CTTTAACTTC
     GTACCGTGGA CATAACATGA GAATAGTCAG TAATATACCT GAAATTGAAG
  51 CCCAGATATT ATTTGGGCTC CTCCATAAGA CTGTGAGCAT CTGACCACTG
     GGGTCTATAA TAAACCCGAG GAGGTATTCT GACACTCGTA GACTGGTGAC
 101 GAGTGTTGCT TCCCATTATA TCCCTGTTAT CAAGCACAAG GTCAGGCACA
     CTCACAACGA AGGGTAATAT AGGGACAATA GTTCGTGTTC CAGTCCGTGT
 151 GAGTAAGACT CAAAACATGT TTTGGAATGT ATGACTGGTA TGAACTACAA
     CTCATTCTGA GTTTTGTACA AAACCTTACA TACTGACCAT ACTTGATGTT
 201 ACCAGTAAGC TGATGTTTTC ATTTTGAGTC TATAAATCTA ATTTTGTGGT
     TGGTCATTCG ACTACAAAAG TAAAACTCAG ATATTTAGAT TAAAACACCA
 251 GGTTTTGTGT ATGGCTCAAG GCTCAAATTG TAAAATTTAA TATTATGTGA
     CCAAAACACA TACCGAGTTC CGAGTTTAAC ATTTTAAATT ATAATACACT
 301 CCAAAGAAAG TTATACCCAG AACCTCAATT TCCTCACCTT CAAAATGGGG
     GGTTTCTTTC AATATGGGTC TTGGAGTTAA AGGAGTGGAA GTTTTACCCC
 351 CAGTTTCTCA CTCATTGGTC TGCTGTCACG ATTTTAATGA GCTCATGCAC
     GTCAAAGAGT GAGTAACCAG ACGACAGTGC TAAAATTACT CGAGTACGTG
 401 AAACAGCCCT TTATATAAGG TAAGTGCTGG ATAAATGTTG GCTACTATAA
     TTTGTCGGGA AATATATTCC ATTCACGACC TATTTACAAC CGATGATATT
 451 TAAAATAAGC CTCTAAGATA CTTGGTCAGC ACAAGTACTA CCCAAGAGTA
     ATTTTATTCG GAGATTCTAT GAACCAGTCG TGTTCATGAT GGGTTCTCAT
 501 TGCACTGTAA GTAAACTGAC AAAATTGTGT ATCTAAAACT GGCCAGATGA
     ACGTGACATT CATTTGACTG TTTTAACACA TAGATTTTGA CCGGTCTACT
 551 AAGAGAAACT TTTAAGGGGC CCTTCTGCGT GCCCGACACT GTGCTAGGCA
     TTCTCTTTGA AAATTCCCCG GGAAGACGCA CGGGCTGTGA CACGATCCGT
 601 CTCACACTAT CCCGACCCGA GAAACCGATC TGCGACCCAG AGGAACTTAC
     GAGTGTGATA GGGCTGGGCT CTTTGGCTAG ACGCTGGGTC TCCTTGAATG
 651 CAAGCCTCCA GCATCTTGTG CAGCCCTACT CATGGGACCA TCTGGATACC
     GTTCGGAGGT CGTAGAACAC GTCGGGATGA GTACCCTGGT AGACCTATGG
 701 CACCCTTGTC TTTACAGGGA GCAGAACACA CCTCTTATGT GTCAGAAAAC
     GTGGGAACAG AAATGTCCCT CGTCTTGTGT GGAGAATACA CAGTCTTTTG
 751 AAAGTCCAGG AAGTATATTT TTACCTGAGG CAATATCTGA AAATTGTATG
     TTTCAGGTCC TTCATATAAA AATGGACTCC GTTATAGACT TTTAACATAC
 801 CTACAGCCTC CAAAGTGAGT CTTCCTCTCA GTACCTCTCT TCTAGGCACA
     GATGTCGGAG GTTTCACTCA GAAGGAGAGT CATGGAGAGA AGATCCGTGT
 851 TGGAGCCCTT TCTTCCAAGT ATTATGTTTA ACCACTTAAT GAATGAAGTC
     ACCTCGGGAA AGAAGGTTCA TAATACAAAT TGGTGAATTA CTTACTTCAG
 901 CTGAAACTGC TTACCCATGC TCCCTATAAT CTCTGAGTAA TCTTCCTTTT
     GACTTTGACG AATGGGTACG AGGGATATTA GAGACTCATT AGAAGGAAAA
 951 CCACAACCTC AGGCATAATC TCATCTTCTG TTTCTATTAC AATTTCAAAT
     GGTGTTGGAG TCCGTATTAG AGTAGAAGAC AAAGATAATG TTAAAGTTTA
1001 TCTGGAAAAA GGAAGTTGTG GTCTGGAATT ATATGGTCCA GATGATCTGA
     AGACCTTTTT CCTTCAACAC CAGACCTTAA TATACCAGGT CTACTAGACT
1051 AACAAAAAGG ACAGCACTAT TAGTAATCAT TTAGTTTTGA AGACAGTCTA
     TTGTTTTTCC TGTCGTGATA ATCATTAGTA AATCAAAACT TCTGTCAGAT
1101 ATAATTTGCT GTCTCTAAAG TACTATATTC CCTATAGTTC TGGCATTTTA
     TATTAAACGA CAGAGATTTC ATGATATAAG GGATATCAAG ACCGTAAAAT
1151 GATAAAGGGT CATAAATTAA ATGCCTATAT GGTGACATTA TTCAGTGATT
     CTATTTCCCA GTATTTAATT TACGGATATA CCACTGTAAT AAGTCACTAA
1201 CAGACTTCAC AGCCTTTTTT TTTTTTTTAC AAAGGTGTTC CAGGCATGAA
     GTCTGAAGTG TCGGAAAAAA AAAAAAAATG TTTCCACAAG GTCCGTACTT
1251 AAATTTTAAA GTACTATACC TTTCCTAATT TTACCTTTAA AGTTGTCCTG
     TTTAAAATTT CATGATATGG AAAGGATTAA AATGGAAATT TCAACAGGAC
1301 GAAATATCTG GGTTGACAAA GGCGATGAAA CTGAACTGAG ACTTAAAAAA
     CTTTATAGAC CCAACTGTTT CCGCTACTTT GACTTGACTC TGAATTTTTT
1351 AAGATTACCC ACCTGGTTGT GCACAAGCCT GCTTATGTCC CAATCTCCAG
     TTCTAATGGG TGGACCAACA CGTGTTCGGA CGAATACAGG GTTAGAGGTC
1401 TCTAGGGTCT GATGCTCCTT GCTGCAGTAA TATGCTTTGT GGCATCTGGA
     AGATCCCAGA CTACGAGGAA CGACGTCATT ATACGAAACA CCGTAGACCT
```

FIG 2: PDCD2/ACTIN ARTIFICIAL UCOE SEQUENCE

```
1451   GCACGTTTTG GGGCCTAAAC AGCCACAAAC CCTGCAGAGA TGAGCACCAG
       CGTGCAAAAC CCCGGATTTG TCGGTGTTTG GGACGTCTCT ACTCGTGGTC
1501   ACTTAAGCTG GAGACACACT GATTCTCCTG TTTCTGGGGG AGGATTCTCA
       TGAATTCGAC CTCTGTGTGA CTAAGAGGAC AAAGACCCCC TCCTAAGAGT
1551   GAAGGTGGCT CATATGAGTA AAAATCGTTT TTCCTGGGTA GTTGATTCCT
       CTTCCACCGA GTATACTCAT TTTTAGCAAA AAGGACCCAT CAACTAAGGA
1601   AAAAACTAAA AAAGAATACA GAGAAAAGTT TTATCTTCAA ACAAAACAGC
       TTTTTGATTT TTTCTTATGT CTCTTTTCAA AATAGAAGTT TGTTTTGTCG
1651   AATTCACATA TTTTATCCTC TGCACGTAAA ACTGAAAATA ACAACAACAA
       TTAAGTGTAT AAAATAGGAG ACGTGCATTT TGACTTTTAT TGTTGTTGTT
1701   AAAAGAAATG AAAGTTTTTG CTTTCAGGAA TAAGCTTTTA AAATCCAGAA
       TTTTCTTTAC TTTCAAAAAC GAAAGTCCTT ATTCGAAAAT TTTAGGTCTT
1751   ACTAGATTTC GTCCGGTACA CGCAACTGAG TTGCCTCCTA GAGGTGGTTT
       TGATCTAAAG CAGGCCATGT GCGTTGACTC AACGGAGGAT CTCCACCAAA
1801   GAGTTAATCA AATTAATAAG ACTGATCGTT AAGAACGACT GCCAAAAATA
       CTCAATTAGT TTAATTATTC TGACTAGCAA TTCTTGCTGA CGGTTTTTAT
1851   CGAAAAAGCT ACTGGGATCC ATCTTTCCAA GACAATTTCT ATTATCTGAA
       GCTTTTTCGA TGACCCTAGG TAGAAAGGTT CTGTTAAAGA TAATAGACTT
1901   TTAACACCAT ACCTGGTACC CACTGATTAA AAGCTGGGGG TTACCAATGC
       AATTGTGGTA TGGACCATGG GTGACTAATT TTCGACCCCC AATGGTTACG
1951   GCGTGGGCAC AGTTAGAAGC TTATGTAGCA AAAATGAGCA CATCCTGGAA
       CGCACCCGTG TCAATCTTCG AATACATCGT TTTTACTCGT GTAGGACCTT
2001   GGGCCCGGGA GAAGGTGCTC CTGGGGCAGC GCGGAGAGGG AGCTCTGAGG
       CCCGGGCCCT CTTCCACGAG GACCCCGTCG CGCCTCTCCC TCGAGACTCC
2051   CTGGGGCGGC AGCGGTGCTT GCCGCCGTCC CCCTGGTCGC TCCCGGAATT
       GACCCCGCCG TCGCCACGAA CGGCGGCAGG GGGACCAGCG AGGGCCTTAA
2101   AACGCCGCGC ACGCGTCGGA GGCATGGCCC CGTCCCGACC CCGTTTGGCG
       TTGCGGCGCG TGCGCAGCCT CCGTACCGGG GCAGGGCTGG GGCAAACCGC
2151   GCTCACCTCG CAGGCCGGCA CAGCACGGCT GCTCGCGGCA GCAGAAGAGG
       CGAGTGGAGC GTCCGGCCGT GTCGTGCCGA CGAGCGCCGT CGTCTTCTCC
2201   AAGATGCAGC GGTGGAAGGC GTCCGGGCGG CCAGGCAGCG GCGCATACAC
       TTCTACGTCG CCACCTTCCG CAGGCCCGCC GGTCCGTCGC CGCGTATGTG
2251   CTGCAGCAGG AAGGAGAGCG GGCGGCCGCA CAGCTCGCAG GCCAGGGCCT
       GACGTCGTCC TTCCTCTCGC CCGCCGGCGT GTCGAGCGTC CGGTCCCGGA
2301   GGGGCCCCGG CAGCCCGGCC GCGCCCAGCC ATGCCGGCCG CCCGCCCACC
       CCCCGGGGCC GTCGGGCCGG CGCGGGTCGG TACGGCCGGC GGGCGGGTGG
2351   TTGCTGGGGA ACTGCTCGCT GCGCAGTCGC CACGCCGGCG CCGACTCGGC
       AACGACCCCT TGACGAGCGA CGCGTCAGCG GTGCGGCCGC GGCTGAGCCG
2401   GAAGCCCAGC TCCACAGGCC TGGCCCCGGC GGCAGCCATG CGGGGCGCGG
       CTTCGGGTCG AGGTGTCCGG ACCGGGGCCG CCGTCGGTAC GCCCCGCGCC
2451   GCTGGCGTGG GGCGCAGCCC ACAGCTGGGT CGGAAGGCGG AAATCGGGCG
       CGACCGCACC CCGCGTCGGG TGTCGACCCA GCCTTCCGCC TTTAGCCCGC
2501   CCGGGCCGGA AGGCAAGAGG CGGGCACCTT TCCGGAGGAC AGGAGGCGGA
       GGCCCGGCCT TCCGTTCTCC GCCCGTGGAA AGGCCTCCTG TCCTCCGCCT
2551   AACGCGTCTG ACGGGAGCGG TTGCAGGACC AATGCGAGGG AACGGGGCAG
       TTGCGCAGAC TGCCCTCGCC AACGTCCTGG TTACGCTCCC TTGCCCCGTC
2601   AGGAAACCTC TCGGCATCAG CCCCGCCCCT GGCGCCTCTG CCTCCGAGCC
       TCCTTTGGAG AGCCGTAGTC GGGGCGGGGA CCGCGGAGAC GGAGGCTCGG
2651   GCTTTCCTGG TGCCTCCGGG TGCTCTGGGA TGGTTCTGGT CTTTGGGAGA
       CGAAAGGACC ACGGAGGCCC ACGAGACCCT ACCAAGACCA GAAACCCTCT
2701   GTGGCAGCTG GTGACGGCGC TCCGCTCACC TCTGCACATG TCTTGCTGTG
       CACCGTCGAC CACTGCCGCG AGGCGAGTGG AGACGTGTAC AGAACGACAC
2751   GGCCTGCGGG TGGCCGCCAG GGAGGCAGAG CCCTCCCGCA AACCTTCCCT
       CCGGACGCCC ACCGGCGGTC CCTCCGTCTC GGGAGGGCGT TTGGAAGGGA
2801   GCTGGTGTCC ACCTCAGGGT GTGGGAAACC TGTGCGCTGG CCGAGTGCTA
       CGACCACAGG TGGAGTCCCA CACCCTTTGG ACACGCGACC GGCTCACGAT
2851   ACCAAGAGTA GGCAGTGAAA GACAAATGAA GGTTGAACAG GTAAAGTGAG
       TGGTTCTCAT CCGTCACTTT CTGTTTACTT CCAACTTGTC CATTTCACTC
```

```
2901  GACCCTACAG CGGAAACCAA GAATCCTGTG TGCCTGAGAG TAATGAAGAA
      CTGGGATGTC GCCTTTGGTT CTTAGGACAC ACGGACTCTC ATTACTTCTT
2951  GCCTCTGCAG AAGAGTCTTT TCTGTCAGTC TTAAGGTCTC TGTTTTAATG
      CGGAGACGTC TTCTCAGAAA AGACAGTCAG AATTCCAGAG ACAAAATTAC
3001  TTAGTGCTGG CTTGCTGTAC CTGAATTCCA AGGGAGGAGT GTATAATGAG
      AATCACGACC GAACGACATG GACTTAAGGT TCCCTCCTCA CATATTACTC
3051  GCATGGCCAA CCCCCACTTC CCATCATTGC CTGAACTAGT TTTTCAGGTT
      CGTACCGGTT GGGGGTGAAG GGTAGTAACG GACTTGATCA AAAAGTCCAA
3101  AACTTCAGAA TGCCCTTGGT ACCGCGGGCC CCCTCTGTGG TCCCACGCCA
      TTGAAGTCTT ACGGGAACCA TGGCGCCCGG GGGAGACACC AGGGTGCGGT
3151  CTGATCGCTG CATGCCCACC ACCTGGGTAC ACACAGTCTG TGATTCCCGG
      GACTAGCGAC GTACGGGTGG TGGACCCATG TGTGTCAGAC ACTAAGGGCC
3201  AGCAGAACGG ACCCTGCCCA CCCGGTCTTG TGTGCTACTC AGTGGACAGA
      TCGTCTTGCC TGGGACGGGT GGGCCAGAAC ACACGATGAG TCACCTGTCT
3251  CCCAAGGCAA GAAAGGGTGA CAAGGACAGG GTCTTCCCAG GCTGGCTTTG
      GGGTTCCGTT CTTTCCCACT GTTCCTGTCC CAGAAGGGTC CGACCGAAAC
3301  AGTTCCTAGC ACCGCCCCGC CCCCAATCCT CTGTGGCACA TGGAGTCTTG
      TCAAGGATCG TGGCGGGGCG GGGGTTAGGA GACACCGTGT ACCTCAGAAC
3351  GTCCCCAGAG TCCCCCAGCG GCCTCCAGAT GGTCTGGGAG GGCAGTTCAG
      CAGGGGTCTC AGGGGGTCGC CGGAGGTCTA CCAGACCCTC CCGTCAAGTC
3401  CTGTGGCTGC GCATAGCAGA CATACAACGG ACGGTGGGCC CAGACCCAGG
      GACACCGACG CGTATCGTCT GTATGTTGCC TGCCACCCGG GTCTGGGTCC
3451  CTGTGTAGAC CCAGCCCCCC CGCCCCGCAG TGCCTAGGTC ACCCACTAAC
      GACACATCTG GGTCGGGGGG GCGGGGCGTC ACGGATCCAG TGGGTGATTG
3501  GCCCCAGGCC TGGTCTTGGC TGGGCGTGAC TGTTACCCTC AAAAGCAGGC
      CGGGGTCCGG ACCAGAACCG ACCCGCACTG ACAATGGGAG TTTTCGTCCG
3551  AGCTCCAGGG TAAAAGGTGC CCTGCCCTGT AGAGCCCACT TCCTTCCCAG
      TCGAGGTCCC ATTTTCCACG GGACGGGACA TCTCGGGTGA AGGAAGGGTC
3601  GGCTGCGGCT GGGTAGGTTT GTAGCCTTCA TCACGGGCCA CCTCCAGCCA
      CCGACGCCGA CCCATCCAAA CATCGGAAGT AGTGCCCGGT GGAGGTCGGT
3651  CTGGACCGCT GGCCCCTGCC CTGTCCTGGG GAGTGTGGTC CTGCGACTCT
      GACCTGGCGA CCGGGGACGG GACAGGACCC CTCACACCAG GACGCTGAGA
3701  AATGGCCGCA AGCCACCTGA CTCCCCCAAC ACCACACTCT ACCTCTCAAG
      TTACCGGCGT TCGGTGGACT GAGGGGGTTG TGGTGTGAGA TGGAGAGTTC
3751  CCCAGGTCTC TCCCTAGTGA CCCACCCAGC ACATTTAGCT AGCTGAGCCC
      GGGTCCAGAG AGGGATCACT GGGTGGGTCG TGTAAATCGA TCGACTCGGG
3801  CACAGCCAGA GGTCCTCAGG CCCTGCTTTC AGGGCAGTTG CTCTGAAGTC
      GTGTCGGTCT CCAGGAGTCC GGGACGAAAG TCCCGTCAAC GAGACTTCAG
3851  GGCAAGGGGG AGTGACTGCC TGGCCACTCC ATGCCCTCCA AGAGCTCCTT
      CCGTTCCCCC TCACTGACGG ACCGGTGAGG TACGGGAGGT TCTCGAGGAA
3901  CTGCAGGAGC GTACAGAACC CAGGGCCCTG GCACCCGTGC AGACCCTGGC
      GACGTCCTCG CATGTCTTGG GTCCCGGGAC CGTGGGCACG TCTGGGACCG
3951  CCACCCCACC TGGGCGCTCA GTGCCCAAGA GATGTCCACA CCTAGGATGT
      GGTGGGGTGG ACCCGCGAGT CACGGGTTCT CTACAGGTGT GGATCCTACA
4001  CCCGCGGTGG GTGGGGGGCC CGAGAGACGG GCAGGCCGGG GGCAGGCCTG
      GGGCGCCACC CACCCCCCGG GCTCTCTGCC CGTCCGGCCC CCGTCCGGAC
4051  GCCATGCGGG GCCGAACCGG GCACTGCCCA GCGTGGGGCG CGGGGGCCAC
      CGGTACGCCC CGGCTTGGCC CGTGACGGGT CGCACCCCGC GCCCCCGGTG
4101  GGCGCGCGCC CCCAGCCCCC GGGCCCAGCA CCCCAAGGCG GCCAACGCCA
      CCGCGCGCGG GGGTCGGGGG CCCGGGTCGT GGGGTTCCGC CGGTTGCGGT
4151  AAACTCTCCC TCCTCCTCTT CCTCAATCTC GCTCTCGCTC TTTTTTTTTT
      TTTGAGAGGG AGGAGGAGAA GGAGTTAGAG CGAGAGCGAG AAAAAAAAAA
4201  TCGCAAAAGG AGGGGAGAGG GGGTAAAAAA ATGCTGCACT GTGCGGCGAA
      AGCGTTTTCC TCCCCTCTCC CCCATTTTTT TACGACGTGA CACGCCGCTT
4251  GCCGGTGAGT GAGCGGCGCG GGGCCAATCA GCGTGCGCCG TTCCGAAAGT
      CGGCCACTCA CTCGCCGCGC CCCGGTTAGT CGCACGCGGC AAGGCTTTCA
4301  TGCCTTTTAT GGCTCGAGCG GCCGCGGCGG CGCCCTATAA AACCCAGCGG
      ACGGAAAATA CCGAGCTCGC CGGCGCCGCC GCGGGATATT TTGGGTCGCC
```

```
4351  CGCGACGCGC CACCACCGCC GAGACCGCGT CCGCCCGCGA GCACAGAGCC
      GCGCTGCGCG GTGGTGGCGG CTCTGGCGCA GGCGGGCGCT CGTGTCTCGG
4401  TCGCCTTTGC CGATCCGCCG CCCGTCCACA CCCGCCGCCA GGTAAGCCCG
      AGCGGAAACG GCTAGGCGGC GGGCAGGTGT GGGCGGCGGT CCATTCGGGC
4451  GCCAGCCGAC CGGGGCATGC GGCCGCGGCC CTTCGCCCGT GCAGAGCCGC
      CGGTCGGCTG GCCCCGTACG CCGGCGCCGG GAAGCGGGCA CGTCTCGGCG
4501  CGTCTGGGCC GCAGCGGGGG GCGCATGGGG CGGAACCGGA CCGCCGTGGG
      GCAGACCCGG CGTCGCCCCC CGCGTACCCC GCCTTGGCCT GGCGGCACCC
4551  GGGCGCGGGA GAAGCCCCTG GGCCTCCGGA GATGGGGGAC ACCCCACGCC
      CCCGCGCCCT CTTCGGGGAC CCGGAGGCCT CTACCCCCTG TGGGGTGCGG
4601  AGTTCGCAGG CGCGAGGCCG CGCTCGGGCG GGCGCGCTCC GGGGGTGCCG
      TCAAGCGTCC GCGCTCCGGC GCGAGCCCGC CCGCGCGAGG CCCCCACGGC
4651  CTCTCGGGGC GGGGGCAACC GGCGGGGTCT TTGTCTGAGC CGGGCTCTTG
      GAGAGCCCCG CCCCCGTTGG CCGCCCCAGA AACAGACTCG GCCCGAGAAC
4701  CCAATGGGGA TCGCACGGTG GGCGCGGCGT AGCCCCCGTC AGGCCCGGTG
      GGTTACCCCT AGCGTGCCAC CCGCGCCGCA TCGGGGGCAG TCCGGGCCAC
4751  GGGGCTGGGG CGCCATGCGC GTGCGCGCTG GTCCTTTGGG CGCTAACTGC
      CCCCGACCCC GCGGTACGCG CACGCGCGAC CAGGAAACCC GCGATTGACG
4801  GTGCGCGCTG GGAATTGGCG CTAATTGCGC GTGCGCGCTG GGACTCAATG
      CACGCGCGAC CCTTAACCGC GATTAACGCG CACGCGCGAC CCTGAGTTAC
4851  GCGCTAATCG CGCGTGCGTT CTGGGGCCCG GGCGCTTGCG CCACTTCCTG
      CGCGATTAGC GCGCACGCAA GACCCCGGGC CCGCGAACGC GGTGAAGGAC
4901  CCCGAGCCGC TGGCGCCCGA GGGTGTGGCC GCTGCGTGCG CGCGCGCGAC
      GGGCTCGGCG ACCGCGGGCT CCCACACCGG CGACGCACGC GCGCGCGCTG
4951  CCGGTCGCTG TTTGAACCGG GCGGAGGCGG GGCTGGCGCC CGGTTGGGAG
      GGCCAGCGAC AAACTTGGCC CGCCTCCGCC CCGACCGCGG GCCAACCCTC
5001  GGGGTTGGGG CCTGGCTTCC TGCCGCGCGC CGCGGGGACG CCTCCGACCA
      CCCCAACCCC GGACCGAAGG ACGGCGCGCG GCGCCCCTGC GGAGGCTGGT
5051  GTGTTTGCCT TTTATGGTAA TAACGCGGCC GGCCCGGCTT CCTTTGTCCC
      CACAAACGGA AAATACCATT ATTGCGCCGG CCGGGCCGAA GGAAACAGGG
5101  CAATCTGGGC GCGCGCCGGC GCCCCCTGGC GGCCTAAGGA CTCGGCGCGC
      GTTAGACCCG CGCGCGGCCG CGGGGGACCG CCGGATTCCT GAGCCGCGCG
5151  CGGAAGTGGC CAGGGCGGGG GCGACTTCGG CTCACAGCGC GCCCGGCTAT
      GCCTTCACCG GTCCCGCCCC CGCTGAAGCC GAGTGTCGCG CGGGCCGATA
5201  TCTCGCAGCT CACCATGCCG GTCGCCACCA TG
      AGAGCGTCGA GTGGTACGGC CAGCGGTGGT AC
```

Figure 3a:

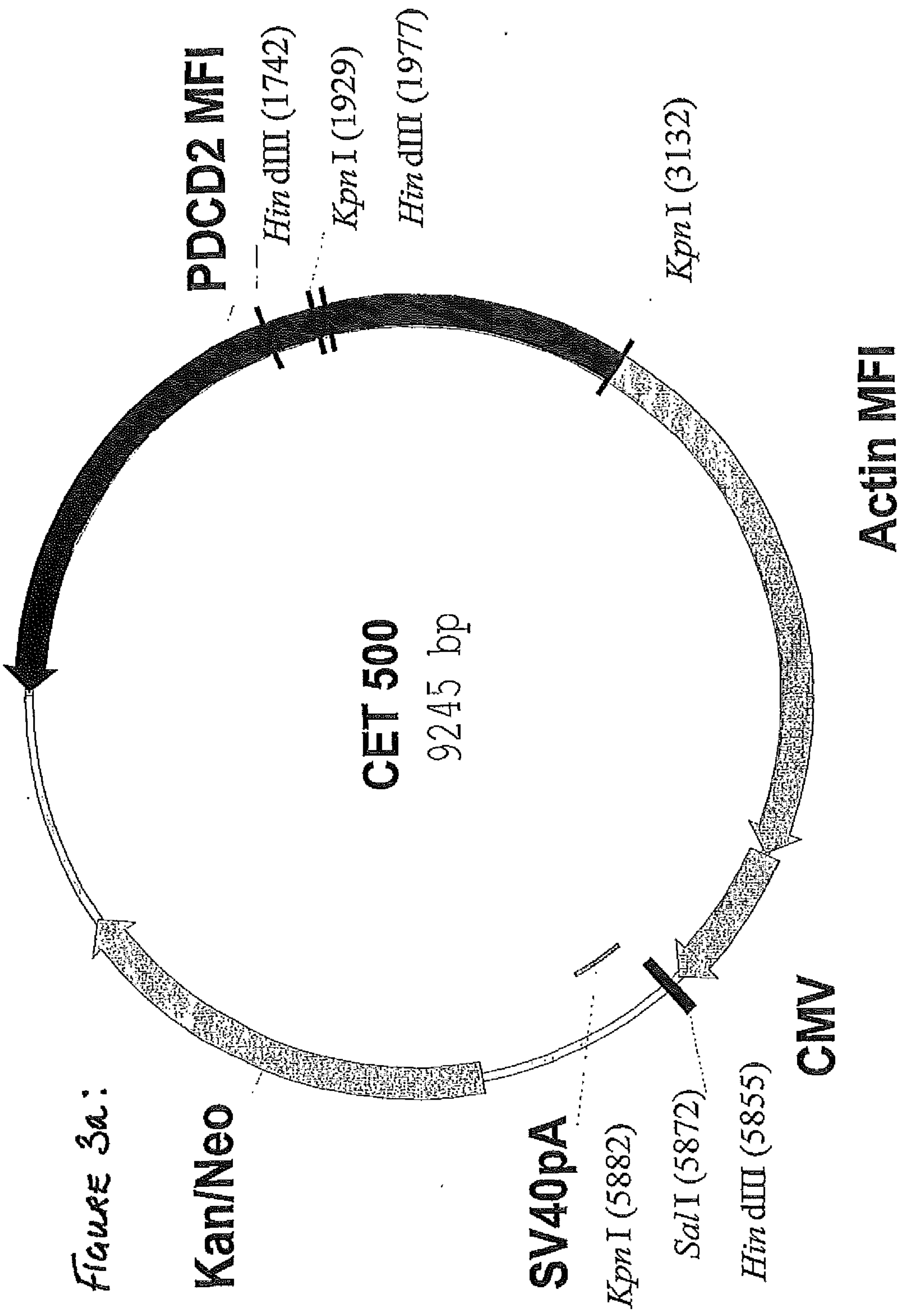
PDCD2 MFI
HindIII (1742)
KpnI (1929)
HindIII (1977)
KpnI (3132)
Actin MFI
CET 500
9245 bp
CMV
HindIII (5855)
SalI (5872)
KpnI (5882)
SV40pA
Kan/Neo

FIGURE 3b:

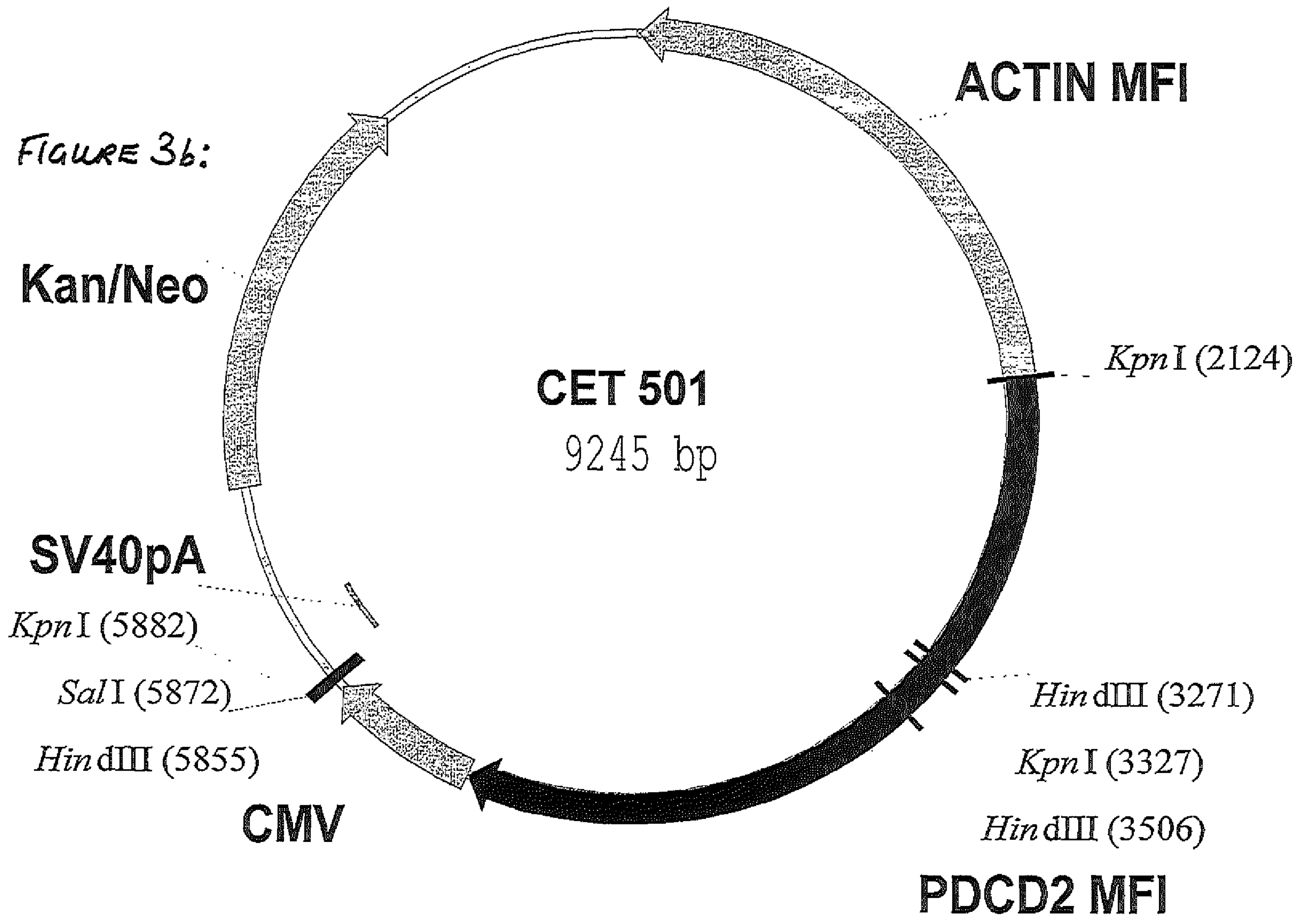
ACTIN MFI
Kan/Neo
CET 501
9245 bp
KpnI (2124)
SV40pA
KpnI (5882)
SalI (5872)
HindIII (5855)
CMV
HindIII (3271)
KpnI (3327)
HindIII (3506)
PDCD2 MFI

Figure 4
CET220 vs PDCD2/actin artificial UCOE
Median Fluorescence

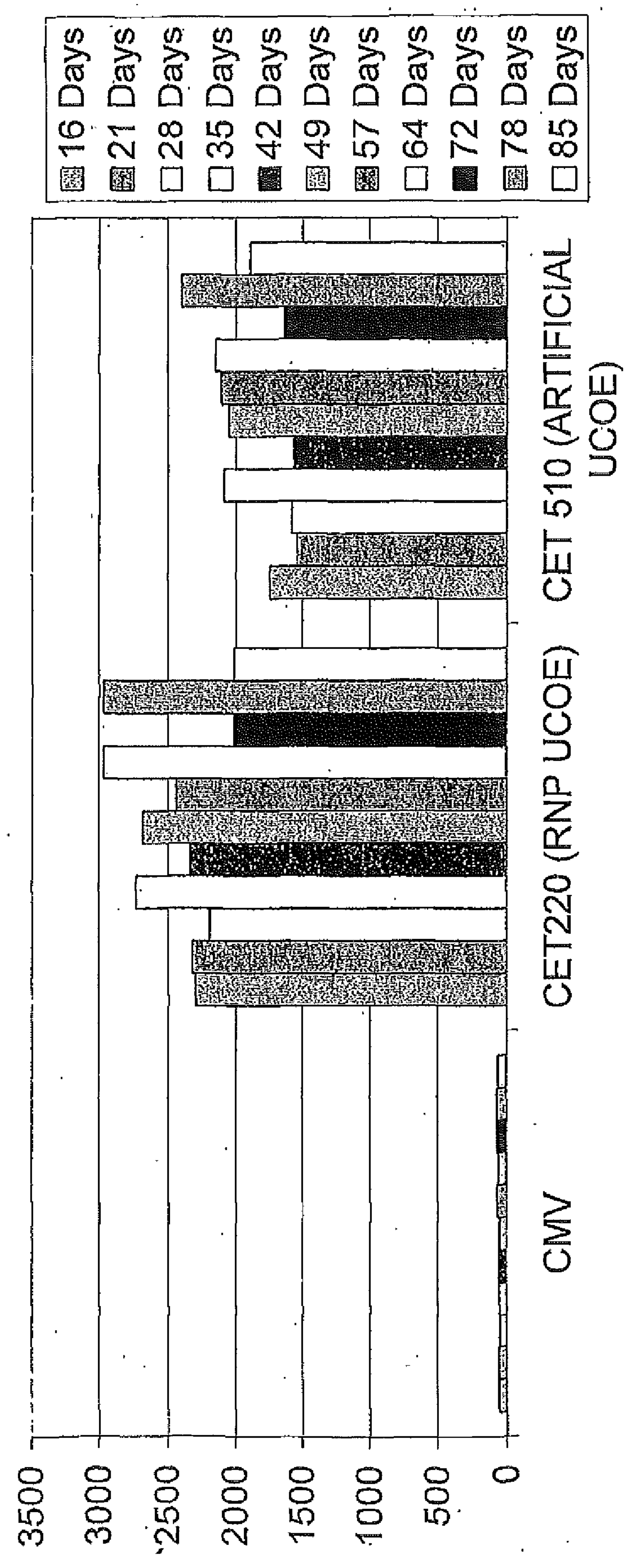

# Figure 5
## CET220 vs PDCD2/actin artificial UCOE
## % Positive Cells

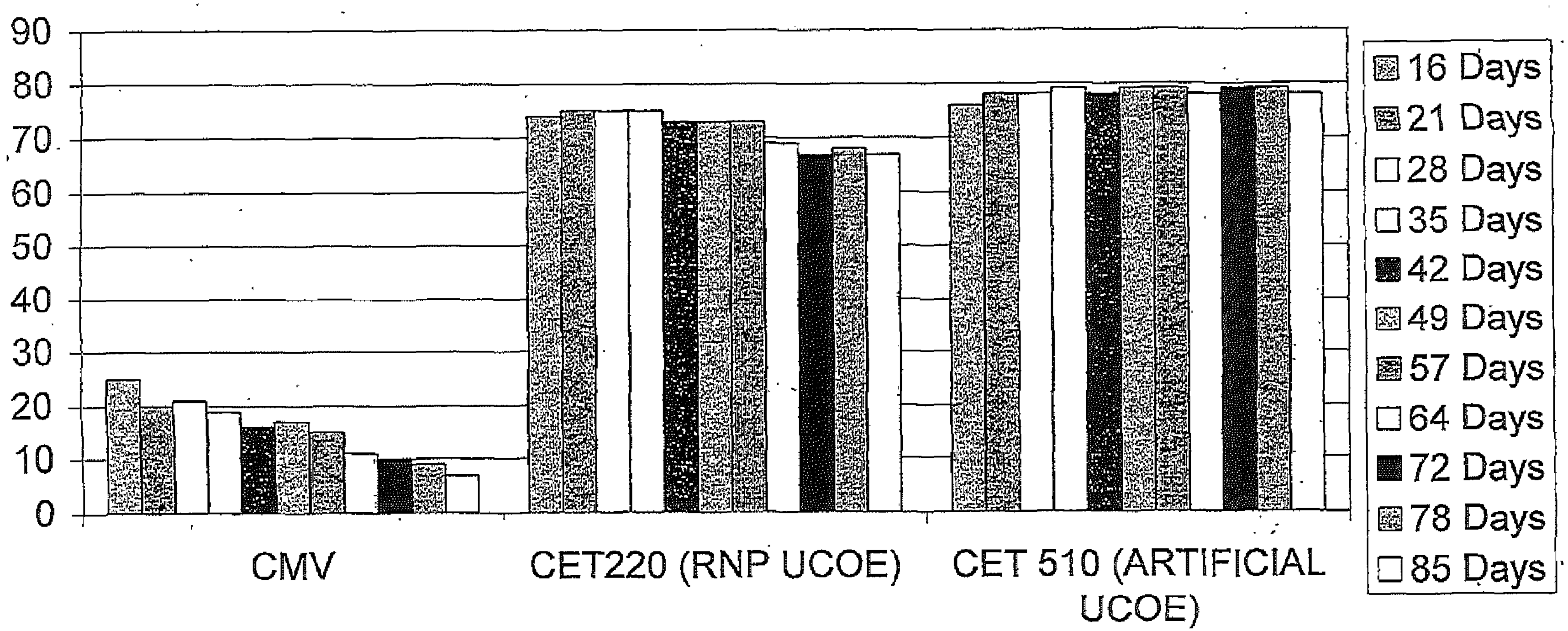

# RNP/HP-1/actin artificial UCOE restriction map

**(Please note that this figure is complementary to the sequence of Figure 7)**

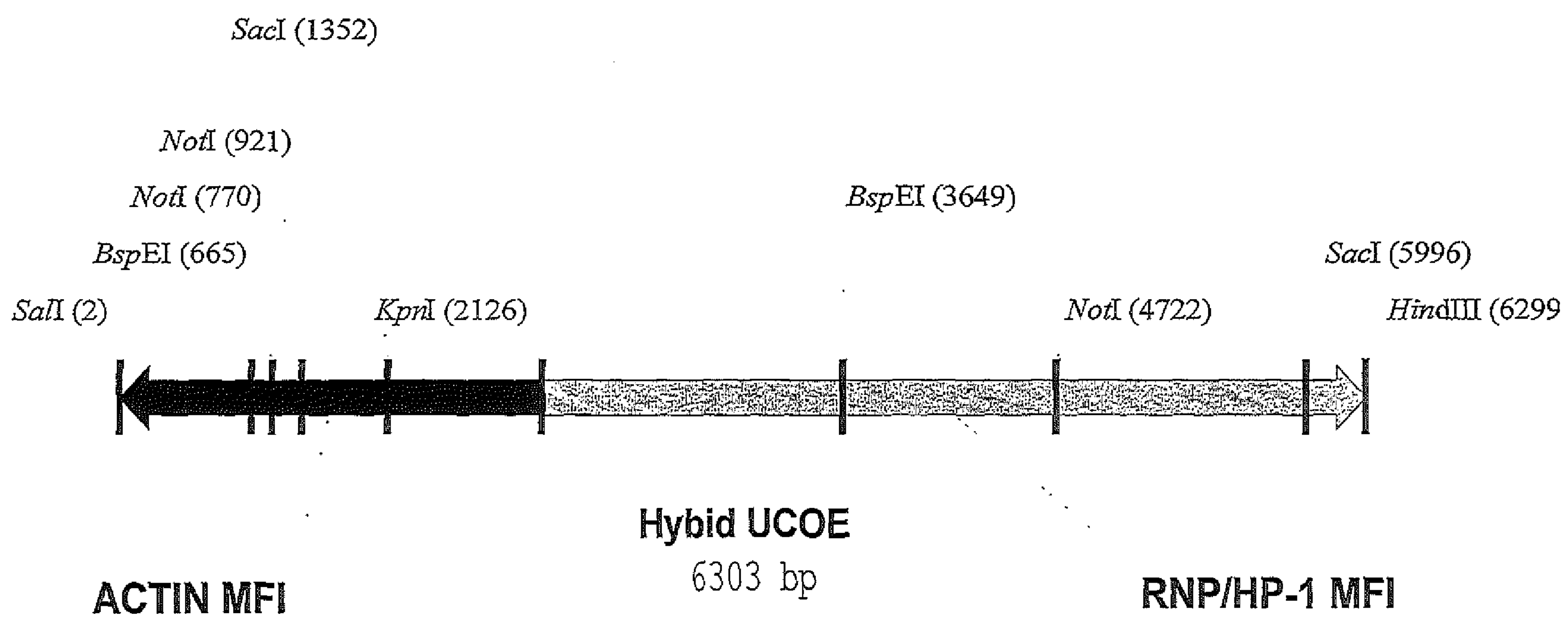


FIGURE 6:

```
1     AAGCTTACTT GATTGGCCAT GTGGCAAGCG ACAGGCACAA AACAATTTTC
      TTCGAATGAA CTAACCGGTA CACCGTTCGC TGTCCGTGTT TTGTTAAAAG
51    CAAGTCAATA GGAAAAACCT CAGAGCTGAA ATCTTTATAT GCTGTACTAC
      GTTCAGTTAT CCTTTTTGGA GTCTCGACTT TAGAAATATA CGACATGATG
101   ACAGCTGTAT TCTGGGCACT TATGAATGTT AAGGAAACCT GTCTTAAAAG
      TGTCGACATA AGACCCGTGA ATACTTACAA TTCCTTTGGA CAGAATTTTC
151   TTAACTAGGT TAAAAAACCT CAAACGAGAG AAAGTGATAT CCAGGACCAA
      AATTGATCCA ATTTTTTGGA GTTTGCTCTC TTTCACTATA GGTCCTGGTT
201   CTGCTACAAA CGCATAATGC AAACTAAAAA GTCACACGTA ATTTTCAATC
      GACGATGTTT GCGTATTACG TTTGATTTTT CAGTGTGCAT TAAAAGTTAG
251   AATTATTTTT TGTTCCTAGC AAGCAGCATT AATTGCTGCT CTCATCCCAG
      TTAATAAAAA ACAAGGATCG TTCGTCGTAA TTAACGACGA GAGTAGGGTC
301   TTCTACGGAG CTCTCCCTCC ATTCGCATGC TCCCAACTCC TAAAAAGTAG
      AAGATGCCTC GAGAGGGAGG TAAGCGTACG AGGGTTGAGG ATTTTTCATC
351   TGGTAAAACC CAGTTCAGAT TTTTTTTCCT GTAGTTTTCA TGACTCGTAA
      ACCATTTTGG GTCAAGTCTA AAAAAAAGGA CATCAAAAGT ACTGAGCATT
401   AAATTAAAGA AAAAATTAAC TGAAATGATC AAACTAGCTC CTATGAGACA
      TTTAATTTCT TTTTTAATTG ACTTTACTAG TTTGATCGAG GATACTCTGT
451   CAAAGCAGTC TTTTGAAATG GTTACTTGTC ACGATAGTTA TTTTCATTTT
      GTTTCGTCAG AAAACTTTAC CAATGAACAG TGCTATCAAT AAAAGTAAAA
501   TTCAGCTAGT TTTTATTCTT AATTGTCGTC AGCACATAGG TTATCTCTAA
      AAGTCGATCA AAAATAAGAA TTAACAGCAG TCGTGTATCC AATAGAGATT
551   ACTGAAATTA CGGATAATGT ACATTTATAA CAAGTTTTAC AAATCACTAA
      TGACTTTAAT GCCTATTACA TGTAAATATT GTTCAAAATG TTTAGTGATT
601   CAAAAAGCAA AAACTCATTA CTTACCTCAC AATTTATCCA AACTTACCCG
      GTTTTTCGTT TTTGAGTAAT GAATGGAGTG TTAAATAGGT TTGAATGGGC
651   ATGTCCACTA TCGATTTTAA ACAATGTTAT TTTATAAACG TGCTTAGGGT
      TACAGGTGAT AGCTAAAATT TGTTACAATA AAATATTTGC ACGAATCCCA
701   CAAAGAAAAA TAACCAGGTA GACCCCCTTC GCTTGAGACC TTATGCTTAT
      GTTTCTTTTT ATTGGTCCAT CTGGGGGAAG CGAACTCTGG AATACGAATA
751   CAATGTAATG TTCAACCAAG ATTGCAAACA AAATGAGAAA AGTAACAAAG
      GTTACATTAC AAGTTGGTTC TAACGTTTGT TTTACTCTTT TCATTGTTTC
801   TTCAAATACA GAGCGGCCCA GGCCCAAAAC AGTTTTGCAC ATCAATCCAT
      AAGTTTATGT CTCGCCGGGT CCGGGTTTTG TCAAAACGTG TAGTTAGGTA
851   ACGCATTACA GGAAGGAGCC TCTGAAGCCA TGTTTTAATC GAAGTATAAC
      TGCGTAATGT CCTTCCTCGG AGACTTCGGT ACAAAATTAG CTTCATATTG
901   TAAGGACAAA ATCGTTATTT CACTTTCCTC GTAATCATCT ATAAAGGTCC
      ATTCCTGTTT TAGCAATAAA GTGAAAGGAG CATTAGTAGA TATTTCCAGG
951   ATGGATCTGT CCCGTAAGGG TTAAACTTCT CAGTAACAAC ATTACTTAAA
      TACCTAGACA GGGCATTCCC AATTTGAAGA GTCATTGTTG TAATGAATTT
1001  ATGAGTCAGC TCTACAACTT AAACGGAATC CTTAAGAACA GTAAAGGATT
      TACTCAGTCG AGATGTTGAA TTTGCCTTAG GAATTCTTGT CATTTCCTAA
1051  CTGACGCGAA TATCCCTCCC CCGCCCAGAA AACCACCTTC GTCCCTGCCC
      GACTGCGCTT ATAGGGAGGG GGCGGGTCTT TTGGTGGAAG CAGGGACGGG
1101  CTCGTGGCCG ATGGCTTCCA ATTTATGTTT ATTTTGCCGC GGTTCATCTG
      GAGCACCGGC TACCGAAGGT TAAATACAAA TAAAACGGCG CCAAGTAGAC
1151  TCGTTTTACT GACTGCAGAC CCAGATAAAA CCGTTACTCA AAGGAAAAAA
      AGCAAAATGA CTGACGTCTG GGTCTATTTT GGCAATGAGT TTCCTTTTTT
1201  AAGACAGGAA AAACATAAAA TGGTTTCTTT GTCCTACGGC TCGCATTGAA
      TTCTGTCCTT TTTGTATTTT ACCAAAGAAA CAGGATGCCG AGCGTAACTT
1251  CCCGGCCCGA CGCCCTGGGT GGTGATATCT TCTCTGAAAC CGGGCCCGCA
      GGGCCGGGCT GCGGGACCCA CCACTATAGA AGAGACTTTG GCCCGGGCGT
1301  AACCCGGAGC ACCCCCCCTC CCCGCTCTTC GGTGTGGCTT CCGAACGCAA
      TTGGGCCTCG TGGGGGGGAG GGGCGAGAAG CCACACCGAA GGCTTGCGTT
1351  TGGCGCCATT TCATCGAGGG GAAGGCTGAG CGCCTTTAAT GAGGTGCGCA
      ACCGCGGTAA AGTAGCTCCC CTTCCGACTC GCGGAAATTA CTCCACGCGT
1401  GGACTCTAAA GATCCAAGCT CACAAAACAC TCCAAATCCA CCTCGAAACG
      CCTGAGATTT CTAGGTTCGA GTGTTTTGTG AGGTTTAGGT GGAGCTTTGC
```

FIGURE 7: RNP/HP-1/ACTIN ARTIFICIAL UCOE SEQUENCE.

```
1451  ATATGAAAAC AGCCCGAGAA GAAAAAAAAA ATAGTTAACC ACTTCTACTT
      TATACTTTTG TCGGGCTCTT CTTTTTTTTT TATCAATTGG TGAAGATGAA
1501  CTTGATAGAG AAAGCACACT AAGAAAATAA AAGAGTTATA AGGAAAACGC
      GAACTATCTC TTTCGTGTGA TTCTTTTATT TTCTCAATAT TCCTTTTGCG
1551  TGAGAGGAAG GCGAGCCATG AAAATGGCGG CCGCCAAATC GGTTCCCGGG
      ACTCTCCTTC CGCTCGGTAC TTTTACCGCC GGCGGTTTAG CCAAGGGCCC
1601  AGAGAGGGGG AGGGGAAGCT CCGCAGCCTC GCTCACGAGG ACCTGCTGCC
      TCTCTCCCCC TCCCCTTCGA GGCGTCGGAG CGAGTGCTCC TGGACGACGG
1651  CGCCGAAACG CTCGCCGAGG AGACGCCGTG GCCCCCGAAG CAGCGTGCTT
      GCGGCTTTGC GAGCGGCTCC TCTGCGGCAC CGGGGGCTTC GTCGCACGAA
1701  TAGAAAGGGA ATAAGAATTC CCGCCTCCGC GCCCCACTTT CACCCCAGCG
      ATCTTTCCCT TATTCTTAAG GGCGGAGGCG CGGGGTGAAA GTGGGGTCGC
1751  GGGCAGCGTC CGCCATGTGA AAGCTCCCCA TCCCCCACCC CCAGTGAAGG
      CCCGTCGCAG GCGGTACACT TTCGAGGGGT AGGGGGTGGG GGTCACTTCC
1801  GAAATGGCGC CGGGAGGCTG AGGGTGGGGA AGCTGTTTGT ACGCTCAGGC
      CTTTACCGCG GCCCTCCGAC TCCCACCCCT TCGACAAACA TGCGAGTCCG
1851  CTCCGCTCAA GACCCCGTTC ATAAACCTTA AGCCCCACTG CTACTGAATT
      GAGGCGAGTT CTGGGGCAAG TATTTGGAAT TCGGGGTGAC GATGACTTAA
1901  GGTCCGATTT CCTGCCTCTC TCCCACGGAG GCGGCTGGCC GACTTCCACT
      CCAGGCTAAA GGACGGAGAG AGGGTGCCTC CGCCGACCGG CTGAAGGTGA
1951  GAGGCGCCAA CGGCCTCGCC ATGCCCTTTT CAATAACTCA TTGATTTCAA
      CTCCGCGGTT GCCGGAGCGG TACGGGAAAA GTTATTGAGT AACTAAAGTT
2001  ACCCGTTACC TCCATCGCGG ACTCAGTCGC TTCAGCCCGA TTTCCCGCAG
      TGGGCAATGG AGGTAGCGCC TGAGTCAGCG AAGTCGGGCT AAAGGGCGTC
2051  CCGAGCGAGA TGAGAGAGAT CTCCGCGGAC GAACACGAAC CGGACTCGTC
      GGCTCGCTCT ACTCTCTCTA GAGGCGCCTG CTTGTGCTTG GCCTGAGCAG
2101  CTGGCGCTGT AGTGAGAACT GCCGCTGCTC GAGAAACAAC TCTGCGAGGA
      GACCGCGACA TCACTCTTGA CGGCGACGAG CTCTTTGTTG AGACGCTCCT
2151  GCACCTCCGC ACGGGACCCG GCGCTGCTGC TACTGCCGCT AGAGCCGCTG
      CGTGGAGGCG TGCCCTGGGC CGCGACGACG ATGACGGCGA TCTCGGCGAC
2201  CCGCCGCTTT TCTAGAACCT TCCCCCCCAC TAACGCGTCT TCCGCTACGT
      GGCGGCGAAA AGATCTTGGA AGGGGGGGTG ATTGCGCAGA AGGCGATGCA
2251  CAGGCCGTCG CGTAAACGCC CTATCCGCCG CCAATGGCGG GAAGGCTCTA
      GTCCGGCAGC GCATTTGCGG GATAGGCGGC GGTTACCGCC CTTCCGAGAT
2301  CGCCCCACCT TACGCCAAAT GCGTACTCCT CCCACCCTTG CGGCCAGAGA
      GCGGGGTGGA ATGCGGTTTA CGCATGAGGA GGGTGGGAAC GCCGGTCTCT
2351  CAGTACCCGA CGTTACTTCC GTAAATGCGC TCAATGAATT GCGGAAGGCT
      GTCATGGGCT GCAATGAAGG CATTTACGCG AGTTACTTAA CGCCTTCCGA
2401  AGAGTCCTGC TAGTTACTAC CTCTTGGAAT AGGGTCCCGG CCCCTGCCTT
      TCTCAGGACG ATCAATGATG GAGAACCTTA TCCCAGGGCC GGGGACGGAA
2451  GGCGAAGGCA GGTGAGAAAC GTCGCGCAGT TTGAAATTAA CGCCGACGGG
      CCGCTTCCGT CCACTCTTTG CAGCGCGTCA AACTTTAATT GCGGCTGCCC
2501  AGGGGCTTAA TCCGCAGCCT GGAGATCCAG CCCCCTCAAC CCGGGAGGTG
      TCCCCGAATT AGGCGTCGGA CCTCTAGGTC GGGGGAGTTG GGCCCTCCAC
2551  GTCCCTGCAG TTACGCCAAT GATAACCCCC GCCAGAAAAA TCTTAGTAGC
      CAGGGACGTC AATGCGGTTA CTATTGGGGG CGGTCTTTTT AGAATCATCG
2601  CTTCCCTTTT TGTTTTCCGT GCCCCAACTC GGCGGATTGA CTCGGCCCCT
      GAAGGGAAAA ACAAAAGGCA CGGGGTTGAG CCGCCTAACT GAGCCGGGGA
2651  TCCGGAAACA CCCGAATCAA CTTCTAGTCA AATTATTGTT CACGCCGCAA
      AGGCCTTTGT GGGCTTAGTT GAAGATCAGT TTAATAACAA GTGCGGCGTT
2701  TGACCCACCC CTGGCCCGCG TCTGTGGAAC TGACCCCTGG TGTACAGGAG
      ACTGGGTGGG GACCGGGCGC AGACACCTTG ACTGGGGACC ACATGTCCTC
2751  AGTTCGCTGC TGAAAGTGGT CCCAAAGGGG TACTAGTTTT TAAGCTCCCA
      TCAAGCGACG ACTTTCACCA GGGTTTCCCC ATGATCAAAA ATTCGAGGGT
2801  ACTCCCCCTC CCCCAGCGTC TGGAGGATTC CACACCCTCG CACCGGCGGG
      TGAGGGGGAG GGGGTCGCAG ACCTCCTAAG GTGTGGGAGC GTGGCCGCCC
2851  CGAGGAAGTG GGCGGAGTCC GGTTTTGGCG CCAGCCGCTG AGGCTGCCAA
      GCTCCTTCAC CCGCCTCAGG CCAAAACCGC GGTCGGCGAC TCCGACGGTT
```

```
2901  GCAGAAAAGC CACCGCTGAG GAGACTCCGG TCACTGTCCT CGCCCCGCCT
      CGTCTTTTCG GTGGCGACTC CTCTGAGGCC AGTGACAGGA GCGGGGCGGA
2951  CCCCCTTCCC TCCCCTTGGG GACCACCGGG CGCCACGCCG CGAACGGTAA
      GGGGGAAGGG AGGGGAACCC CTGGTGGCCC GCGGTGCGGC GCTTGCCATT
3001  GTGCCGCGGT CGTCGGCGCC TCCGCCCTCC CCCTAGGGCC CCAATTCCCA
      CACGGCGCCA GCAGCCGCGG AGGCGGGAGG GGGATCCCGG GGTTAAGGGT
3051  GCGGGCGCGG CGCCGGCCCC TCCCCCCGCC GCGCGCGCGC CCGCTGCCCC
      CGCCCGCGCC GCGGCCGGGG AGGGGGGCGG CGCGCGCGCG GGCGACGGGG
3101  GCCCTTCGTG GCCGCCCGGC GTGGGCGGTG CCACCCCTCC CCCCGGCGGC
      CGGGAAGCAC CGGCGGGCCG CACCCGCCAC GGTGGGGAGG GGGGCCGCCG
3151  CCCGCGCGCA GCTCCCGGCT CCCTCCCCCT TCGGATGTGG CTTGAGCTGT
      GGGCGCGCGT CGAGGGCCGA GGGAGGGGGA AGCCTACACC GAACTCGACA
3201  AGGCGCGGAG GGCCGGAGAC GCTGCAGACC CGCGACCCGG AGCAGCTCGG
      TCCGCGCCTC CCGGCCTCTG CGACGTCTGG GCGCTGGGCC TCGTCGAGCC
3251  AGGCGGTGAA GTCGGTGGCT TTCCTTCTCT CTAGCTCTCG CTCGCTGGTG
      TCCGCCACTT CAGCCACCGA AAGGAAGAGA GATCGAGAGC GAGCGACCAC
3301  GTGCTTCAGA TGCCACACGC GTCCCGGGGG CCCGGTTCTC CGCTCCCCTC
      CACGAAGTCT ACGGTGTGCG CAGGGCCCCC GGGCCAAGAG GCGAGGGGAG
3351  CCCTCCCCTT CTCGCCGGAC CCCGCGCCGG GAGCTGCGGG AAGGAGTGGA
      GGGAGGGGAA GAGCGGCCTG GGGCGCGGCC CTCGACGCCC TTCCTCACCT
3401  GGGTCGGGCG GTGGCCTCGC GGCTGGCCTG GCGCGCGGCC AGCCCGGTAG
      CCCAGCCCGC CACCGGAGCG CCGACCGGAC CGCGCGCCGG TCGGGCCATC
3451  TTAGTGGGGG GACTGCTCTG CCCTCGAGGG GGTAGGGAGC TGTGGCGACG
      AATCACCCCC CTGACGAGAC GGGAGCTCCC CCATCCCTCG ACACCGCTGC
3501  GTTGCCCCAT TTCGAGACAA AGCGCATTTC CCCCTCCCCT CCCCCACCCG
      CAACGGGGTA AAGCTCTGTT TCGCGTAAAG GGGGAGGGGA GGGGGTGGGC
3551  CGTTCCGGCG GAGGCGCCCC CTCCCCCAGC GCCACGCGGG GCTGGGTCGA
      GCAAGGCCGC CTCCGCGGGG GAGGGGGTCG CGGTGCGCCC CGACCCAGCT
3601  GACTTGGGCC TCCCGGAGGG CGGCGCGTGG TCCCGCGTCC GCGAGCCTGG
      CTGAACCCGG AGGGCCTCCC GCCGCGCACC AGGGCGCAGG CGCTCGGACC
3651  CGGCGCGCGG CCGGCTGTCC CGAGGCTGCG GCGACCGCCA GTTAACGTGG
      GCCGCGCGCC GGCCGACAGG GCTCCGACGC CGCTGGCGGT CAATTGCACC
3701  CCGCGCGGGG GTAGGCGCGT GCGGTGTGGC GCAGTGCCCT TGAGCCCCCG
      GGCGCGCCCC CATCCGCGCA CGCCACACCG CGTCACGGGA ACTCGGGGGC
3751  TGCCGCGGCC TTTGTTTCTC CCCGCGGATG CGCTGACCAC GAGGCCCGCG
      ACGGCGCCGG AAACAAAGAG GGGCGCCTAC GCGACTGGTG CTCCGGGCGC
3801  CTCCCGGGTG GGGGCGGGCA CCCGCGCTTA GGCCTCTGGA CGCCGGGCTT
      GAGGGCCCAC CCCCGCCCGT GGGCGCGAAT CCGGAGACCT GCGGCCCGAA
3851  CAGCGGCGGG GGTCGGGAGC GGGTGTTTGC AAGAGGTGAT TCTTTTTTCA
      GTCGCCGCCC CCAGCCCTCG CCCACAAACG TTCTCCACTA AGAAAAAAGT
3901  AAGTGTCACG AAACGGGGTT GAAGCATCTT AAGTTTTTTC CTTTTGTTAT
      TTCACAGTGC TTTGCCCCAA CTTCGTAGAA TTCAAAAAAG GAAAACAATA
3951  TTAATTACCG ATTGGAAAGA GGGAGGGTTT CTGAGCAGAA ACCAAGTTGG
      AATTAATGGC TAACCTTTCT CCCTCCCAAA GACTCGTCTT TGGTTCAACC
4001  GATTGCAGAA CAGAGAAGAT TCACAGTGCT TTACCGTTGT GAGTTGTTTG
      CTAACGTCTT GTCTCTTCTA AGTGTCACGA AATGGCAACA CTCAACAAAC
4051  GGTAATCGTG CCTGGTTTTA AACCGAAAGG ATTGTCCTTT AAAAATGGAA
      CCATTAGCAC GGACCAAAAT TTGGCTTTCC TAACAGGAAA TTTTTACCTT
4101  CATGGACTTT ATTATAAATG GGACTTAGAT TGGAAAAGAC ATTGGTCCCC
      GTACCTGAAA TAATATTTAC CCTGAATCTA ACCTTTTCTG TAACCAGGGG
4151  TATTTTAAGC CATGTGAAGC TGTTTTAGGT ACCGCGGGCC CCCTCTGTGG
      ATAAAATTCG GTACACTTCG ACAAAATCCA TGGCGCCCGG GGGAGACACC
4201  TCCCACGCCA CTGATCGCTG CATGCCCACC ACCTGGGTAC ACACAGTCTG
      AGGGTGCGGT GACTAGCGAC GTACGGGTGG TGGACCCATG TGTGTCAGAC
4251  TGATTCCCGG AGCAGAACGG ACCCTGCCCA CCCGGTCTTG TGTGCTACTC
      ACTAAGGGCC TCGTCTTGCC TGGGACGGGT GGGCCAGAAC ACACGATGAG
4301  AGTGGACAGA CCCAAGGCAA GAAAGGGTGA CAAGGACAGG GTCTTCCCAG
      TCACCTGTCT GGGTTCCGTT CTTTCCCACT GTTCCTGTCC CAGAAGGGTC
```

```
4351  GCTGGCTTTG AGTTCCTAGC ACCGCCCCGC CCCCAATCCT CTGTGGCACA
      CGACCGAAAC TCAAGGATCG TGGCGGGGCG GGGGTTAGGA GACACCGTGT
4401  TGGAGTCTTG GTCCCCAGAG TCCCCCAGCG GCCTCCAGAT GGTCTGGGAG
      ACCTCAGAAC CAGGGGTCTC AGGGGGTCGC CGGAGGTCTA CCAGACCCTC
4451  GGCAGTTCAG CTGTGGCTGC GCATAGCAGA CATACAACGG ACGGTGGGCC
      CCGTCAAGTC GACACCGACG CGTATCGTCT GTATGTTGCC TGCCACCCGG
4501  CAGACCCAGG CTGTGTAGAC CCAGCCCCCC CGCCCCGCAG TGCCTAGGTC
      GTCTGGGTCC GACACATCTG GGTCGGGGGG GCGGGGCGTC ACGGATCCAG
4551  ACCCACTAAC GCCCCAGGCC TGGTCTTGGC TGGGCGTGAC TGTTACCCTC
      TGGGTGATTG CGGGGTCCGG ACCAGAACCG ACCCGCACTG ACAATGGGAG
4601  AAAAGCAGGC AGCTCCAGGG TAAAAGGTGC CCTGCCCTGT AGAGCCCACT
      TTTTCGTCCG TCGAGGTCCC ATTTTCCACG GGACGGGACA TCTCGGGTGA
4651  TCCTTCCCAG GGCTGCGGCT GGGTAGGTTT GTAGCCTTCA TCACGGGCCA
      AGGAAGGGTC CCGACGCCGA CCCATCCAAA CATCGGAAGT AGTGCCCGGT
4701  CCTCCAGCCA CTGGACCGCT GGCCCCTGCC CTGTCCTGGG GAGTGTGGTC
      GGAGGTCGGT GACCTGGCGA CCGGGGACGG GACAGGACCC CTCACACCAG
4751  CTGCGACTCT AATGGCCGCA AGCCACCTGA CTCCCCCAAC ACCACACTCT
      GACGCTGAGA TTACCGGCGT TCGGTGGACT GAGGGGGTTG TGGTGTGAGA
4801  ACCTCTCAAG CCCAGGTCTC TCCCTAGTGA CCCACCCAGC ACATTTAGCT
      TGGAGAGTTC GGGTCCAGAG AGGGATCACT GGGTGGGTCG TGTAAATCGA
4851  AGCTGAGCCC CACAGCCAGA GGTCCTCAGG CCCTGCTTTC AGGGCAGTTG
      TCGACTCGGG GTGTCGGTCT CCAGGAGTCC GGGACGAAAG TCCCGTCAAC
4901  CTCTGAAGTC GGCAAGGGGG AGTGACTGCC TGGCCACTCC ATGCCCTCCA
      GAGACTTCAG CCGTTCCCCC TCACTGACGG ACCGGTGAGG TACGGGAGGT
4951  AGAGCTCCTT CTGCAGGAGC GTACAGAACC CAGGGCCCTG GCACCCGTGC
      TCTCGAGGAA GACGTCCTCG CATGTCTTGG GTCCCGGGAC CGTGGGCACG
5001  AGACCCTGGC CCACCCCACC TGGGCGCTCA GTGCCCAAGA GATGTCCACA
      TCTGGGACCG GGTGGGGTGG ACCCGCGAGT CACGGGTTCT CTACAGGTGT
5051  CCTAGGATGT CCCGCGGTGG GTGGGGGGCC CGAGAGACGG GCAGGCCGGG
      GGATCCTACA GGGCGCCACC CACCCCCCGG GCTCTCTGCC CGTCCGGCCC
5101  GGCAGGCCTG GCCATGCGGG GCCGAACCGG GCACTGCCCA GCGTGGGGCG
      CCGTCCGGAC CGGTACGCCC CGGCTTGGCC CGTGACGGGT CGCACCCCGC
5151  CGGGGGCCAC GGCGCGCGCC CCCAGCCCCC GGGCCCAGCA CCCCAAGGCG
      GCCCCCGGTG CCGCGCGCGG GGGTCGGGGG CCCGGGTCGT GGGGTTCCGC
5201  GCCAACGCCA AAACTCTCCC TCCTCCTCTT CCTCAATCTC GCTCTCGCTC
      CGGTTGCGGT TTTGAGAGGG AGGAGGAGAA GGAGTTAGAG CGAGAGCGAG
5251  TTTTTTTTTT TCGCAAAAGG AGGGGAGAGG GGGTAAAAAA ATGCTGCACT
      AAAAAAAAAA AGCGTTTTCC TCCCCTCTCC CCCATTTTTT TACGACGTGA
5301  GTGCGGCGAA GCCGGTGAGT GAGCGGCGCG GGGCCAATCA GCGTGCGCCG
      CACGCCGCTT CGGCCACTCA CTCGCCGCGC CCCGGTTAGT CGCACGCGGC
5351  TTCCGAAAGT TGCCTTTTAT GGCTCGAGCG GCCGCGGCGG CGCCCTATAA
      AAGGCTTTCA ACGGAAAATA CCGAGCTCGC CGGCGCCGCC GCGGGATATT
5401  AACCCAGCGG CGCGACGCGC CACCACCGCC GAGACCGCGT CCGCCCGCGA
      TTGGGTCGCC GCGCTGCGCG GTGGTGGCGG CTCTGGCGCA GGCGGGCGCT
5451  GCACAGAGCC TCGCCTTTGC CGATCCGCCG CCCGTCCACA CCCGCCGCCA
      CGTGTCTCGG AGCGGAAACG GCTAGGCGGC GGGCAGGTGT GGGCGGCGGT
5501  GGTAAGCCCG GCCAGCCGAC CGGGGCATGC GGCCGCGGCC CTTCGCCCGT
      CCATTCGGGC CGGTCGGCTG GCCCCGTACG CCGGCGCCGG GAAGCGGGCA
5551  GCAGAGCCGC CGTCTGGGCC GCAGCGGGGG GCGCATGGGG CGGAACCGGA
      CGTCTCGGCG GCAGACCCGG CGTCGCCCCC CGCGTACCCC GCCTTGGCCT
5601  CCGCCGTGGG GGGCGCGGGA GAAGCCCCTG GGCCTCCGGA GATGGGGGAC
      GGCGGCACCC CCCGCGCCCT CTTCGGGGAC CCGGAGGCCT CTACCCCCTG
5651  ACCCCACGCC AGTTCGCAGG CGCGAGGCCG CGCTCGGGCG GGCGCGCTCC
      TGGGGTGCGG TCAAGCGTCC GCGCTCCGGC GCGAGCCCGC CCGCGCGAGG
5701  GGGGGTGCCG CTCTCGGGGC GGGGGCAACC GGCGGGGTCT TTGTCTGAGC
      CCCCCACGGC GAGAGCCCCG CCCCCGTTGG CCGCCCCAGA AACAGACTCG
5751  CGGGCTCTTG CCAATGGGGA TCGCACGGTG GGCGCGGCGT AGCCCCCGTC
      GCCCGAGAAC GGTTACCCCT AGCGTGCCAC CCGCGCCGCA TCGGGGGCAG
```

```
5801   AGGCCCGGTG GGGGCTGGGG CGCCATGCGC GTGCGCGCTG GTCCTTTGGG
       TCCGGGCCAC CCCCGACCCC GCGGTACGCG CACGCGCGAC CAGGAAACCC
5851   CGCTAACTGC GTGCGCGCTG GGAATTGGCG CTAATTGCGC GTGCGCGCTG
       GCGATTGACG CACGCGCGAC CCTTAACCGC GATTAACGCG CACGCGCGAC
5901   GGACTCAATG GCGCTAATCG CGCGTGCGTT CTGGGGCCCG GGCGCTTGCG
       CCTGAGTTAC CGCGATTAGC GCGCACGCAA GACCCCGGGC CCGCGAACGC
5951   CCACTTCCTG CCCGAGCCGC TGGCGCCCGA GGGTGTGGCC GCTGCGTGCG
       GGTGAAGGAC GGGCTCGGCG ACCGCGGGCT CCCACACCGG CGACGCACGC
6001   CGCGCGCGAC CCGGTCGCTG TTTGAACCGG GCGGAGGCGG GGCTGGCGCC
       GCGCGCGCTG GGCCAGCGAC AAACTTGGCC CGCCTCCGCC CCGACCGCGG
6051   CGGTTGGGAG GGGGTTGGGG CCTGGCTTCC TGCCGCGCGC CGCGGGGACG
       GCCAACCCTC CCCCAACCCC GGACCGAAGG ACGGCGCGCG GCGCCCCTGC
6101   CCTCCGACCA GTGTTTGCCT TTTATGGTAA TAACGCGGCC GGCCCGGCTT
       GGAGGCTGGT CACAAACGGA AAATACCATT ATTGCGCCGG CCGGGCCGAA
6151   CCTTTGTCCC CAATCTGGGC GCGCGCCGGC GCCCCCTGGC GGCCTAAGGA
       GGAAACAGGG GTTAGACCCG CGCGCGGCCG CGGGGGACCG CCGGATTCCT
6201   CTCGGCGCGC CGGAAGTGGC CAGGGCGGGG GCGACTTCGG CTCACAGCGC
       GAGCCGCGCG GCCTTCACCG GTCCCGCCCC CGCTGAAGCC GAGTGTCGCG
6251   GCCCGGCTAT TCTCGCAGCT CACCATGCCG GTCGCCACCA TGATACCGTC
       CGGGCCGATA AGAGCGTCGA GTGGTACGGC CAGCGGTGGT ACTATGGCAG
6301   GAC
       CTG
```

Figure 8a:

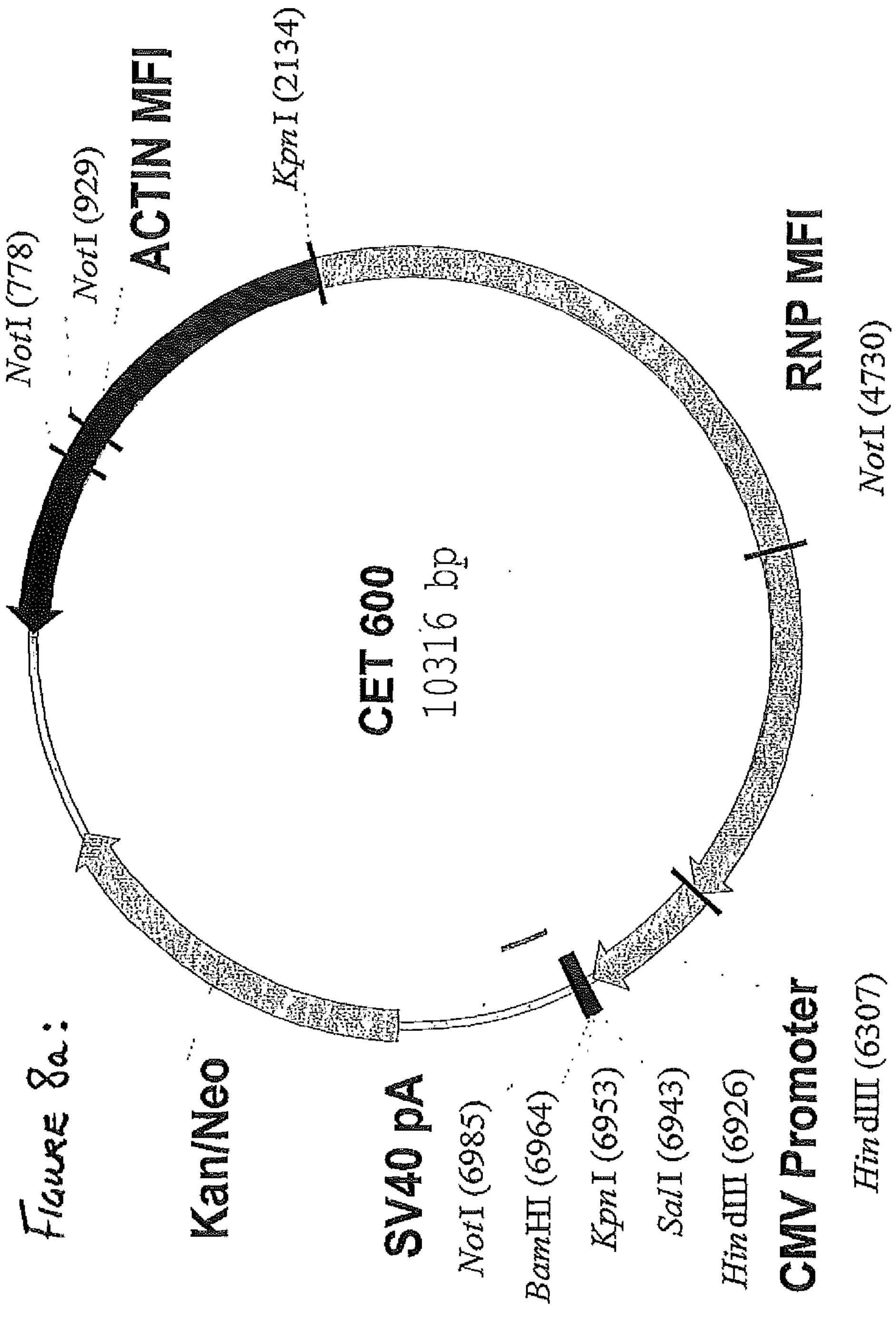
CET 600
10316 bp
ACTIN MFI
NotI (778)
NotI (929)
KpnI (2134)
RNP MFI
NotI (4730)
CMV Promoter
HindIII (6307)
HindIII (6926)
SalI (6943)
KpnI (6953)
BamHI (6964)
NotI (6985)
SV40 pA
Kan/Neo

Figure 8b:

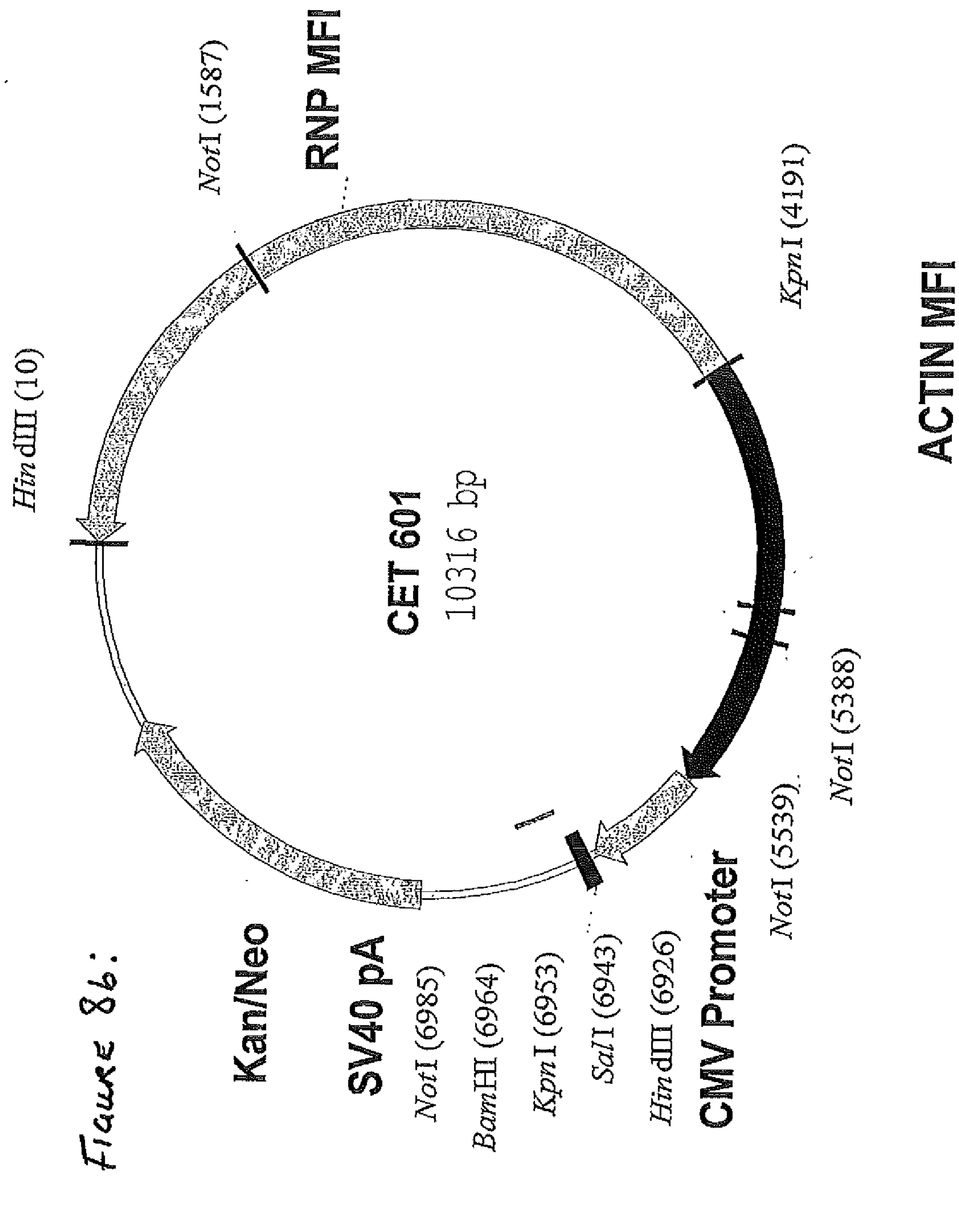
CET 601
10316 bp
HindIII (10)
NotI (1587)
RNP MFI
KpnI (4191)
ACTIN MFI
NotI (5388)
NotI (5539)
CMV Promoter
HindIII (6926)
SalI (6943)
KpnI (6953)
BamHI (6964)
NotI (6985)
SV40 pA
Kan/Neo

Figure 9
**Comparison of RNP UCOE with RNP/HP-1/actin artificial UCOE**

**EGFP Expression in CHO Cells**
**Median Fluorescence**

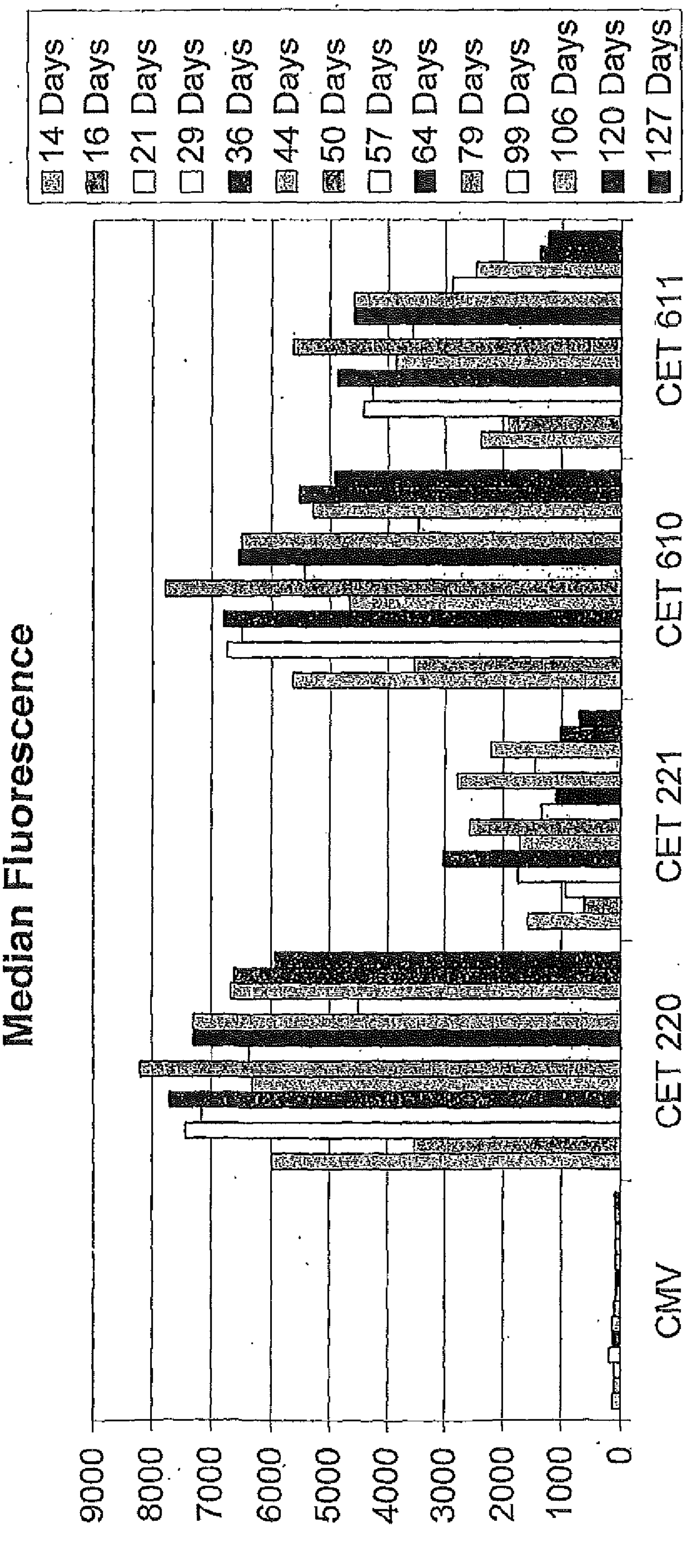

# Figure 10
## Comparison of RNP UCOE with RNP/HP-1/actin artificial UCOE EGFP Expression in CHO Cells
## % Positive Cells

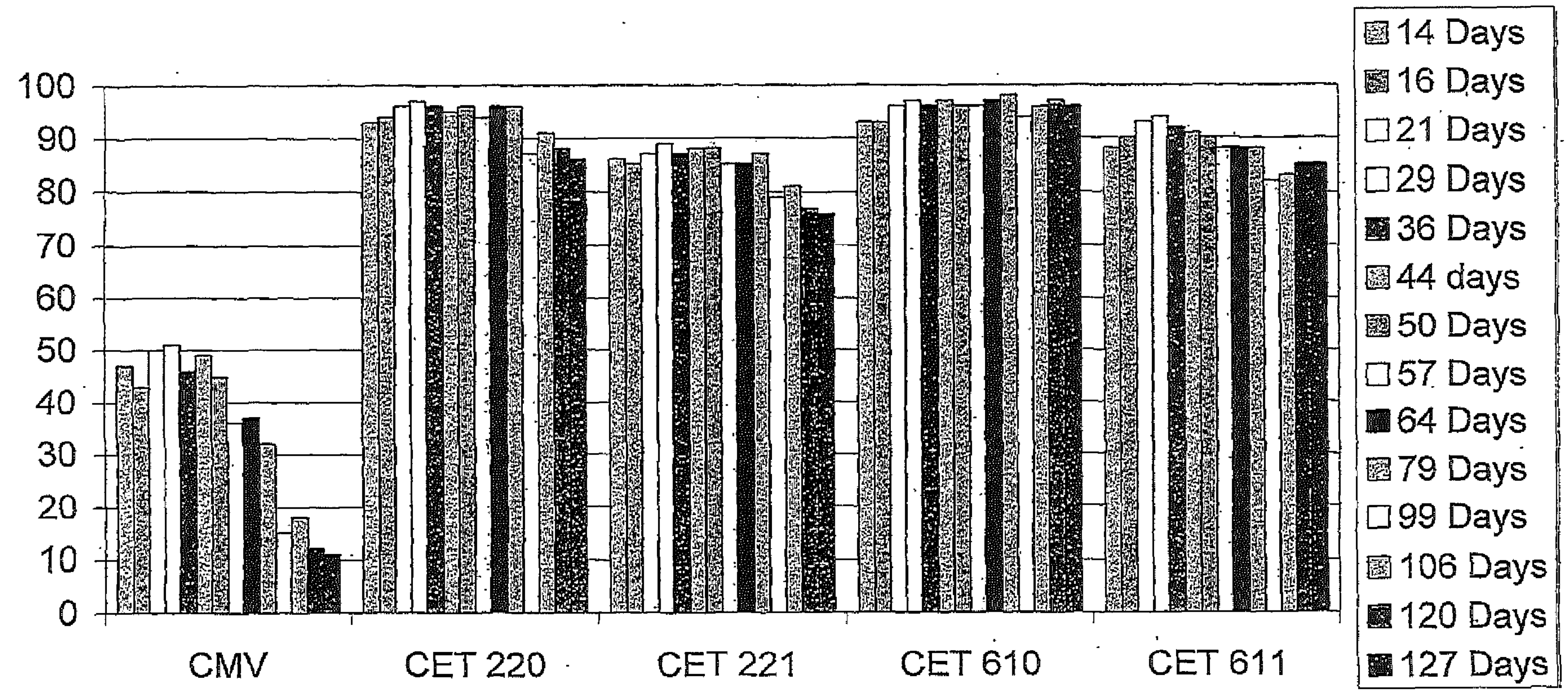

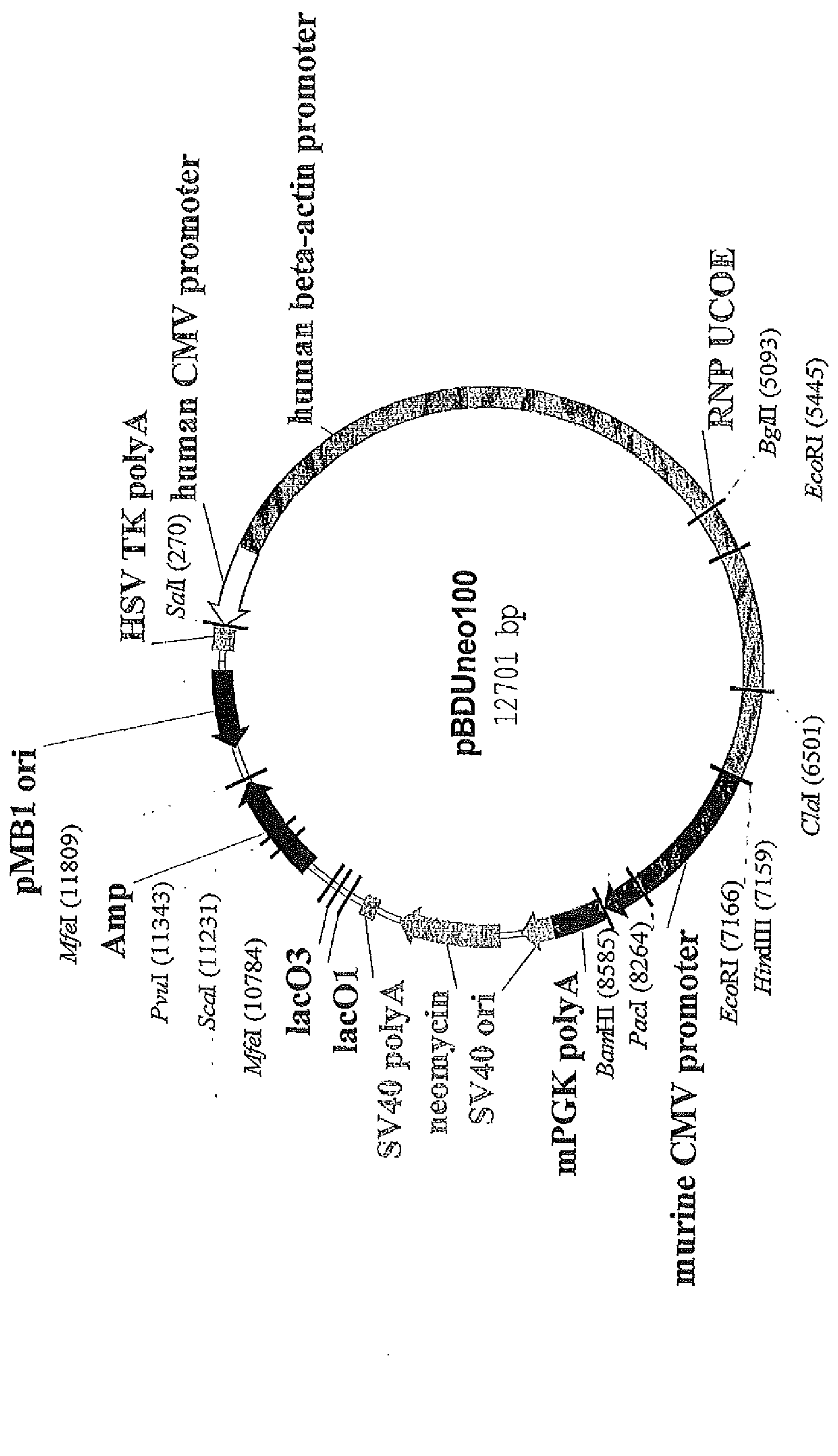
pBDUneo100
12701 bp
HSV TK polyA
SalI (270)
human CMV promoter
human beta-actin promoter
RNP UCOE
BglI (5093)
EcoRI (5445)
ClaI (6501)
EcoRI (7166)
HindIII (7159)
murine CMV promoter
PacI (8264)
BamHI (8585)
mPGK polyA
SV40 ori
neomycin
SV40 polyA
lacO1
lacO3
MfeI (10784)
ScaI (11231)
PvuI (11343)
Amp
MfeI (11809)
pMB1 ori

Figure 11a:

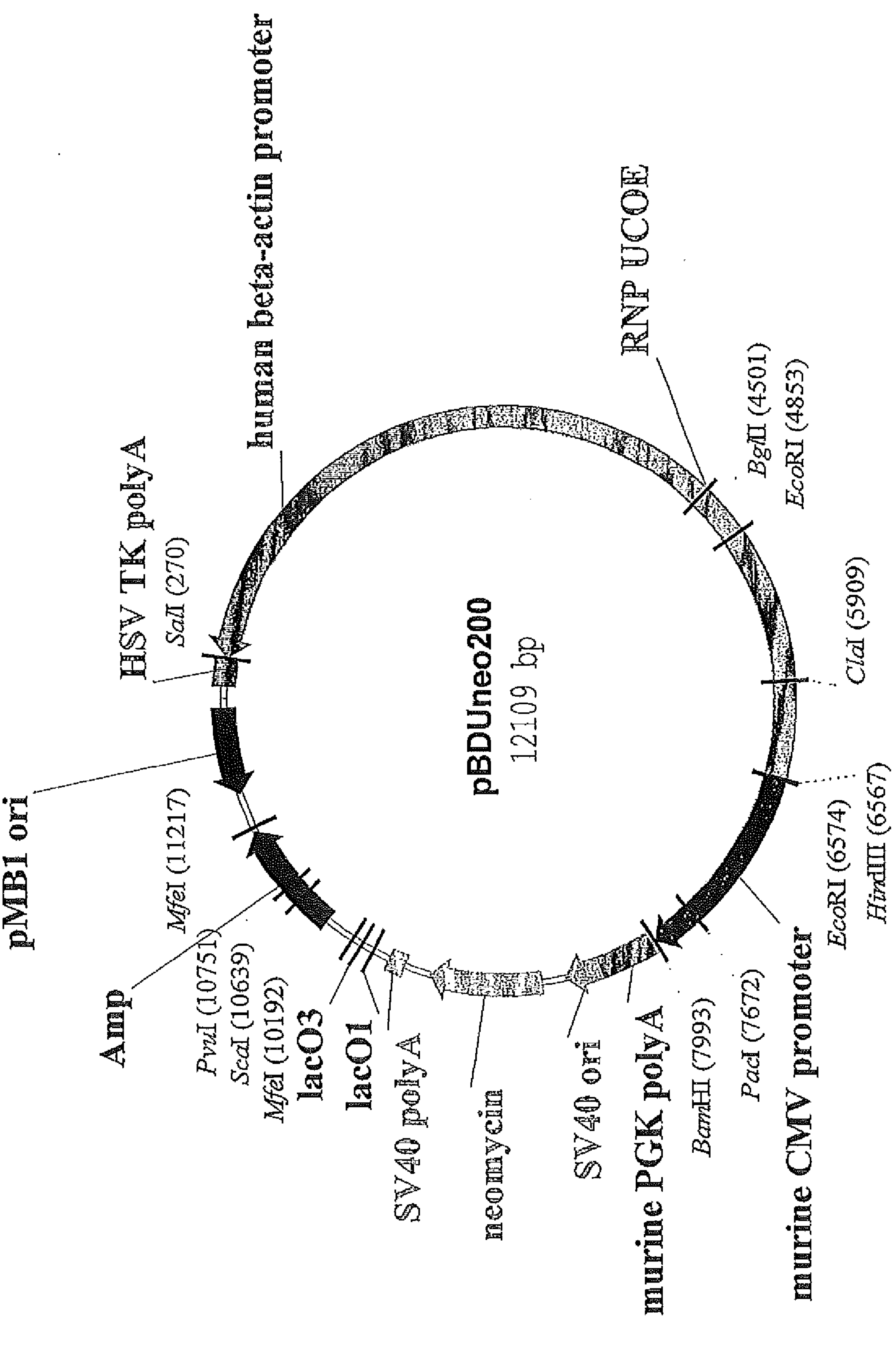
pBDUneo200
12109 bp
HSV TK polyA
SalI (270)
human beta-actin promoter
RNP UCOE
BglII (4501)
EcoRI (4853)
ClaI (5909)
EcoRI (6574)
HindIII (6567)
murine CMV promoter
PacI (7672)
BamHI (7993)
murine PGK polyA
SV40 ori
neomycin
SV40 polyA
lacO1
lacO3
MfeI (10192)
ScaI (10639)
PvuI (10751)
Amp
MfeI (11217)
pMB1 ori

FIGURE 11b:

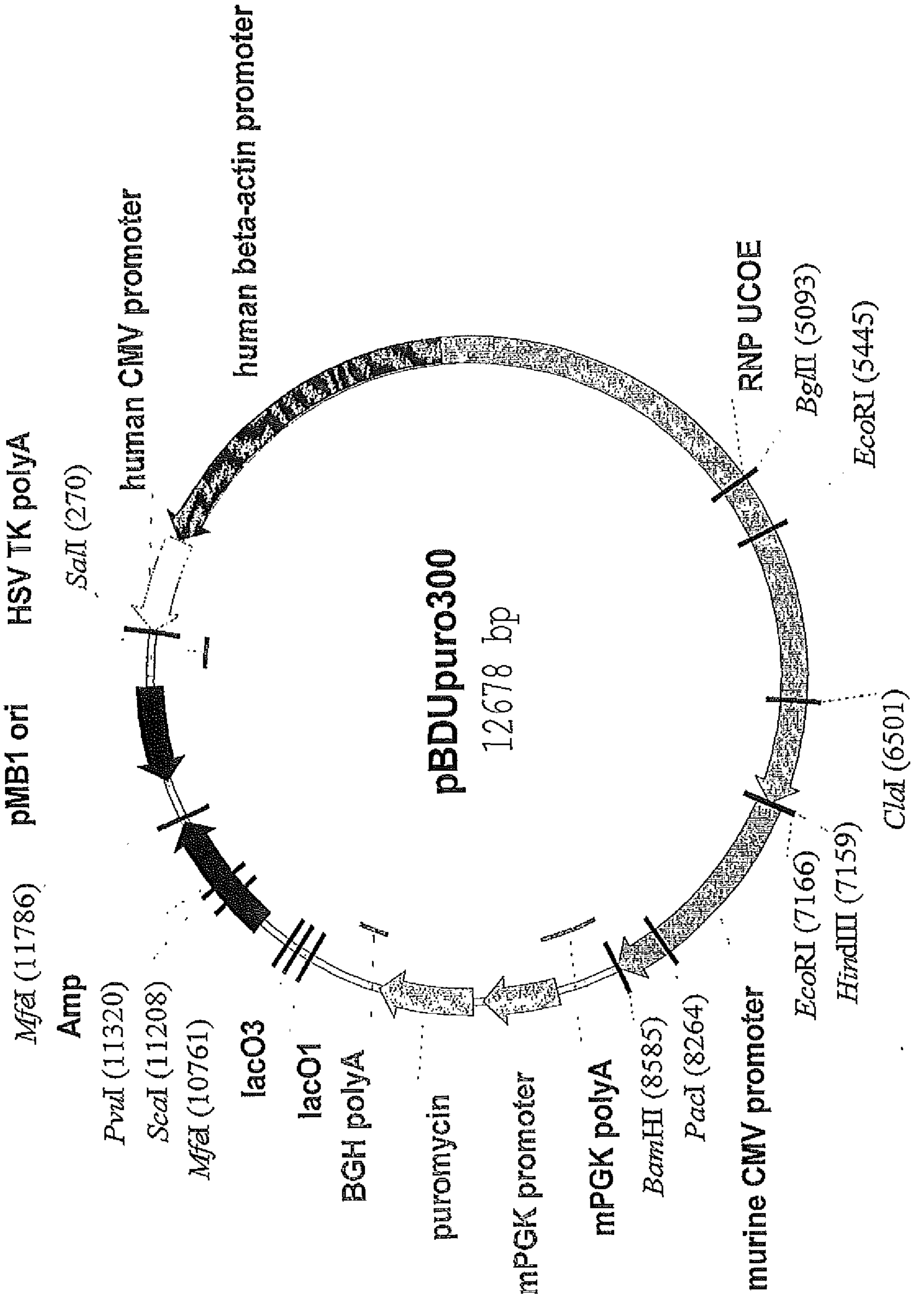
pBDUpuro300
12678 bp
HSV TK polyA
SalI (270)
human CMV promoter
human beta-actin promoter
RNP UCOE
BglII (5093)
EcoRI (5445)
ClaI (6501)
HindIII (7159)
EcoRI (7166)
murine CMV promoter
PacI (8264)
BamHI (8585)
mPGK polyA
mPGK promoter
puromycin
BGH polyA
lacO1
lacO3
MfeI (10761)
ScaI (11208)
PvuI (11320)
Amp
MfeI (11786)
pMB1 ori

FIGURE 11c:

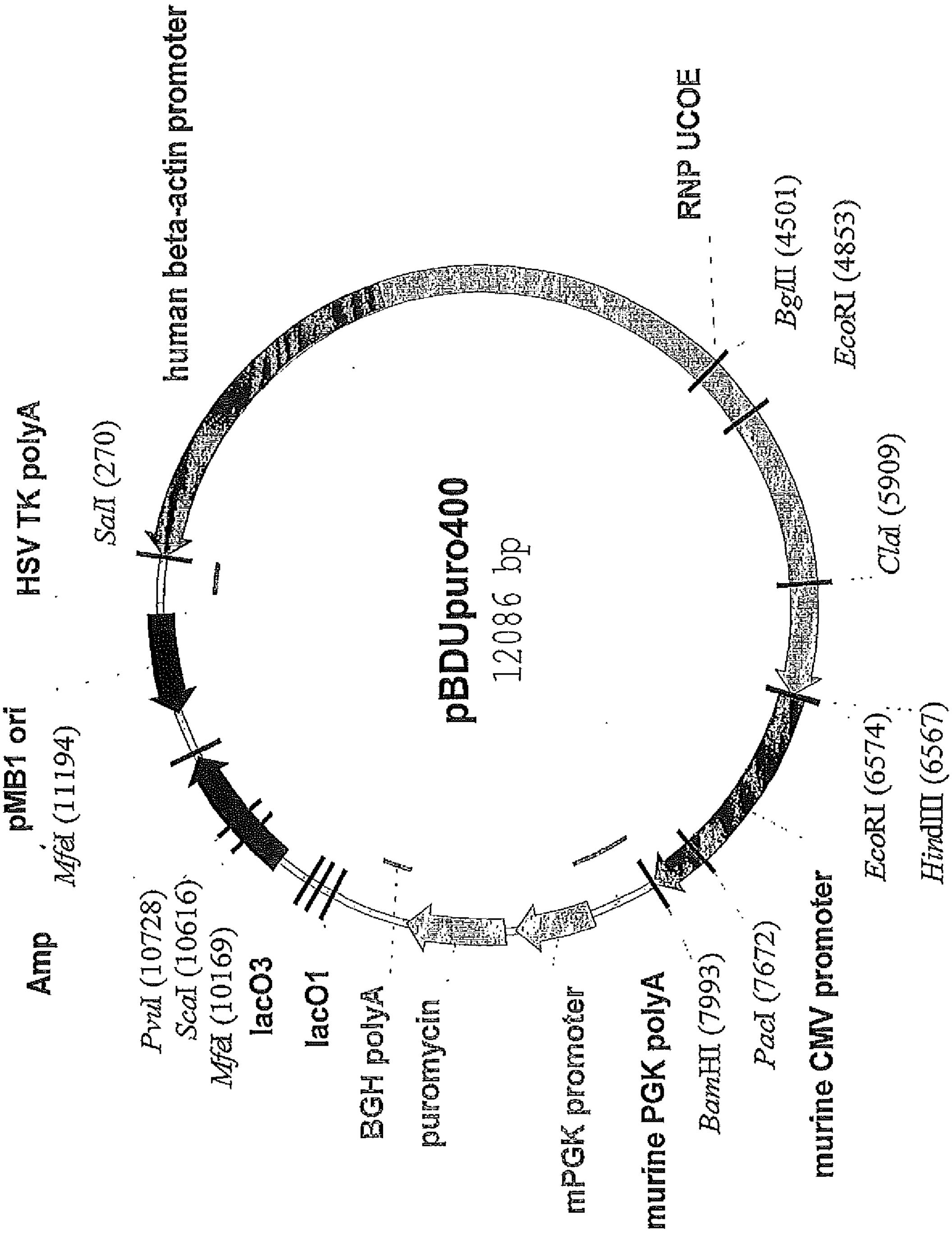
pBDUpuro400
12086 bp
human beta-actin promoter
HSV TK polyA
SalI (270)
pMB1 ori
MfeI (11194)
Amp
PvuI (10728)
ScaI (10616)
MfeI (10169)
lacO3
lacO1
BGH polyA
puromycin
mPGK promoter
murine PGK polyA
BamHI (7993)
PacI (7672)
murine CMV promoter
EcoRI (6574)
HindIII (6567)
ClaI (5909)
EcoRI (4853)
BglII (4501)
RNP UCOE

Figure 11d:

Europäisches Patentamt
European Patent Office
Office européen des brevets

# EUROPEAN SEARCH REPORT

Application Number
EP 10 18 4573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 00/05393 A (CROMBIE ROBERT ;ANTONIOU MICHAEL (GB); COBRA THERAPEUTICS LTD (GB)) 3 February 2000 (2000-02-03)<br>* the whole document *<br>* page 8, line 19 - line 33 *<br>* page 9, line 10 - line 21 *<br>* page 10, line 23 *<br>* page 10, line 32 - line 33 *<br>* page 12, line 2 - line 4 *<br>* figures 1,3 * | 1<br>2-15 | INV.<br>C12N15/11<br>C12N15/85<br>C12N5/10<br>C12Q1/68<br>A61K48/00<br>A01K67/027<br>C12N15/00 |
| X<br>Y | -& DATABASE Geneseq [Online]<br>23 May 2000 (2000-05-23), "hnRNP A2 genomic clone including UCOE.", XP000002650314, retrieved from EBI accession no. GSN:AAZ58411<br>Database accession no. AAZ58411<br>* sequence *<br>----- | 1<br>2-15 | |
| X<br>Y | DATABASE EMBL [Online]<br>23 January 1995 (1995-01-23), "Homo sapiens HNRPA2B1 gene for hnRNP protein A2 and B1, complete cds.", XP000002650315, retrieved from EBI accession no. EMBL:D28877<br>Database accession no. D28877<br>* sequence *<br>-----<br>-/-- | 1<br>2-15 | TECHNICAL FIELDS SEARCHED (IPC)<br>C12N<br>C12Q<br>A61K<br>A01K |

The present search report has been drawn up for all claims

| Place of search | Date of completion of the search | Examiner |
|---|---|---|
| Munich | 19 July 2011 | Petri, Bernhard |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 4573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online]<br><br>27 September 1995 (1995-09-27), "PDCD2=programmed cell death-2/Rp8 homolog [human, fetal lung, mRNA, 1282 nt].", XP000002650316, retrieved from EBI accession no. EM_HUM:S78085 Database accession no. S78085 | 1 | |
| Y | * sequence *<br>-& KAWAKAMI T ET AL: "ISOLATION AND MAPPING OF A HUMAN GENE (PDCD2) THAT IS HIGHLY HOMOLOGOUS TO RP8, A RAT GENE ASSOCIATED WITH PROGRAMMED CELL DEATH", CYTOGENETICS AND CELL GENETICS, BASEL, CH, vol. 71, no. 1, 1 January 1995 (1995-01-01), pages 41-43, XP001057875, ISSN: 0301-0171<br>----- | 2-15 | |
| X | DATABASE Nucleotide [Online] NCBI; 11 January 2000 (2000-01-11), Smalley, C.: "Human DNA sequence from clone RP1-191N21 on chromosome 6q27 Contains a 7 transmembrane receptor (rhodopsin family) (olfactory receptor like) pseudogene, the PDCD2 gene for programmed cell death 2 (RP8 homolog), ... two CpG islands, complete sequence.", XP000002650317, retrieved from http://www.ncbi.nlm.nih.gov Database accession no. GI:3790132 | 1 | TECHNICAL FIELDS SEARCHED (IPC) |
| Y | * compound *<br>-----<br>-/-- | 2-15 | |

The present search report has been drawn up for all claims

| Place of search | Date of completion of the search | Examiner |
|---|---|---|
| Munich | 19 July 2011 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

Europäisches Patentamt
European Patent Office
Office européen des brevets

# EUROPEAN SEARCH REPORT

Application Number
EP 10 18 4573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE EMBL [Online]<br><br>2 April 1988 (1988-04-02),<br>"Human beta-actin gene 5'-flanking region",<br>XP000002650318,<br>retrieved from EBI accession no. EM_HUM:Y00474<br>Database accession no. Y00474<br>* sequence * | 2-15 | |
| Y | -& JP 62 262995 A (TAKARA SHUZO CO; KANEGAFUCHI CHEMICAL IND)<br>16 November 1987 (1987-11-16)<br>* abstract *<br>----- | 2-15 | |
| T | S. SAXONOV: "A genome-wide analysis of CpG dinucleotides in the human genome distinguishes two distinct classes of promoters",<br>PROCEEDINGS OF THE NATIONAL ACADEMY OF SCIENCES,<br>vol. 103, no. 5,<br>31 January 2006 (2006-01-31), pages 1412-1417, XP55002395,<br>ISSN: 0027-8424, DOI: 10.1073/pnas.0510310103<br>* page 1412, right-hand column, paragraph 2nd *<br>-----<br>-/-- | | TECHNICAL FIELDS SEARCHED (IPC) |

The present search report has been drawn up for all claims

| Place of search | Date of completion of the search | Examiner |
|---|---|---|
| Munich | 19 July 2011 | Petri, Bernhard |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 4573

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| T | SHE XINWEI ET AL: "Definition, conservation and epigenetics of housekeeping and tissue-enriched genes", BMC GENOMICS, BIOMED CENTRAL, LONDON, GB, vol. 10, no. 1, 17 June 2009 (2009-06-17), page 269, XP021056079, ISSN: 1471-2164, DOI: 10.1186/1471-2164-10-269 * page 2, right-hand column, paragraph 3 - page 3, left-hand column, paragraph first * ----- | | |
| | | | TECHNICAL FIELDS SEARCHED (IPC) |

The present search report has been drawn up for all claims

| Place of search | Date of completion of the search | Examiner |
|---|---|---|
| Munich | 19 July 2011 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

4

EPO FORM 1503 03.82 (P04C01)

## ANNEX TO THE EUROPEAN SEARCH REPORT ON EUROPEAN PATENT APPLICATION NO. EP 10 18 4573

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2011

| Patent document cited in search report | Publication date | Patent family member(s) | Publication date |
|---|---|---|---|
| WO 0005393 A | 03-02-2000 | AU 771111 B2 | 11-03-2004 |
| | | AU 5053499 A | 14-02-2000 |
| | | CA 2333852 A1 | 03-02-2000 |
| | | CN 1334878 A | 06-02-2002 |
| | | EP 1098986 A2 | 16-05-2001 |
| | | JP 4220673 B2 | 04-02-2009 |
| | | JP 2002522027 A | 23-07-2002 |
| | | JP 2005052149 A | 03-03-2005 |
| | | JP 2008109931 A | 15-05-2008 |
| | | JP 2009102331 A | 14-05-2009 |
| | | KR 20070108336 A | 09-11-2007 |
| | | KR 20090117675 A | 12-11-2009 |
| | | MX PA01000830 A | 04-06-2002 |
| JP 62262995 A | 16-11-1987 | JP 1967934 C | 18-09-1995 |
| | | JP 6007168 A | 18-01-1994 |
| | | JP 6102036 B | 14-12-1994 |
| | | JP 2071369 C | 10-07-1996 |
| | | JP 7106152 B | 15-11-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 9902357 W
- WO 0005393 A
- WO 9807876 A


### Non-patent literature cited in the description


- **KAUFMAN.** *Methods Enzymol.,* 1990, vol. 185, 537-566
- **NEEDHAM et al.** *Nucleic Acids Res.,* 1992, vol. 20, 997-1003
- **NEEDHAM et al.** *Protein Expr. Purif.,* 1995, vol. 6, 124-131
- **BIRD et al.** *Cell,* 1985, vol. 40, 91-99
- **TAZI ; BIRD.** *Cell,* 1990, vol. 60, 909-920
- **WISE ; PRAVTCHEVA.** *Genomics,* 1999, vol. 60, 258-271
- **SAMBROOK et al.** Molecular Cloning; A Laboratory Approach. 1989
- **YOUSSOUFIAN ; LODISH.** Transcriptional inhibition of the murine erythropoietin receptor gene by an upstream repetitive element. *Mol Cell Biol,* 1993, vol. 13 (1), 98-104
- **DAVID S. LATCHMAN.** Eukaryotic Transcription Factors. Academic Press Ltd
- **SAMBROOK et al.** Molecular Cloning: A Laboratory Manual. Cold Spring Harbour Laboratory, 1989
- **MARSTON, F.** DNA Cloning Techniques: A Practical Approach. IRL Press, 1987, vol. III
- **F M AUSUBEL et al.** Current Protocols in Molecular Biology. John Wiley & Sons, Inc, 1994
- **CHOWDHURY et al.** *Science,* 1991, vol. 254, 1802-1805
- **WILSON.** *Hum. Gene Ther.,* 1992, vol. 3, 179-222
- **GORDON et al.** *Proc. Natl. Acad. Sci. USA,* 1980, vol. 77, 7380
- **HARBERS et al.** *Nature,* 1981, vol. 293, 540
- **WAGNER et al.** *Proc. Natl. Acad. Sci. USA,* 1981, vol. 78, 5016
- **WAGNER et al.** *Proc. Natl. Acad. Sci. USA,* 1981, vol. 78, 6376
- **HAMMER et al.** *Nature,* 1985, vol. 315, 680
- **G.HICKS et al.** *Nature Genetics,* vol. 16, 338-334
- **DILLON, N. ; GROSVELD, F.** Chromatin domains as potential units of eukaryotic gene function. *Curr. Opin, Genet. Dev.,* 1994, vol. 4, 260-264
- **HIGGS, D.R.** Do LCRs open chromatin domains?. *Cell,* 1998, vol. 95, 299-302
- **PALMITER, R.D. ; BRINSTER, R.L.** Germline transformation of mice. *Ann. Ref. Genet.,* 1986, vol. 20, 465-499
- **ALLEN, N.D. et al.** Transgenes as probes for active chromosomal domains in mouse development. *Nature,* 1988, vol. 333, 852-855
- **BONNEROT, C. ; GRIMBER, G. ; BRIAND, P. ; NICOLAS, J.F.** Patterns of expression of position-dependent integrated transgenes in mouse embryo. *Proc. Natl. Acad. Sci. USA,* 1990, vol. 87, 6331-6335
- **KIOUSSIS, D. ; FESTENSTEIN, R.** Locus control regions: overcoming heterochromatin-induced gene inactivation in mammals. *Curr. Opin. Genet. Dev.,* 1997, vol. 7, 614-619
- **PIKAART, M.J. ; RECILLAS-TARGA, F. ; FELSENFIELD, G.** Loss of transcriptional activity of a transgene is accompanied by DNA methylation and histone deacetylation and is prevented by insulators. *Genes Dev.,* 1998, vol. 12, 2852-2862
- **FUSSENEGGER, M. ; BAILEY, J.E. ; HAUSER, H. ; MUELLER, P.P.** Genetic optimization of recombinant glycoprotein production by mammalian cells. *Trends Biotech.,* 1999, vol. 17, 35-42
- **FRASER, P. ; GROSVELD, F.** Locus control regions; chromatin activation and transcription. *Curr. Opin. Cell Biol.,* 1998, vol. 10, 361-365
- **LI, Q. ; HARJU, S. ; PETERSON, K.R.** Locus Control Regions: coming of age at a decade plus. *Trends Genet.,* 1999, vol. 15, 403-408
- **BULGER, M. ; GROUDINE, M.** Looping versus linking: toward a model for long-distance gene activation. *Genes Dev.,* 1999, vol. 13, 2465-2477
- **GROSVELD, F.** Activation by locus control regions?. *Curr. Opin. Genet. Dev.,* 1999, vol. 9, 152-157
- **BENDER, M.A. ; BULGER, M. ; CLOSE, J. ; GROUDINE, M.** β-globin Gene Switching and Dnase I Sensitivity of the Endogenous β-globin Locus in Mice Do Not Require the Locus Control Region. *Mol. Cell,* 2000, vol. 5, 387-393

- **ORTIZ, B.D. ; CADO, D. ; CHEN, V. ; DIAZ, P.W. ; WINOTO, A.** Adjacent DNA elements dominantly restrict the ubiquitous activity of a novel chromatin-opening region to specific tissues. *EMBO J.,* 1997, vol. 16, 5037-5045
- **ORTIZ, B.D. ; CADO, D. ; WINOTO, A.** A new element within the T-cell receptor alpha locus required for tissue-specific locus control region activity. *Mol. Cell. Biol.,* 1999, vol. 19, 1901-1909
- **ANTEQUERA, F. ; BIRD, A.** Number of CpG islands and genes in human and mouse. *Proc. Natl. Acad Sci. USA,* 1993, vol. 90, 1195-11999
- **CROSS, S.H. ; BIRD, A.P.** CpG islands and genes. *Curr. Opin, Genet. Dev.,* 1995, vol. 5, 309-314
- **TAZI, J. ; BIRD, A.** Alternative chromatin structure at CpG islands. *Cell,* 1990, vol. 60, 909-920
- **IMBERT, G. ; TROTTIER, Y. ; BECHMANN, J. ; MANDEL, J.L.** The gene for the TATA binding protein (TBP) that contains a highly polymorphic protein coding CAG repeat maps to 6q27. *Genomics,* 1994, vol. 21, 667-668
- **PURRELLO, M. et al.** Physical mapping at 6q27 of the locus for the TATA-box binding protein, the DNA bining subunit of TFIID and a component of SL1 and TFIIIB, strongly suggests that it is single copy in the human genome. *Genomics,* 1994, vol. 22, 94-100
- **TRACHTULEC, Z et al.** Linkage of TATA-binding protein and proteasome subunit C5 genes in mice and humans reveals synteny conserved between mammals and invertebrates. *Genomics,* 1997, vol. 44, 1-7
- **OWENS, G.P. ; HAHAN, W.E. ; COHEN, J.J.** Identification of mRNAs associated with programmed cell death in immature thymocytes. *Mol. Cell. Biol.,* 1991, vol. 11, 4177-4188
- **CHALUT, C. ; GALLOIS, Y. ; POTERSZMAN, A. ; MONCOLLIN, V. ; EGLY, J.-M.** Genomic structure of the human TATA-box-binding protein (TBP. *Gene,* 1995, vol. 161, 277-282
- **SCHMIDT, E.E. ; SCHIBLER, U.** High accumulation of components of the RNA polymerase II transcription machinery in rodent spermatids. *Development,* 1995, vol. 121, 2373-2383
- **BIAMONTI, G. ; RUGGIU, M. ; SACCONE, S. ; DELLA VALLE, G. ; RIVA, S.** Two homologous genes, originated by duplication, encode the human hnRNP proteins A2 and A1. *Nucl. Acids Res.,* 1994, vol. 22, 1996-2002
- **KOZU, T. ; HENRICH, B. ; SCHAFER, K.P.** Structure and expression of the gene (HNRPA2B1) encoding the human hnRNP protein A2/B1. *Genomics,* 1995, vol. 25, 365-371
- **YE, Q. ; WOMAN, H.J.** Interaction between an integral protein of the nuclear envelope inner membrane and human chromodomain proteins homologous to Drosophila HP1. *J. Biol. Chem.,* 1996, vol. 271, 14653-14656
- **JAMES, T.C. ; ELGIN, S.C.R.** Identification of a nonhistone chromosomal protein associated with heterochromatin in Drosophila melanogaster and its gene. *Mol. Cell. Biol.,* 1986, vol. 6, 3861-3872
- **SINGH, P.B. et al.** A sequence motif found in a drosophila heterochromatin protein is conserved in animals and plants. *Nucl. Acids Res.,* 1991, vol. 19, 789-794
- **GILLIAND, G. ; PERRIN,M S. ; BLANCHARD, K. ; BUNN, H.F.** Analysis of cytokine Mrna and DNA: detection and quantitation by competitive polymerase chain reaction. *Proc. Natl. Acad. Sci. USA,* 1990, vol. 87, 2725-2729
- **FURTH, P.A. ; HENNIGHAUSEN, L. ; BAKER, C. ; BEATTY, B. ; WOYCHICK, R.** The viarability in activity of the universally expressed human cytomegalovirus immediate early gene 1 promoter/enhancer in transgenic mice. *Nucl. Acids Res.,* 1991, vol. 19, 6205-6208
- **RAY, P. et al.** Ectopic expression of a c-kit minigene in transgenic mice: recapitulation of W phenotypes and evidence for c-kit function in melanoblast progenitors. *Genes Dev.,* 1991, vol. 5, 2265-2273
- **YAMASHITA, T. et al.** High level expression of human $\alpha$-fetoprotoin in transgenic mice. *Biochem, Biophys, Res, Comm.,* 1993, vol. 191, 715-720
- **MILOT, E. et al.** Heterochromatin effects on the frequency and duration of LCR-mediated gene transcription. *Cell,* 1996, vol. 87, 104-114
- **FESTENSTEIN, R. et al.** Locus control region function and heterochromatin-induced position effect variegation. *Science,* 1996, vol. 271, 1123-1125
- **SABBATTINI, P. ; GEORGIOU, A. ; SINCLAIRE, C. ; DILLON, N.** Analysis of mice with single and multiple copies of transgenes reveals a novel arrangement for the $\lambda$5-V-preBI locus control region. *Mol. Cell. Biol.,* 1999, vol. 19, 671-679
- **NG, S.Y. et al.** Evolution of the functional human ß-actin gene and its multi-pseudogene family: conservation of noncoding regions and chromosomal dispersion of pseudogenes. *Mol. Cell. Biol.,* 1985, vol. 5, 2720-2732
- **PRAVTCHEVA, D.D. ; WISE, T.L. ; ENSOR, N.J. ; RUDDLE, F.H.** Mosaic expression of an HPRT transgene integrated in a region of Y heterochromatin. *J. Exp. Zool,* 1994, vol. 268, 452-468
- **BELL, A.C. ; FELSENFIELD, G.** Stopped at the border: boundaries and insulators. *Curr. Opin. Genet. Dev.,* 1999, vol. 9, 191-198
- **WINSTON, J.H. ; HONG, L. ; DATTA, S.K. ; KELLEMS, R.E.** An intron 1 regulatory region from the murine adenosine deaminase gene can activate Heterologous promoters for ubiquitous expression in transgenic mice. *Somat, Cell Mol. Genet.,* 1996, vol. 22, 261-278
- **HART, C.M. ; LAEMMLI, U.K.** Facilitation of chromatin dynamics by SARs. *Curr. Opin. Genet. Develop.,* 1998, vol. 8, 519-525

- **KLEHR, D. ; MAASS, K. ; BODE, J.** Scaffold-attached regions from the human interferon ß domain can be used to enhance the stable expression of genes under the control of various promoters. *Biochemistry,* 1991, vol. 30, 1264-1270
- **MCKNIGHT, R.A. ; SHAMAY, L. ; SANKARAN, L. ; WALL, R.J. ; HENNINGHAUSEN, L.** Matrix-attachment regions can impart position-independent regulation of a tissue-specific gene in transgenic mice. *Proc. Natl. Acad. Sci. USA,* 1992, vol. 89, 6943-6947
- **VAN DRUNEN, C.M. et al.** A bipartite sequence element associated with matrix/scaffold attachment regions. *Nucl. Acids Res.,* 1999, vol. 27, 2924-2930
- **VERMA, I.M. ; SOMIA, N.** Gene Therapy - promises, problems and prospects. *Nature,* 1997, vol. 389, 239-242
- **BROWN, S.A ; KINGSTON, R.E.** Disruption of downstream chromatin directed by a transcriptional activator. *Genes Dev.,* 1997, vol. 11.3, 116-3121
- **ORPHANIDES, G. ; LEROY, G. ; CHANG, C.H. ; LUSE, D.S. ; REINBERG, D.** FACT, a factor that facilitates transcript elongation through nucleosomes. *Cell,* 1998, vol. 92, 105-116
- **SCHNITZLER, G. ; SIF, S. ; KINGSTON, R.E.** Human SWI/SNP interconverts a nucleosome between its base state and a stable remodelled state. *Cell,* 1998, vol. 94, 17-. 27
- **TRAVERS, A.** Chromatin modification by DNA tracking. *Proc. Natl. Acad. Sci. USA,* 1999, vol. 96, 13634-13637
- **ASHE, H.L. ; MONKS, J. ; WIJGERDE, M. ; FRASER, P. ; PROUDFOOT, N.J.** Intergenic transcription and transinduction of the human ß-globin locus. *Genes Dev,* 1997, vol. 11, 2494-2509
- **ROGAN, D.F. ; COUSINS, D.J. ; STAYNOV, D.Z.** Intergenic transcription occurs throughout the human IL-4/IL-13 gene cluster. *Biochem, Biophys, Res. Commun.,* 1999, vol. 255, 556-561
- **GRIBNAU, J. ; DIDERICH, K. ; PRUZINA, S. ; CALZORLARI, R. ; FRASER P.** Intergenic Transcription and Developmental Remodeling of Chromatin Subdomains in the Human ß-globin Locus. *Mol. Cell,* 2000, vol. 5, 377-386
- **VYAS, P. et al.** Cis-acting sequences regulating expression of the human $\alpha$-globin cluster lie within constitutively open Chromatin. *Cell,* 1992, vol. 69, 781-793
- **IOANNOU, P.A. et al.** A new bacteriophage' P1-derived vector for the propagation of large human DNA fragments. *Nature Gene,* 1994, vol. 6, 84-89
- **RAGUZ, S. et al.** Muscle-specific locus control region activity associated with the human desmin gene. *Dev. Biol,* 1998, vol. 201, 26-42
- Transcriptional analysis using transgenic animals. **DILLON, N. ; GROSVELD, F.** Gene Transcription: A practical approach. IRL Press, 1993, 153-188
- **MORGENSTERN, J.P. ; LAND, H.** Advanced mammalian gene transfer: high titre retroviral vectors with multiple drug selection markers and a complementary helper-free packaging cell line. *Nucl. Acids Res,* 1990, vol. 18, 3587-3595
- **HORZ, W. ; ALTENBURGER, W.** Nucleotide sequence of mouse satellite DNA. *Nucl. Acids Res.,* 1981, vol. 9, 683-696
- **MONFOUILLOUX, S. et al.** Recent human-specific spreading of a subtelomeric domain. *Genomics,* 1998, vol. 51, 165-176

SEQUENCE LISTING

<110> ML Laboratories plc

<120> Polynucleotide

<130> SW/P015985KR

<140> PCT/GB01/04210
<141> 2001-09-20

<150> 0022995.5
<151> 2000-09-20

<160> 12

<170> PatentIn version 3.3

<210> 1
<211> 5232
<212> DNA
<213> Artificial

<220>
<223> PDCD2/Actin artificial UCOE sequence

<400> 1
catggcacct gtattgtact cttatcagtc attatatgga ctttaacttc cccagatatt 60

atttgggctc ctccataaga ctgtgagcat ctgaccactg gagtgttgct tcccattata 120

tccctgttat caagcacaag gtcaggcaca gagtaagact caaaacatgt tttggaatgt 180

atgactggta tgaactacaa accagtaagc tgatgttttc attttgagtc tataaatcta 240

attttgtggt ggttttgtgt atggctcaag gctcaaattg taaaatttaa tattatgtga 300

ccaaagaaag ttatacccag aacctcaatt tcctcacctt caaaatgggg cagtttctca 360

ctcattggtc tgctgtcacg attttaatga gctcatgcac aaacagccct ttatataagg 420

taagtgctgg ataaatgttg gctactataa taaaataagc ctctaagata cttggtcagc 480

acaagtacta cccaagagta tgcactgtaa gtaaactgac aaaattgtgt atctaaaact 540

ggccagatga aagagaaact tttaaggggc ccttctgcgt gcccgacact gtgctaggca 600

ctcacactat cccgacccga gaaaccgatc tgcgacccag aggaacttac caagcctcca 660

gcatcttgtg cagccctact catgggacca tctggatacc caccettgtc tttacaggga 720

gcagaacaca cctcttatgt gtcagaaaac aaagtccagg aagtatattt ttacctgagg

780

caatatctga aaattgtatg ctacagcctc caaagtgagt cttcctctca gtacctctct
840

tctaggcaca tggagccctt tcttccaagt attatgttta accacttaat gaatgaagtc
900

ctgaaactgc ttacccatgc tccctataat ctctgagtaa tcttcctttt ccacaacctc
960

aggcataatc tcatcttctg tttctattac aatttcaaat tctggaaaaa ggaagttgtg
1020

gtctggaatt atatggtcca gatgatctga aacaaaaagg acagcactat tagtaatcat
1080

ttagttttga agacagtcta ataatttgct gtctctaaag tactatattc cctatagttc
1140

tggcatttta gataaagggt cataaattaa atgcctatat ggtgacatta ttcagtgatt
1200

cagacttcac agcctttttt ttttttttac aaaggtgttc caggcatgaa aaattttaaa
1260

gtactatacc tttcctaatt ttacctttaa agttgtcctg gaaatatctg ggttgacaaa
1320

ggcgatgaaa ctgaactgag acttaaaaaa aagattaccc acctggttgt gcacaagcct
1380

gcttatgtcc caatctccag tctagggtct gatgctcctt gctgcagtaa tatgctttgt
1440

ggcatctgga gcacgttttg gggcctaaac agccacaaac cctgcagaga tgagcaccag
1500

acttaagctg gagacacact gattctcctg tttctggggg aggattctca gaaggtggct
1560

catatgagta aaaatcgttt ttcctgggta gttgattcct aaaaactaaa aaagaataca
1620

gagaaaagtt ttatcttcaa acaaaacagc aattcacata ttttatcctc tgcacgtaaa
1680

actgaaaata acaacaacaa aaaagaaatg aaagtttttg ctttcaggaa taagctttta
1740

aaatccagaa actagatttc gtccggtaca cgcaactgag ttgcctccta gaggtggttt
1800

gagttaatca aattaataag actgatcgtt aagaacgact gccaaaaata cgaaaaagct
1860

actgggatcc atctttccaa gacaatttct attatctgaa ttaacaccat acctggtacc
1920

cactgattaa aagctggggg ttaccaatgc gcgtgggcac agttagaagc ttatgtagca
1980

aaaatgagca catcctggaa gggcccggga gaaggtgctc ctggggcagc gcggagaggg
2040

agctctgagg ctggggcggc agcggtgctt gccgccgtcc ccctggtcgc tcccggaatt 2100

aacgccgcgc acgcgtcgga ggcatggccc cgtcccgacc ccgtttggcg gctcacctcg 2160

caggccggca cagcacggct gctcgcggca gcagaagagg aagatgcagc ggtggaaggc 2220

gtccgggcgg ccaggcagcg gcgcatacac ctgcagcagg aaggagagcg ggcggccgca 2280

cagctcgcag gccagggcct ggggccccgg cagcccggcc gcgcccagcc atgccggccg 2340

cccgcccacc ttgctgggga actgctcgct gcgcagtcgc cacgccggcg ccgactcggc 2400

gaagcccagc tccacaggcc tggccccggc ggcagccatg cggggcgcgg gctggcgtgg 2460

ggcgcagccc acagctgggt cggaaggcgg aaatcgggcg ccgggccgga aggcaagagg 2520

cgggcacctt tccggaggac aggaggcgga aacgcgtctg acgggagcgg ttgcaggacc 2580

aatgcgaggg aacggggcag aggaaacctc tcggcatcag ccccgcccct ggcgcctctg 2640

cctccgagcc gctttcctgg tgcctccggg tgctctggga tggttctggt ctttgggaga 2700

gtggcagctg gtgacggcgc tccgctcacc tctgcacatg tcttgctgtg ggcctgcggg 2760

tggccgccag ggaggcagag ccctcccgca aaccttccct gctggtgtcc acctcagggt 2820

gtgggaaacc tgtgcgctgg ccgagtgcta accaagagta ggcagtgaaa gacaaatgaa 2880

ggttgaacag gtaaagtgag gaccctacag cggaaaccaa gaatcctgtg tgcctgagag 2940

taatgaagaa gcctctgcag aagagtcttt tctgtcagtc ttaaggtctc tgttttaatg 3000

ttagtgctgg cttgctgtac ctgaattcca agggaggagt gtataatgag gcatggccaa 3060

cccccacttc ccatcattgc ctgaactagt ttttcaggtt aacttcagaa tgcccttggt 3120

accgcgggcc ccctctgtgg tcccacgcca ctgatcgctg catgcccacc acctgggtac 3180

acacagtctg tgattcccgg agcagaacgg accctgccca cccggtcttg tgtgctactc 3240

agtggacaga cccaaggcaa gaaagggtga caaggacagg gtcttcccag gctggctttg 3300

agttcctagc accgccccgc ccccaatcct ctgtggcaca tggagtcttg gtccccagag 3360

tcccccagcg gcctccagat ggtctgggag ggcagttcag ctgtggctgc gcatagcaga 3420

catacaacgg acggtgggcc cagacccagg ctgtgtagac ccagcccccc cgccccgcag 3480

tgcctaggtc acccactaac gccccaggcc tggtcttggc tgggcgtgac tgttaccctc 3540

aaaagcaggc agctccaggg taaaaggtgc cctgccctgt agagcccact tccttcccag 3600

ggctgcggct gggtaggttt gtagccttca tcacgggcca cctccagcca ctggaccgct 3660

ggcccctgcc ctgtcctggg gagtgtggtc ctgcgactct aatggccgca agccacctga 3720

ctcccccaac accacactct acctctcaag cccaggtctc tccctagtga cccacccagc 3780

acatttagct agctgagccc cacagccaga ggtcctcagg ccctgctttc agggcagttg 3840

ctctgaagtc ggcaaggggg agtgactgcc tggccactcc atgccctcca agagctcctt 3900

ctgcaggagc gtacagaacc cagggccctg gcacccgtgc agaccctggc ccaccccacc 3960

tgggcgctca gtgcccaaga gatgtccaca cctaggatgt cccgcggtgg gtgggggccc 4020

cgagagacgg gcaggccggg ggcaggcctg gccatgcggg gccgaaccgg gcactgccca 4080

gcgtggggcg cgggggccac ggcgcgcgcc cccagccccc gggcccagca ccccaaggcg 4140

gccaacgcca aaactctccc tcctcctctt cctcaatctc gctctcgctc tttttttttt 4200

tcgcaaaagg aggggagagg gggtaaaaaa atgctgcact gtgcggcgaa gccggtgagt 4260

gagcggcgcg gggccaatca gcgtgcgccg ttccgaaagt tgccttttat ggctcgagcg 4320

gccgcggcgg cgccctataa aacccagcgg cgcgacgcgc caccaccgcc gagaccgcgt 4380

ccgcccgcga gcacagagcc tcgcctttgc cgatccgccg cccgtccaca cccgccgcca 4440

ggtaagcccg gccagccgac cggggcatgc ggccgcggcc cttcgcccgt gcagagccgc 4500

cgtctgggcc gcagcggggg gcgcatgggg cggaaccgga ccgccgtggg gggcgcggga 4560

gaagcccctg ggcctccgga gatgggggac accccacgcc agttcgcagg cgcgaggccg

4620

cgctcgggcg ggcgcgctcc gggggtgccg ctctcggggc gggggcaacc ggcggggtct
4680

ttgtctgagc cgggctcttg ccaatgggga tcgcacggtg ggcgcggcgt agcccccgtc
4740

aggcccggtg gggctgggg cgccatgcgc gtgcgcgctg gtcctttggg cgctaactgc
4800

gtgcgcgctg ggaattggcg ctaattgcgc gtgcgcgctg ggactcaatg gcgctaatcg
4860

cgcgtgcgtt ctggggcccg ggcgcttgcg ccacttcctg cccgagccgc tggcgcccga
4920

gggtgtggcc gctgcgtgcg cgcgcgcgac ccggtcgctg tttgaaccgg gcggaggcgg
4980

ggctggcgcc cggttgggag gggttgggg cctggcttcc tgccgcgcgc cgcggggacg
5040

cctccgacca gtgtttgcct tttatggtaa taacgcggcc ggcccggctt cctttgtccc
5100

caatctgggc gcgcgccggc gccccctggc ggcctaagga ctcggcgcgc cggaagtggc
5160

cagggcgggg gcgacttcgg ctcacagcgc gcccggctat tctcgcagct caccatgccg
5220

gtcgccacca tg
5232

<210> 2
<211> 6303
<212> DNA
<213> Artificial

<220>
<223> RNP/HP-1/actin artificial UCOE sequence

<400> 2
aagcttactt gattggccat gtggcaagcg acaggcacaa aacaattttc caagtcaata
60

ggaaaaacct cagagctgaa atctttatat gctgtactac acagctgtat tctgggcact
120

tatgaatgtt aaggaaacct gtcttaaaag ttaactaggt taaaaaacct caaacgagag
180

aaagtgatat ccaggaccaa ctgctacaaa cgcataatgc aaactaaaaa gtcacacgta
240

attttcaatc aattattttt tgttcctagc aagcagcatt aattgctgct ctcatcccag
300

ttctacggag ctctccctcc attcgcatgc tcccaactcc taaaaagtag tggtaaaacc
360

cagttcagat ttttttcct gtagttttca tgactcgtaa aaattaaaga aaaaattaac

420

tgaaatgatc aaactagctc ctatgagaca caaagcagtc ttttgaaatg gttacttgtc
480

acgatagtta ttttcatttt ttcagctagt ttttattctt aattgtcgtc agcacatagg
540

ttatctctaa actgaaatta cggataatgt acatttataa caagttttac aaatcactaa
600

caaaaagcaa aaactcatta cttacctcac aatttatcca aacttacccg atgtccacta
660

tcgattttaa acaatgttat tttataaacg tgcttagggt caaagaaaaa taaccaggta
720

gacccccttc gcttgagacc ttatgcttat caatgtaatg ttcaaccaag attgcaaaca
780

aaatgagaaa agtaacaaag ttcaaataca gagcggccca ggcccaaaac agttttgcac
840

atcaatccat acgcattaca ggaaggagcc tctgaagcca tgttttaatc gaagtataac
900

taaggacaaa atcgttattt cactttcctc gtaatcatct ataaaggtcc atggatctgt
960

cccgtaaggg ttaaacttct cagtaacaac attacttaaa atgagtcagc tctacaactt
1020

aaacggaatc cttaagaaca gtaaaggatt ctgacgcgaa tatccctccc ccgcccagaa
1080

aaccaccttc gtccctgccc ctcgtggccg atggcttcca atttatgttt attttgccgc
1140

ggttcatctg tcgttttact gactgcagac ccagataaaa ccgttactca aaggaaaaaa
1200

aagacaggaa aaacataaaa tggtttcttt gtcctacggc tcgcattgaa cccggcccga
1260

cgccctgggt ggtgatatct tctctgaaac cgggcccgca aacccggagc accccccctc
1320

cccgctcttc ggtgtggctt ccgaacgcaa tggcgccatt tcatcgaggg gaaggctgag
1380

cgcctttaat gaggtgcgca ggactctaaa gatccaagct cacaaaacac tccaaatcca
1440

cctcgaaacg atatgaaaac agcccgagaa gaaaaaaaaa atagttaacc acttctactt
1500

cttgatagag aaagcacact aagaaaataa aagagttata aggaaaacgc tgagaggaag
1560

gcgagccatg aaaatggcgg ccgccaaatc ggttcccggg agagaggggg aggggaagct
1620

ccgcagcctc gctcacgagg acctgctgcc cgccgaaacg ctcgccgagg agacgccgtg
1680

gcccccgaag cagcgtgctt tagaaaggga ataagaattc ccgcctccgc gccccacttt
1740

caccccagcg gggcagcgtc cgccatgtga aagctcccca tcccccaccc ccagtgaagg
1800

gaaatggcgc cgggaggctg agggtgggga agctgtttgt acgctcaggc ctccgctcaa
1860

gaccccgttc ataaacctta agccccactg ctactgaatt ggtccgattt cctgcctctc
1920

tcccacggag gcggctggcc gacttccact gaggcgccaa cggcctcgcc atgccctttt
1980

caataactca ttgatttcaa acccgttacc tccatcgcgg actcagtcgc ttcagcccga
2040

tttcccgcag ccgagcgaga tgagagagat ctccgcggac gaacacgaac cggactcgtc
2100

ctggcgctgt agtgagaact gccgctgctc gagaaacaac tctgcgagga gcacctccgc
2160

acgggacccg gcgctgctgc tactgccgct agagccgctg ccgccgcttt tctagaacct
2220

tccccccac taacgcgtct tccgctacgt caggccgtcg cgtaaacgcc ctatccgccg
2280

ccaatggcgg gaaggctcta cgccccacct tacgccaaat gcgtactcct cccacccttg
2340

cggccagaga cagtacccga cgttacttcc gtaaatgcgc tcaatgaatt gcggaaggct
2400

agagtcctgc tagttactac ctcttggaat agggtcccgg cccctgcctt ggcgaaggca
2460

ggtgagaaac gtcgcgcagt ttgaaattaa cgccgacggg aggggcttaa tccgcagcct
2520

ggagatccag ccccctcaac ccgggaggtg gtccctgcag ttacgccaat gataaccccc
2580

gccagaaaaa tcttagtagc cttccctttt tgttttccgt gccccaactc ggcggattga
2640

ctcggcccct tccggaaaca cccgaatcaa cttctagtca aattattgtt cacgccgcaa
2700

tgacccaccc ctggcccgcg tctgtggaac tgacccctgg tgtacaggag agttcgctgc
2760

tgaaagtggt cccaaagggg tactagtttt taagctccca actccccctc ccccagcgtc
2820

tggaggattc cacaccctcg caccggcggg cgaggaagtg ggcggagtcc ggttttggcg
2880

ccagccgctg aggctgccaa gcagaaaagc caccgctgag gagactccgg tcactgtcct
2940

cgccccgcct ccccccttccc tccccttggg gaccaccggg cgccacgccg cgaacggtaa 3000

gtgccgcggt cgtcggcgcc tccgccctcc ccctagggcc ccaattccca gcgggcgcgg 3060

cgccggcccc tccccccgcc gcgcgcgcgc ccgctgcccc gcccttcgtg gccgcccggc 3120

gtgggcggtg ccacccctcc ccccggcggc cccgcgcgca gctcccggct ccctccccct 3180

tcggatgtgg cttgagctgt aggcgcggag ggccggagac gctgcagacc cgcgacccgg 3240

agcagctcgg aggcggtgaa gtcggtggct ttccttctct ctagctctcg ctcgctggtg 3300

gtgcttcaga tgccacacgc gtcccggggg cccggttctc cgctcccctc ccctcccctt 3360

ctcgccggac cccgcgccgg gagctgcggg aaggagtgga gggtcgggcg gtggcctcgc 3420

ggctggcctg gcgcgcggcc agcccggtag ttagtggggg gactgctctg ccctcgaggg 3480

ggtagggagc tgtggcgacg gttgccccat ttcgagacaa agcgcatttc cccctcccct 3540

cccccacccg cgttccggcg gaggcgcccc ctcccccagc gccacgcggg gctgggtcga 3600

gacttgggcc tcccggaggg cggcgcgtgg tcccgcgtcc gcgagcctgg cggcgcgcgg 3660

ccggctgtcc cgaggctgcg gcgaccgcca gttaacgtgg ccgcgcgggg gtaggcgcgt 3720

gcggtgtggc gcagtgccct tgagcccccg tgccgcggcc tttgtttctc cccgcggatg 3780

cgctgaccac gaggcccgcg ctcccgggtg ggggcgggca cccgcgctta ggcctctgga 3840

cgccgggctt cagcggcggg ggtcgggagc gggtgtttgc aagaggtgat tctttttttca 3900

aagtgtcacg aaacggggtt gaagcatctt aagttttttc cttttgttat ttaattaccg 3960

attggaaaga gggagggttt ctgagcagaa accaagttgg gattgcagaa cagagaagat 4020

tcacagtgct ttaccgttgt gagttgtttg ggtaatcgtg cctggtttta aaccgaaagg 4080

attgtccttt aaaaatggaa catggacttt attataaatg ggacttagat tggaaaagac 4140

attggtcccc tattttaagc catgtgaagc tgttttaggt accgcgggcc ccctctgtgg 4200

tcccacgcca ctgatcgctg catgcccacc acctgggtac acacagtctg tgattcccgg

4260

agcagaacgg accctgccca cccggtcttg tgtgctactc agtggacaga cccaaggcaa
4320

gaaagggtga caaggacagg gtcttcccag gctggctttg agttcctagc accgccccgc
4380

ccccaatcct ctgtggcaca tggagtcttg gtccccagag tcccccagcg gcctccagat
4440

ggtctgggag ggcagttcag ctgtggctgc gcatagcaga catacaacgg acggtgggcc
4500

cagacccagg ctgtgtagac ccagcccccc cgccccgcag tgcctaggtc acccactaac
4560

gccccaggcc tggtcttggc tgggcgtgac tgttaccctc aaaagcaggc agctccaggg
4620

taaaaggtgc cctgccctgt agagcccact tccttcccag ggctgcggct gggtaggttt
4680

gtagccttca tcacgggcca cctccagcca ctggaccgct ggcccctgcc ctgtcctggg
4740

gagtgtggtc ctgcgactct aatggccgca agccacctga ctcccccaac accacactct
4800

acctctcaag cccaggtctc tccctagtga cccacccagc acatttagct agctgagccc
4860

cacagccaga ggtcctcagg ccctgctttc agggcagttg ctctgaagtc ggcaaggggg
4920

agtgactgcc tggccactcc atgccctcca agagctcctt ctgcaggagc gtacagaacc
4980

cagggccctg gcacccgtgc agaccctggc ccaccccacc tgggcgctca gtgcccaaga
5040

gatgtccaca cctaggatgt cccgcggtgg gtgggggcc cgagagacgg gcaggccggg
5100

ggcaggcctg gccatgcggg gccgaaccgg gcactgccca gcgtggggcg cgggggccac
5160

ggcgcgcgcc cccagccccc gggcccagca ccccaaggcg gccaacgcca aaactctccc
5220

tcctcctctt cctcaatctc gctctcgctc ttttttttt tcgcaaaagg aggggagagg
5280

gggtaaaaaa atgctgcact gtgcggcgaa gccggtgagt gagcggcgcg gggccaatca
5340

gcgtgcgccg ttccgaaagt tgccttttat ggctcgagcg gccgcggcgg cgccctataa
5400

aacccagcgg cgcgacgcgc caccaccgcc gagaccgcgt ccgcccgcga gcacagagcc
5460

tcgcctttgc cgatccgccg cccgtccaca cccgccgcca ggtaagcccg gccagccgac
5520

cggggcatgc ggccgcggcc cttcgcccgt gcagagccgc cgtctgggcc gcagcggggg
5580

gcgcatgggg cggaaccgga ccgccgtggg gggcgcggga gaagcccctg ggcctccgga
5640

gatgggggac accccacgcc agttcgcagg cgcgaggccg cgctcgggcg ggcgcgctcc
5700

gggggtgccg ctctcggggc gggggcaacc ggcggggtct ttgtctgagc cgggctcttg
5760

ccaatgggga tcgcacggtg ggcgcggcgt agcccccgtc aggcccggtg ggggctgggg
5820

cgccatgcgc gtgcgcgctg gtcctttggg cgctaactgc gtgcgcgctg ggaattggcg
5880

ctaattgcgc gtgcgcgctg ggactcaatg gcgctaatcg cgcgtgcgtt ctggggcccg
5940

ggcgcttgcg ccacttcctg cccgagccgc tggcgcccga gggtgtggcc gctgcgtgcg
6000

cgcgcgcgac ccggtcgctg tttgaaccgg gcggaggcgg ggctggcgcc cggttgggag
6060

ggggttgggg cctggcttcc tgccgcgcgc cgcggggacg cctccgacca gtgtttgcct
6120

tttatggtaa taacgcggcc ggcccggctt cctttgtccc caatctgggc gcgcgccggc
6180

gccccctggc ggcctaagga ctcggcgcgc cggaagtggc cagggcgggg gcgacttcgg
6240

ctcacagcgc gcccggctat tctcgcagct caccatgccg gtcgccacca tgataccgtc
6300

gac
6303

<210> 3
<211> 29
<212> DNA
<213> Artificial

<220>
<223> Primer

<400> 3
gcggtaccaa gggcattctg aagttaacc
29

<210> 4
<211> 16
<212> DNA
<213> Artificial

<220>
<223> Primer

<400> 4
agctccacag gcctgg
16

<210> 5
<211> 13
<212> DNA
<213> Artificial

<220>
<223> Oligonucleotide

<400> 5
aacaattggc ggc
13

<210> 6
<211> 13
<212> DNA
<213> Artificial

<220>
<223> Oligonucleotide

<400> 6
gccaattgtt gcc
13

<210> 7
<211> 31
<212> DNA
<213> Artificial

<220>
<223> Primer

<400> 7
acgcgtcgac ggaaggagac aataccggaa g
31

<210> 8
<211> 29
<212> DNA
<213> Artificial

<220>
<223> Primer

<400> 8
ccgctcgagt tggggtgggg gaaaaggaa
29

<210> 9
<211> 30
<212> DNA
<213> Artificial

<220>

<223> Primer

<400> 9
cgggatccgc ctgagaaagg aagtgagctg
30

<210> 10
<211> 29
<212> DNA
<213> Artificial

<220>
<223> Primer

<400> 10
gaagatctgg aggaatgagc tggcccctta
29

<210> 11
<211> 35
<212> DNA
<213> Artificial

<220>
<223> Primer

<400> 11
ctcgagttat taatagtaat caattacggg gtcat
35

<210> 12
<211> 33
<212> DNA
<213> Artificial

<220>
<223> Primer

<400> 12
gtcgacgatc tgacggttca ctaaaccagc tct
33